# EUROPEAN PATENT APPLICATION

(11) **EP 2 060 572 A1**
(43) Date of publication of application: **20.05.2009**
(21) Application number: 07828195.3
(22) Date of filing: 05.09.2007
(51) Int. Cl.: C07D 471/04, A61K 31/444, A61P 11/00, A61P 11/02, A61P 11/06, A61P 19/02, A61P 25/00, A61P 25/14, A61P 25/16, A61P 25/18, A61P 25/28, A61P 29/00, A61P 37/08, A61P 43/00

(54) **PYRAZOLOPYRIDINE DERIVATIVE AND PHOSPHODIESTERASE (PDE) INHIBITOR CONTAINING THE SAME AS ACTIVE INGREDIENT**

(30) Priority: 06.09.2006 JP 2006241617
(71) Applicant: Kyorin Pharmaceutical Co., Ltd., Tokyo 101-8311 (JP)
(72) Inventor: KOHNO, Yasushi, Shimotsuga-gun, Tochigi 3290114 (JP); TAKITA, Satoshi, Shimotsuga-gun, Tochigi 3290114 (JP); KOJMA, Akihiko, Shimotsuga-gun, Tochigi 3290114 (JP); KISHI, Tetsuya, Shimotsuga-gun, Tochigi 3290114 (JP)
(74) Representative: Vossius & Partner
(86) International application number: PCT/JP2007/067267
(87) International publication number: WO 2008/029829

(57) **Abstract**

A novel pyrazolopyridine derivative is provided which is useful as a pharmaceutical drug having phosphodiesterase inhibitory activity.

The pyrazolopyridine derivative is represented by the following general formula (1): 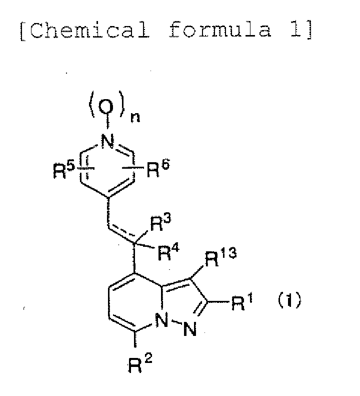 [wherein R¹ is a hydrogen atom, an optionally substituted alkyl group having 1 to 6 carbon atoms, or the like, R² is an optionally substituted alkyl group having 1 to 6 carbon atoms or the like, R⁵ and R⁶ are independently a hydrogen atom or a halogen atom, R¹³ is a hydrogen atom or a halogen atom, n is 0 or 1, 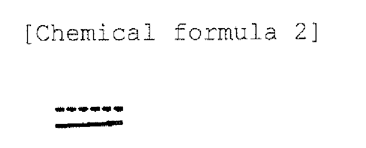 is a single or double bond, R³ is a hydrogen atom, a hydroxyl group, or the like, and R⁴ is a hydrogen atom, a phenyl group, or the like] or a pharmaceutically acceptable salt thereof, and a hydrate thereof.

## Description

### TECHNICAL FIELD

The present invention relates to a pyrazolopyridine derivative useful as a phosphodiesterase (PDE) inhibitor.

### BACKGROUND ART

Phosphodiesterases (PDE) are enzymes that break down cyclic AMP (cAMP) and cyclic GMP (cGMP), which are second messengers in living organisms. At present, 11 families of PDEs being PDE1 to PDE11 have been identified, and each family specifically breaks down either cAMP or cGMP or both. The PDE families are distributed differently in various tissues. It has been considered that the cell reactions in different organs are controlled by different PDE families.

Up to the present, a large number of PDE inhibitors have been developed. For example, PDE3 inhibitors are expected to be effective drugs for angina pectoris, cardiac failure, hypertension, and the like and to be platelet aggregation inhibitors and antiasthmatic drugs. PDE4 inhibitors are expected to be effective drugs for bronchial asthma, chronic obstructive pulmonary disease (CCPD), interstitial pneumonia, allergic rhinitis, atopic dermatitis, rheumatic arthritis, multiple sclerosis, Alzheimer's disease, dementia, Parkinson's disease, and the like. PDE5 inhibitors have already been clinically used as therapeutic drugs for male sexual dysfunction. Moreover, it has been recently reported that the use of minocycline as a PDE10A modulator is effective for patients with Huntington's disease (Patent Document 1). Also, a patent application publication (Patent Document 2) discloses that PDE10 inhibitors are effective drugs for various psychiatric disorders such as Huntington's disease, Alzheimer's disease, dementia, Parkinson's disease, and schizophrenia.

Patent Documents 3 to 6 disclose pyrazolopyridine derivatives with PDE inhibitory activity. However, the compounds of the present application, i.e., derivatives in which a pyridine ring is linked to a pyrazolopyridine ring via a carbon chain, are not disclosed in Patent Documents 3 to 6. Moreover, PDE inhibitors based on heterocyclic rings such as benzofuran, benzodioxol, benzodioxocin, benzodioxepin, and indole and similar to the inhibitors of the present patent are disclosed in Patent Documents 7 to 14. However, the compounds of the present patent are characterized by being based on pyrazolopyridine, and such compounds are not disclosed in Patent Documents 7 to 14.
[Patent Document 1] WO 01024781 pamphlet.
[Patent Document 2] Japanese Patent Application Laid-Open No. 2002-363103.
[Patent Document 3] Domestic re-publication of PCT international application WO9814448.
[Patent Document 4] Japanese Patent Application Laid-Open No. H10-109988.
[Patent Document 5] Japanese Patent Application Laid-Open No. 2006-117647.
[Patent Document 6] Japanese Patent Application Laid-Open No. 2006-169138.
[Patent Document 7] WO 2003066044 pamphlet.
[Patent Document 8] US 2002128290 pamphlet.
[Patent Document 9] NO 9937640 pamphlet.
[Patent Document 10] WO 9916768 pamphlet.
[Patent Document 11] WO 9636624 pamphlet.
[Patent Document 12] Japanese Patent Application Laid-Open No. 2004-196785.
[Patent Document 13] WO 9822455 pamphlet.
[Patent Document 14] WO 9748697 pamphlet.

### DISCLOSURE OF THE INVENTION

### PROBLEMS TO BE SOLVED BY THE INVENTION

It is an object of the present invention to provide pyrazolopyridine derivatives having high phosphodiesterase inhibitory activity with few side effects.

### MEANS FOR SOLVING THE PROBLEMS

The present inventors have conducted intensive studies to develop highly safe compounds with phosphodiesterase inhibitory activity and have consequently found that novel pyrazolopyridine derivatives having structures different from those of known PDE inhibitors have very high PDE inhibitory activity. Thus, the invention has been completed.

Accordingly, the present invention relates to the following:
1) A pyrazolopyridine derivative represented by the general formula (1):

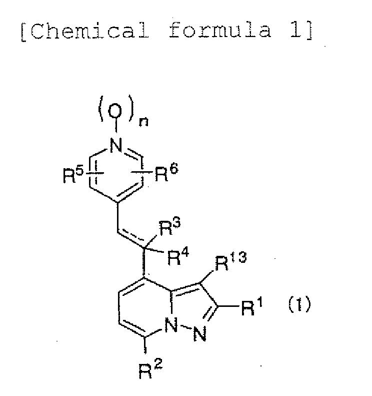

[wherein R¹ is a hydrogen atom, an optionally substituted alkyl group having 1 to 6 carbon atoms (optionally substituted with a halogen atom, an alkoxy group having 1 to 6 carbon atoms, or a hydroxyl group), a cycloalkyl group having 3 to 8 carbon atoms, an alkanoyl group having 1 to 6 carbon atoms, an oxime group, or a cyano group, R² is a hydrogen atom, an optionally substituted alkyl group having 1 to 6 carbon atoms (optionally substituted with an alkoxy group having 1 to 6 carbon atoms or a hydroxyl group), an alkoxy group having 1 to 6 carbon atoms, an alkylsulfanyl group having 1 to 6 carbon atoms, an alkylsulfinyl group having 1 to 6 carbon atoms, an alkylsulfonyl group having 1 to 6 carbon atoms, an optionally substituted amino group (optionally substituted with an alkyl group having 1 to 6 carbon atoms), or an alkanoyl group having 1 to 6 carbon atoms, R⁵ and R⁶ are independently a hydrogen atom or a halogen atom, R¹³ is a hydrogen atom or a halogen atom, n is 0 or 1, and

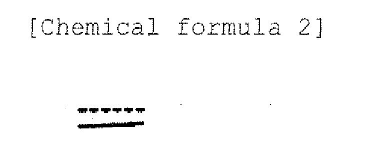

represents a single or double bond, wherein, when

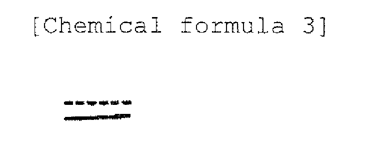

is a double bond, R³ is involved in formation of the double bond, and R⁴ is a hydrogen atom or a phenyl group, and wherein, when

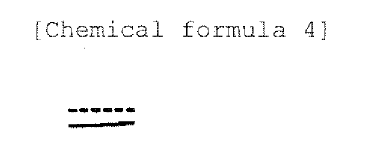

is a single bond, R³ is a hydrogen atom or a hydroxyl group and R⁴ is a hydrogen atom or a phenyl group, or R³ and R⁴ together form oxo] or a pharmaceutically acceptable salt or hydrate thereof.

2) The pyrazolopyridine derivative according to 1), or a pharmaceutically acceptable salt or hydrate thereof, wherein the compound represented by the general formula (1) is represented by the general formula (1a):

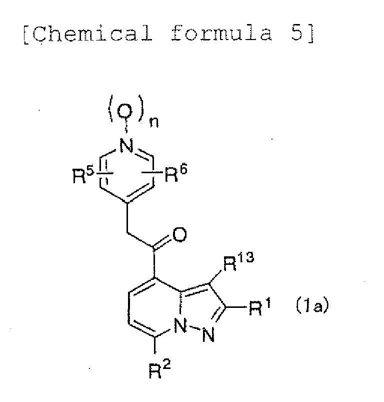

[wherein R¹, R², R⁵, R⁶, R¹³, and n are as described above].

3) The pyrazolopyridine derivative according to 1), or a pharmaceutically acceptable salt or hydrate thereof, wherein the compound represented by the general formula (1) is represented by the general formula (1b):

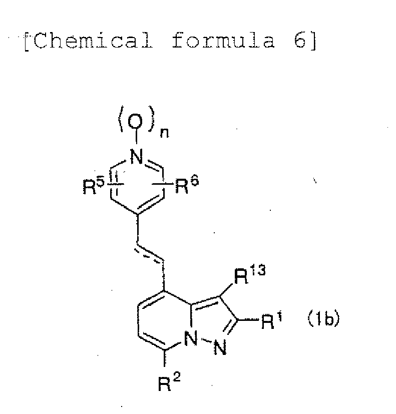

[wherein R¹, R², R⁵, R⁶, R¹³, n, and

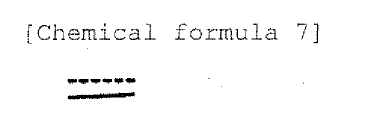

1are as described above].

4) The pyrazolopyridine derivative according to 1), or a pharmaceutically acceptable salt or hydrate thereof, wherein, in the compound represented by the general formula (1), R¹³ is a hydrogen atom.

5) The pyrazolopyridine derivative according to 1), or a pharmaceutically acceptable salt or hydrate thereof, wherein the compound represented by the general formula (1) is
   7-methoxy-4-[(2-pyridin-4-yl )vinyl]-2-trifluoromethylpyrazolo [1,5-a]pyridine,
   7-methoxy-4-[(2-pyridin-4-yl )ethyl]-2-trifluoromethylpyrazolo [1,5-a]pyridine,
   1-(7-methoxy-2-trifluoromethylpyrazolo[1,5-a]pyridin-4-yl )-2-(pyridin-4-yl )ethanone,
   2-(3,5-dichloropyridin-4-yl )-1-(7-methoxy-2-trifluoromethylpy razolo[1,5-a]py-ridin-4-yl )ethanone,
   (E)-4-[2-(3,5-dichloropyridin-4-yl )vinyl]-7-methoxy-2-trifluo romethylpyrazolo[1,5-a]pyridine,
   2-(pyridin-4-yl )-1-(7-methoxy-2-trifluoromethylpyrazolo[1,5-a ]pyridin-4-yl )ethanone,
   4-(2-(7-methoxy-2-(trifluoromethyl)pyrazolo[1, 5-a]pyridin-4-yl )ethyl)pyridine 1-oxide,
   3,5-dichloro-4-(2-(7-methoxy-2-(trifluoromethyl)pyrazolo[1, 5-a]pyridin-4-yl )-2-oxoethyl)pyridine 1-oixide,
   4-(2-(7-methoxy-2-(trifluoromethyl)pyrazolo[1,5-a]pyridin-4-yl )-2-oxoethyl)pyridine 1-oxide,
   2-(3,5-dichloropyridin-4-yl )-1-(7-(methylamino)-2-(trifluorom ethyl)pyrazolo[1,5-a]pyridin-4-yl )ethanone,
   3,5-dichloro-4-(2-(2-(difluoromethyl)-7-methoxypyrazolo[1,5-a] pyridin-4-yl )-2-oxoethyl)pyridine 1-oxide,
   1-(2-cyclopropyl-7-methoxypyrazolo[1,5-a]pyridin-4-yl )-2-(pyr idin-4-yl )ethanone,
   1-(3-chloro-2-cyclopropyl-7-methoxypyrazolo[1,5-a]pyridin-4-yl )-2-(3,5-dichloropyridin-4-yl )ethanone, or
   3,5-dichloro-4-(2-(2-cyano-7-methoxypyrazolo[1,5-a]pyridin-4-y l )-2-oxoethyl)pyridine 1-oxide.

6) A phosphodiesterase (PDE) inhibitor comprising the pyrazolopyridine derivative according to any of 1) to 5), or a pharmaceutically acceptable salt or hydrate thereof.

7) A pharmaceutical comprising the pyrazolopyridine derivative according to any of 1) to 5), or a pharmaceutically acceptable salt or hydrate thereof.

### EFFECTS OF THE INVENTION

The novel pyrazolopyridine derivatives of the present invention and addition salts thereof have high phosphodiesterase (PDE) inhibitory activity. Therefore, the pyrazolopyridine derivatives and the addition salts thereof are useful as preventive and therapeutic drugs for bronchial asthma, chronic obstructive pulmonary disease (COPD), interstitial pneumonia, allergic rhinitis, atopic dermatitis, rheumatic arthritis, multiple sclerosis, Huntington's disease, Alzheimer's disease, dementia, Parkinson's disease, schizophrenia, and the like.

### BEST MODE FOR CARRYING OUT THE INVENTION

In the present invention, the "alkoxy group having 1 to 6 carbon atoms" of R¹ and R² is a linear or branched alkoxy group having 1 to 6 carbon atoms and is preferably an alkoxy group having 1 to 4 carbon atoms. Examples of such an alkoxy group include a methoxy group, an ethoxy group, a propoxy group, an isopropoxy group, a butoxy group, an isobutoxy group, a sec-butoxy group, and a t-butoxy group.

The "halogen atom" of R¹, R⁵, R⁶, and R¹³ is a fluorine atom, a chlorine atom, a bromine atom, or an iodine atom.

The "alkyl group having 1 to 6 carbon atoms" of R¹ and R² is a linear or branched alkyl group having 1 to 6 carbon atoms and is preferably an alkyl group having 1 to 4 carbon atoms. Examples of such an alkyl group include a methyl group, an ethyl group, a propyl group, an isopropyl group, a butyl group, an isobutyl group, a sec-butyl group, and a t-butyl group.

Examples of the substituted alkyl group of the "optionally substituted alkyl group having 1 to 6 carbon atoms" of R¹ include a hydroxymethyl group, a methoxymethyl group, an ethoxymethyl group, a propoxymethyl group, an isopropoxymethyl group, a butoxymethyl group, an isobutoxymethyl group, a sec-butoxymethyl group, a t-butoxymethyl group, a monofluoromethyl group, a difluoromethyl group, anda trifluoromethyl group. Of these, a hydroxymethyl group, a methoxymethyl group, and a trifluoromethyl group are preferred.

Examples of the substituted alkyl group of the "optionally substituted alkyl group having 1 to 6 carbon atoms" of R² include a hydroxymethyl group, a ethoxymethyl group, an ethoxymethyl group, a propoxymethyl group, an isopropoxymethyl group, a butoxymethyl group, an isobutoxymethyl group, a sec-butoxymethyl group, and a t-butoxymethyl group. Of these, a hydroxymethyl group and a methoxymethyl group are preferred.

Examples of the "cycloalkyl group having 3 to 8 carbon atoms" of R¹ include a cyclopropyl group, a cyclobutyl group, a cyclopentyl group, a cyclohexyl group, and a cyclooctyl group.

The "alkanoyl group having 1 to 6 carbon atoms" of R¹ and R² is a linear or branched alkanoyl group having 1 to 6 carbon atoms and is preferably an alkanoyl group having 1 to 4 carbon atoms. Examples of such an alkanoyl group include a formyl group, an acetyl group, a propionyl group, a butyryl group, and an isobutyryl group.

The "alkylsulfinyl group having 1 to 6 carbon atoms" of R² is a linear or branched alkylsulfanyl group having 1 to 6 carbon atoms and is preferably an alkylsulfanyl group having 1 to 4 carbon atoms. Examples of such an alkylsulfanyl group include a methylsulfanyl group, an ethylsulfanyl group, a propylsulfanyl group, an isopropylsulfanyl group, a butylsulfanyl group, a isobutylsulfanyl group, a sec-butylsulfanyl group, and a t-butylsulfanyl group.

The "alkylsulfinyl group having 1 to 6 carbon atoms" of R² is a linear of branched alkylsulfinyl group having 1 to 6 carbon atoms and is preferably an alkylsulfinyl group having 1 to 4 carbon atoms. Examples of such an alkylsulfinyl group include a methylsulfinyl group, an ethylsulfinyl group, a propylsulfinyl group, an isopropylsulfinyl group, a butylsulfinyl group, an isobutylsulfinyl group, a sec-butylsulfinyl group, and a t-butylsulfinyl group.

The "alkylsulfonyl group having 1 to 6 carbon atoms" of R² is a linear or branched alkylsulfonyl group having 1 to 6 carbon atoms and is preferably an alkylsulfonyl group having 1 to 4 carbon atoms. Examples of such an alkylsulfonyl group include a methylsulfonyl group, an ethylsulfonyl group, a propylsulfonyl group, an isopropylsulfonyl group, a butylsulfonyl group, an isobutylsulfonyl group, a sec-butylsulfonyl group, and a t-butylsulfonyl group.

The "optionally substituted amino group" of R² is an amino group optionally substituted with a linear or branched alkyl group having 1 to 6 carbon atoms and is preferably an alkylamino group having 1 to 4 carbon atoms. Examples of the substituted amino group include a methylamino group, an ethylamino group, a propylamino group, an isopropylamino group, a butylamino group, an isobutylamino group, a sec-butylamino group, a t-butylamino group, a dimethylamino group, a diethylamino group, a dipropylamino group, a diisopropylamino group, and an ethylmethylamino group.

The phrase "R³ is involved in formation of the double bond" refers to that R³ forms the bond between the carbon atom to which R³ is bonded and the carbon atom adjacent to this carbon atom so that the carbon chain has the double bond.

In the present invention, examples of the pharmaceutically acceptable salt include acid addition salts such as hydrochlorides, hydrobromides, acetates, trifluoroacetates, methanesulfonates, citrates, and tartrates.

When R³ is a hydroxyl group, R⁴ is a phenyl group, R¹³ is a hydrogen atom, n is 0, and

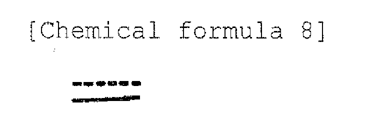

is a single bond, then the compounds represented by the general formula (1) are represented by the general formula (1c):

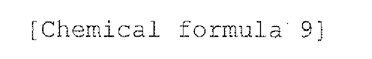

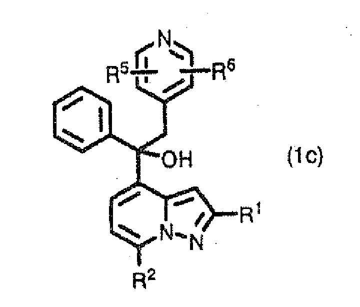

[wherein R¹, R², R⁵, and R⁶ are as described above]. In the present invention, the compounds represented by the general formula (1c) can be produced via, for example, the synthesis route A described below.

<Synthesis route A>

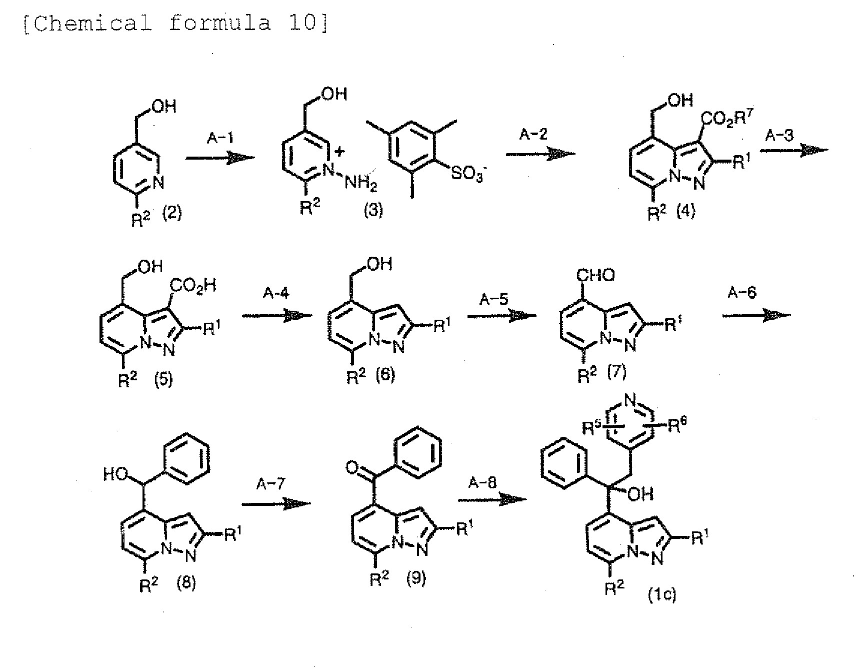

In the synthesis route A, the compound represented by the general formula (3) can be produced by allowing the compound represented by the general formula (2) and O-mesitylenesulfonylhydroxyamine (hereinafter referred to as MSH) to act (step A-1). Preferably, the reaction is carried out by dissolving the compound represented by the general formula (2) in methylene chloride and allowing a methylene chloride solution of MSH and the prepared solution to act at 0°C to room temperature.

In the synthesis route A, the compound represented by the general formula (4):

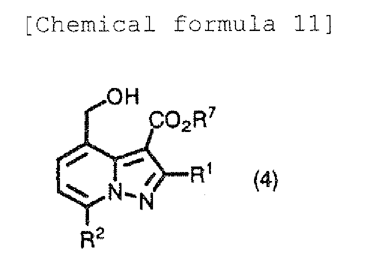

[wherein R⁷ is a lower alkyl group having 1 to 6 carbon atoms or a benzyl group, and R¹ and R² are as described above] can be produced by allowing the compound represented by the general formula (3) and the compound represented by the general formula (10):

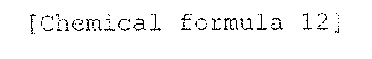

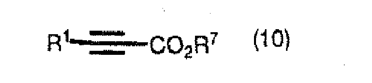

[wherein R¹ and R⁷ are as described above] to act in the presence of a base (step A-2).

The reaction may be carried out in a reaction solvent such as methanol, ethanol, 1,4-dioxane, dimethyl sulfoxide (DMSO), N,N-dimethylformamide (DMF), tetrahydrofuran (THF), cyclopentyl methyl ether (CPME), toluene, benzene, cyclohexane, cyclopentane, methylene chloride, chloroform, or acetonitrile at a reaction temperature of 0°C to room temperature in the presence of an inorganic base such as sodium hydrogen carbonate, sodium carbonate, potassium hydrogen carbonate, or potassium carbonate or an organic base such as triethylamine.

In the synthesis route A, the compound represented by the general formula (5) can be produced by subjecting the compound represented by the general formula (4) to hydrolysis (step A-3).

The reaction may be carried out in a solvent such as methanol, ethanol, THF, CPME, DMSO, DMF, or 1,4-dioxane at room temperature or under heating and reflux by adding an aqueous solution of potassium, sodium, or lithium hydroxide, preferably an aqueous solution of sodium hydroxide.

In the synthesis route A, the compound represented by the general formula (6) can be produced by decarboxylation of the compound represented by the general formula (5) (step A-4) or hydrolysis and decarboxylation of the compound represented by the general formula (4).

The reaction in the step A-4 may be carried out by heating the compound represented by the general formula (5) at 100°C to 160°C in an organic solvent such as benzene, chlorobenzene, dichlorobenzene, bromobenzene, toluene, or xylene. Alternatively, this reaction may be carried out by heating the compound at 80°C to 120°C in ethanol or 1, 4-dioxane after addition of a 2 to 10% aqueous sulfuric acid solution or by heating the compound at 80°C to 120°C in a 50% aqueous sulfuric acid solution.

When the compound represented by the general formula (4) is used, the reaction may be carried out by using hydrobromic acid or acetic acid containing hydrogen bromide under heating to reflux. Alternatively, this reaction may be carried out by heating the compound at 80°C to 120°C in ethanol or 1,4-dioxane after addition of a 2 to 10% aqueous sulfuric acid solution or by heating the compound at 80°C to 120°C in a 50% aqueous sulfuric acid solution.

In the synthesis route A, the compound represented by the general formula (7) can be produced by oxidizing the compound represented by the general formula (6) (step A-5).

The reaction may be carried out using any method commonly used to oxidize alcohols to aldehydes or ketones. For example, the reaction maybe carried out by using a chromium oxide-pyridine complex such as pyridinium chlorochromate or pyridinium dichromate, a metal oxidizing agent such as chromium oxide, silver carbonate, or manganese dioxide, or a DMSO oxidation activating agent such as a sulfur trioxide-pyridine complex, oxalyl chloride, trifluoroacetic anhydride, acetic anhydride, or dicyclohexylcarbodiimide (DCC), or by Dess-Martin oxidation. The reaction may be performed at a temperature of -78°C to 100°C.

In the synthesis route A, the compound represented by the general formula (8) can be produced by reacting the compound represented by the general formula (7) with the compound represented by the general formula (11):

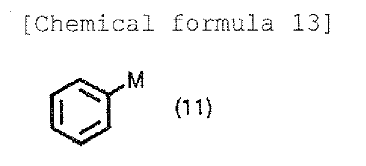

[wherein M is Li, MgCl, MgBr, or MgI] (step A-6).

Preferably, the reaction is carried out by mixing the compounds represented by the general formulas (7) and (11) in a solvent such as THE, 1,4-dioxane, or ether at -78°C to 0°C and slowly heating the mixture to room temperature.

In the synthesis route A, the compound represented by the general formula (9) can be produced by oxidizing the compound represented by the general formula (8) (step A-7).

The reaction may be carried out using any method commonly used to oxidize alcohols to aldehydes or ketones and may be carried out as in, for example, the step A-5.

In the synthesis route A, the compound represented by the general formula (1c) can be produced by reacting the compound represented by the general formula (9) with the compound represented by the general formula (12):

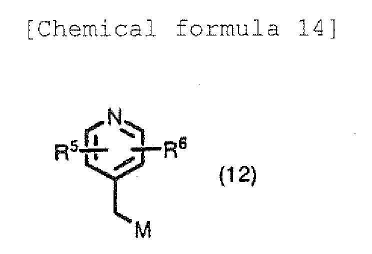

[wherein R⁵, R⁶, and M are as described above] (step A-8).

Preferably, the reaction is carried out by mixing the compounds represented by the general formulas (9) and (12) in a solvent such as THF, 1,4-dioxane, or ether at -78°C to 0°C and slowly heating the mixture to room temperature.

When R⁴ is a phenyl group, R¹³ is a hydrogen atom, n is 0, and

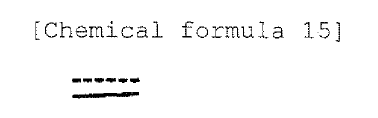

is a double bond (R³ is involved in the formation of the double bond), then the compound represented by the general formula (1) is represented by the general formula (1d):

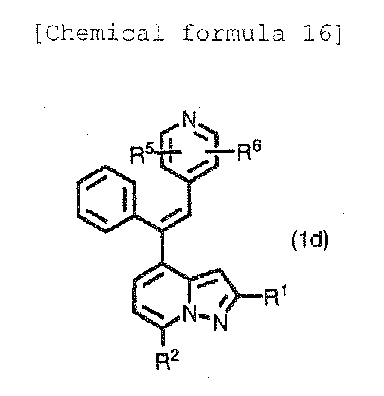

[wherein R¹, R², R⁵, and R⁶ are as described above]. This compound can be produced by dehydration of the compound represented by the general formula (1c).

The reaction may be carried out in a reaction solvent such as benzene, toluene, or xylene by adding, as a dehydrating agent, p-toluenesulfonic acid, pyridinium p-toluenesulfonate, sulfuric acid, or methanesulfonic acid, preferably methanesulfonic acid, at 70°C or under heating and reflux.

When R³ is a hydrogen atom, R⁴ is a phenyl group, R¹³ is a hydrogen atom, n is 0, and

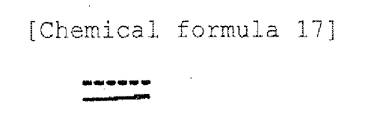

is a single bond, then the compound of general formula (1) is represented by the general formula (1e):

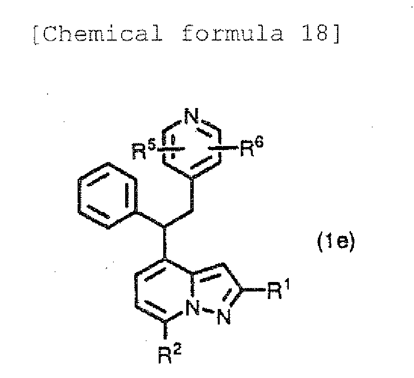

[wherein R¹, R², R⁵, and R⁶ are as described above]. This compound can be produced by reduction of the compound represented by the general formula (1d).

The reaction may be carried out in a solvent such as ethanol, methanol, THF, DMF, or ethyl acetate at room temperature under normal pressure or high hydrogen pressure in the presence of a catalytic reduction catalyst such as palladium-carbon, platinum-carbon, platinum oxide, rhodium-carbon, or ruthenium-carbon.

The compound represented by the general formula (1e) can also be produced from the compound represented by the general formula (1c). The reaction may be carried out in a solvent such as ethanol, methanol, THF, DMF, or ethyl acetate by adding hydrochloric acid or acetic acid at room temperature under normal pressure or high hydrogen pressure in the presence of a catalytic reduction catalyst such as palladium-carbon, platinum-carbon, platinum oxide, rhodium-carbon, or ruthenium-carbon. Alternatively, the compound can be produced as follows. First, a sulfonate is formed by reacting with methanesulfonyl chloride or p-toluenesulfonyl chloride in a solvent such as THF at 0°C to room temperature in the presence of a base such as triethylamine or pyridine, and then the resultant product is reacted with LiAlH₄ in a solvent such as THF at 0°C to room temperature.

When R³ is a hydroxyl group, R⁴ is a hydrogen atom, R¹³ is a hydrogen atom, n is 0, and

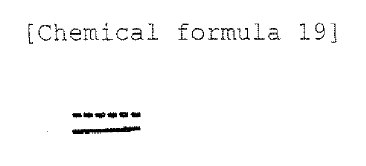

is a single bond, then the compound represented by the general formula (1) is represented by the general formula (1f):

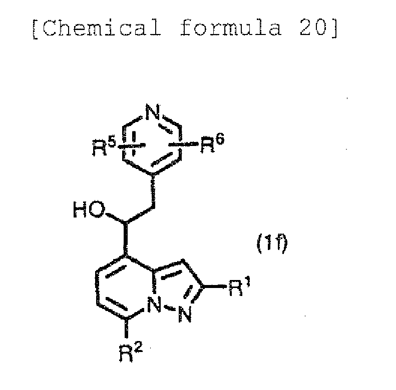

[wherein R¹, R², R⁵, and R⁶ are as described above]. When R⁴ is a hydrogen atom, R¹³ is a hydrogen atom, n is 0, and

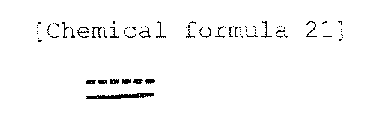

is a double bond (R³ is involved in the formation of the double bond), then the compound represented by the general formula (1) is represented by the general formula (1g):

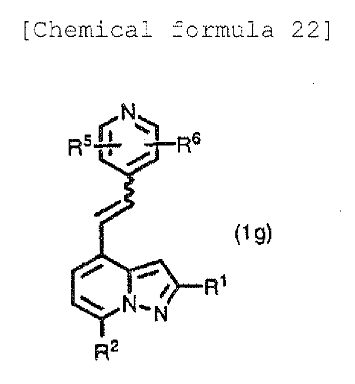

[wherein R¹, R², R⁵, and R⁶ are as described above]. When R³ and R⁴ together form oxo, R¹³ is a hydrogen atom, n is 0, and

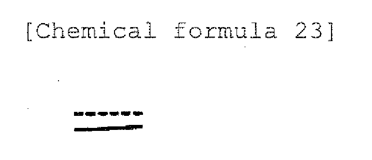

is a single bond, then the compound represented by the general formula (1) is represented by the general formula (1h):

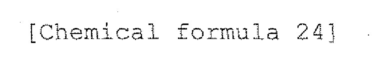

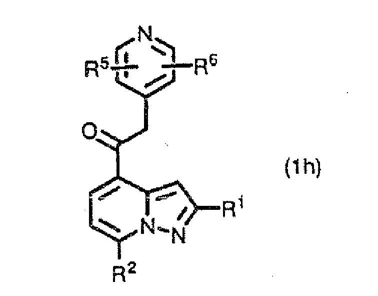

[wherein R¹, R², R⁵, and R⁶ are as described above] . When R³ and R⁴ are each a hydrogen atom, R¹³ is a hydrogen atom, n is 0, and

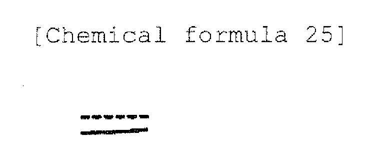

is a single bond, then the compound represented by the general formula (1) is represented by the general formula (1j):

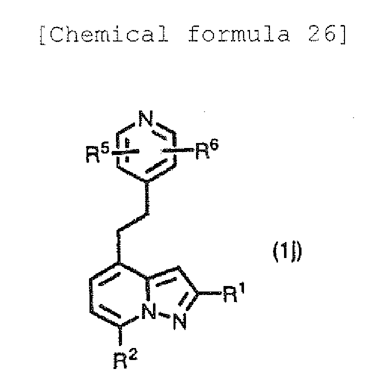

[wherein R¹, R², R⁵, and R⁶ are as described above]. The compounds represented by the general formulas (1f), (1g), (1h), and (1j) can be synthesized via, for example, the synthesis route B described below.

<Synthesis route B>

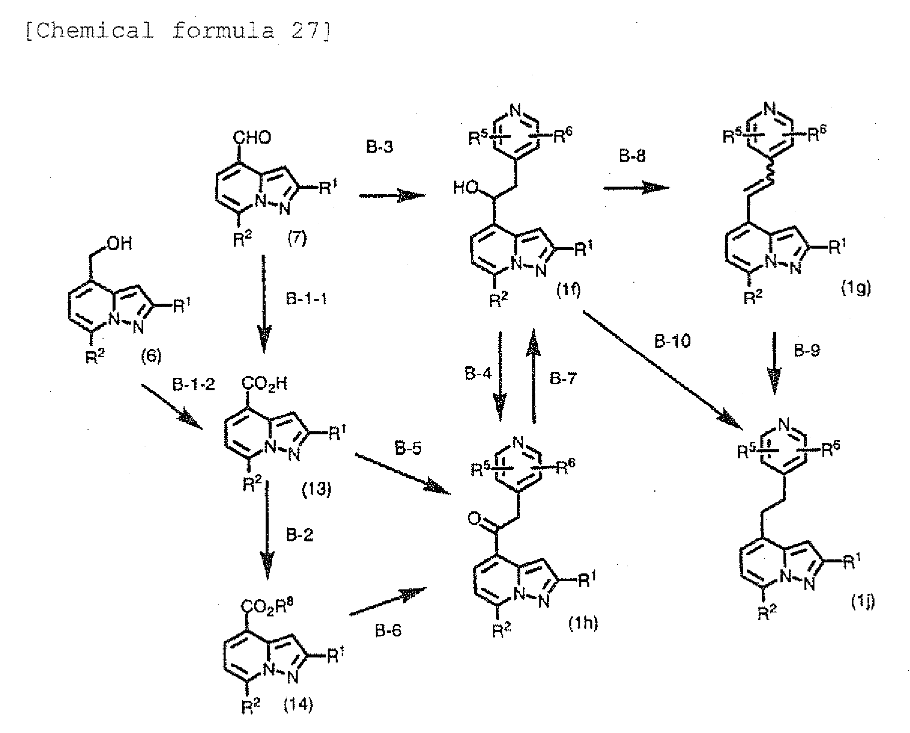

In the synthesis route B, the compound represented by the general formula (13) can be produced by oxidizing the compound represented by the general formula (7) (step B-1-1) or by oxidizing the compound represented by the general formula (6) (step B-1-2).

The oxidation reaction in the step B-1-1 may be carried out using any method commonly used to oxidize aldehydes to carboxylic acids. For example, the oxidizing reaction may be carried out by air oxidation, oxygen oxidation, or oxidation by a chromium oxide-pyridine complex (such as pyridinium chlorochromate or pyridiniumdichromate), chromium oxide, silveroxide, silvernitrate, potassium permanganate, ruthenium oxide, sodium periodate with ruthenium as a catalyst, iodosobenzene with ruthenium as a catalyst, sodium chlorite, bleaching powder, hydrogen peroxide, chlorine, or N-bromosuccinimide. The reaction may be performed at a temperature of 0°C to 100°C.

The oxidation reaction in the step B-1-2 may be carried out using any method commonly used to oxidize alcohols to carboxylic acids. For example, the reaction may be carried out by oxygen oxidation or oxidation by chromic acid, potassium chromate, a chromium oxide-pyridine complex (such as pyridinium chlorochromate or pyridinium dichromate), potassium permanganganate, ruthenium oxide, sodium periodate with ruthenium as a catalyst, silver oxide, bleaching powder, or hydrogen peroxide. The reaction may be performed at a temperature of 0°C to 100°C.

In the synthesis route B, the compound represented by the general formula (14):

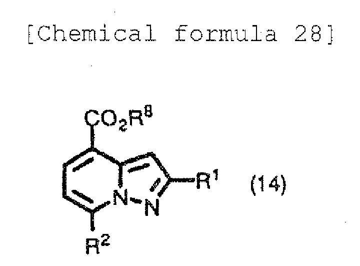

[wherein R⁸ is an alkyl group having 1 to 6 carbon atoms, and R¹ and R² are as described above] can be produced by esterifying the compound represented by the general formula (13) (step B-2).

The reaction may be carried out using any method commonly used to convert a carboxylic acid to an ester. For example, the compound represented by the general formula (14) can be produced by adding sulfuric acid or hydrochloric acid under heating to reflux using the compound represented by the general formula (15):

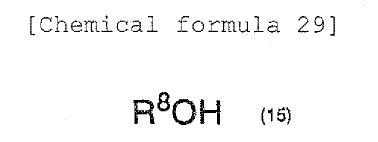

[wherein R⁸ is as defined above] as a solvent. The compound represented by the general formula (14) can also be produced by reacting with the compound represented by the general formula (15) in a solvent such as DMF, methylene chloride, chloroform, benzene, toluene, THF, or 1,4-dioxane at 0°C to room temperature using a condensation agent such as diethyl azodicarboxylate or DCC.

Moreover, the compound represented by the general formula (14) can also be produced by converting the compound represented by the general formula (13) to an acid chloride using thionyl chloride, oxalyl chloride, or the like and reacting the resultant product with the compound represented by the general formula (15) in a solvent such as methylene chloride, chloroform, benzene, toluene, THF, or 1,4-dioxane at 0°C to room temperature in the presence of a base such as triethylamine or pyridine.

In addition, the compound represented by the general formula (14) can also be produced in high yield by reacting the compound represented by the general formula (13) with the compound represented by the general formula (16):

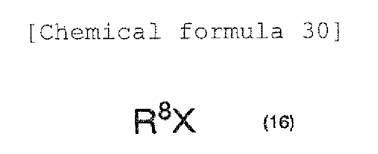

[wherein X is a halogen atom, and R⁸ is as defined above] in a solvent such as DMF, THF, or 1,4-dioxane at room temperature to 50°C using a base such as sodium carbonate, potassium carbonate, sodium hydrogen carbonate, potassium hydrogen carbonate, or lithium carbonate.

In the synthesis route B, the compound represented by the general formula (1f) can be produced by reacting the compound represented by the general formula (7) with the compound represented by the general formula (12) (step B-3) or by reduction of the compound represented by the general formula (1h) (step B-7).

The reaction via step B-3 may be carried out as in the step A-8.

The reaction via the step B-7 may be carried out in a reaction solvent such as THF, 1,4-dioxane, methanol, or ethanol at 0°C to room temperature using an alkylborane derivative such as a borane or 9-borabicyclo[3.3.1]nonane (9-BBN) or a metal-hydrogen complex compound such as diisobutylaluminum hydride (iBU)₂AlH), sodium borohydride (NaBH₄), lithium borohydride (LiBH₄), or lithium aluminum hydride (LiAlH₄).

In the synthesis route B, the compound represented by the general formula (1h) can be produced by oxidizing the compound represented by the general formula (1f) (step B-4), by reacting the compound represented by the general formula (13) with the compound represented by the general formula (12) (step B-5), or by reacting the compound represented by the general formula (14) with the compound represented by the general formula (12) (step B-6).

The reaction via the step B-4 may be carried out using any method commonly used to oxidize alcohols to aldehydes or ketones and may be carried out as in, for example, the step A-5.

The reaction via the step B-5 or the step B-6 may be carried out as in the step A-8.

In the synthesis route B, the compound represented by the general formula (1g) can be produced by dehydration of the compound represented by the general formula (1f) (step B-8).

Preferably, the reaction is carried out in a reaction solvent such as benzene, toluene, or xylene by adding, as a dehydrating agent, p-toluenesulfonic acid, pyridinium p-toluenesulfonate, sulfuric acid, or methanesulfonic acid, preferably methanesulfonic acid, at 70°C or under heating to reflux.

In the synthesis route B, the compound represented by the general formula (1j) can be produced by dehydroxylation of the compound represented by the general formula (1f) (step B-10) or by reduction of the compound represented by the general formula (1g) (step B-9).

The reaction in the step B-9 may be carried out in a solvent such as ethanol, methanol, THF, DMF, or ethyl acetate at room temperature under normal pressure or high hydrogen pressure in the presence of a catalytic reduction catalyst such as palladium-carbon, platinum-carbon, platinum oxide, rhodium-carbon, or ruthenium-carbon.

The reaction in the step B-10 can be easily carried out using the reaction procedure in the step B-9 described above with addition of hydrochloric acid or acetic acid. Moreover, the compound represented by the general formula (1j) can also be produced as follows. First, a sulfonate is formed by reacting with methanesulfonyl chloride or p-toluenesulfonyl chloride in a solvent such as THF at 0°C to room temperature in the presence of a base such as triethylamine or pyridine, and then the resultant product is reacted with LiAlH₄ in a solvent such as THF at 0°C to room temperature.

In the synthesis routes A and B, when R¹ is a difluoromethyl group, the compound represented by the general formula (6) is represented by the general formula (6c):

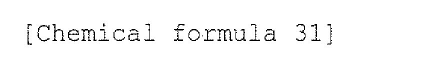

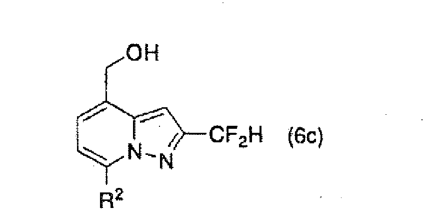

[wherein R² is as defined above], and the compound represented by the general formula (7) is represented by the general formula (7c) :

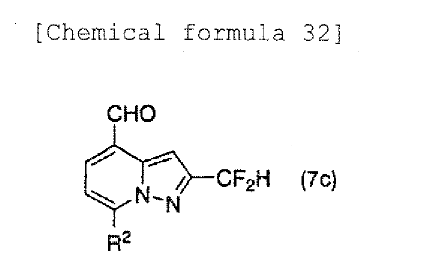

[wherein R² is as defined above]. These compounds can also be produced via the synthesis route C described below.

<Synthesis route C>

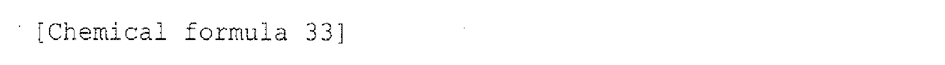

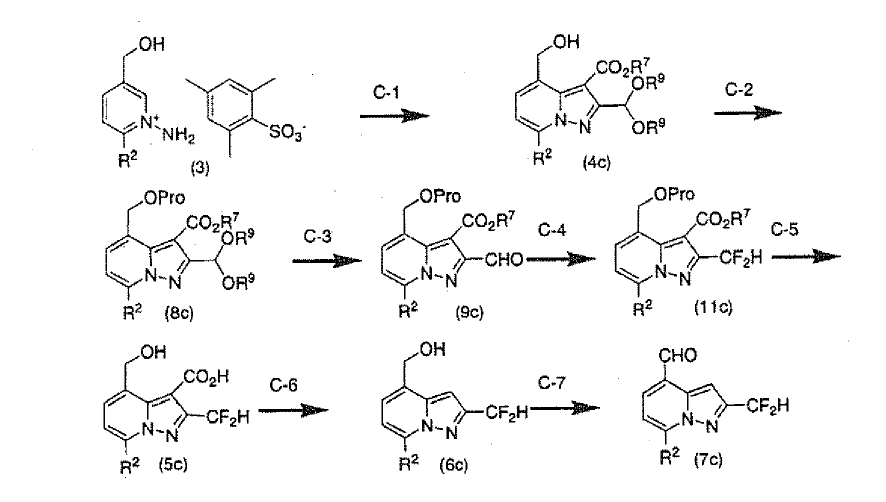

In the synthesis route C, the compound represented by the general formula (4c):

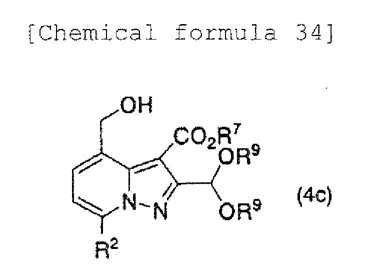

[wherein R⁹ is a lower alkyl group having 1 to 6 carbon atoms or two R⁹ groups are joined to form a methylene chain having 2 to 4 carbon atoms (the methylene chain may have a lower alkyl group having 1 to 4 carbon atoms), and R² and R⁷ are as described above] can be produced by allowing the compound represented by the general formula (3) and the compound represented by the general formula (10c):

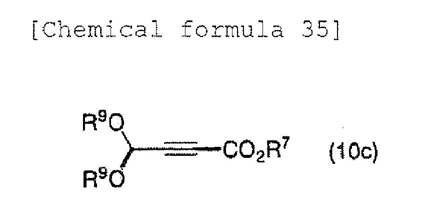

[wherein R⁷ and R⁹ are as described above] to act in the presence of a base (step C-1).

The reaction may be carried out as in the step A-2.

In the synthesis route C, the compound represented by the general formula (8c):

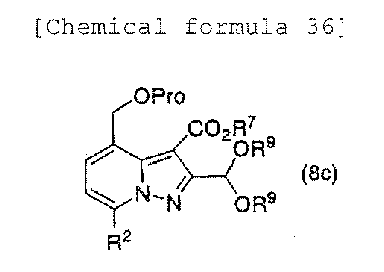

[wherein Pro is an alcohol protecting group such as a methoxymethyl group, a t-butyldimethylsilyl group, t-butyldiphenylsilyl group, a triisopropylsilyl group, a tetrahydropyranyl group, or an acetyl group, and R², R⁷, and R⁹ are as described above] can be produced by subjecting the compound represented by the general formula (4c) to various alcohol protecting group-introducing reactions (step C-2).
For example, when a methoxymethyl group is introduced, the reaction may be carried out by allowing the compound represented by the general formula (4c) and methoxymethyl chloride or methoxymethyl bromide to act in THF, acetonitrile, or methylene chloride at 0°C to room temperature in the presence of a base such as sodium hydride, triethylamine, or diisopropylethylamine. When a t-butyldimethylsilyl group, a t-butyldiphenylsilyl group, or a triisopropylsilyl group is introduced, the reaction may be carried out by allowing the compound represented by the general formula (4c) and one of silyl chloride, silyl bromide, and silyl trifluoromethanesulfonate to act in a solvent such as THF, CPME, DMF, acetonitrile, or methylene chloride at 0°C to room temperature in the presence of a base such as triethylamine or imidazole. When a tetrahydropyranyl group is introduced, it is preferable that the reaction be carried out by allowing the compound represented by the general formula (4c) and dihydropyran to act in a solvent such as methylene chloride at 0°C to room temperature in the presence of an acid catalyst such as p-toluenesulfonic acid. When an acetyl group is introduced, the reaction may be carried out by reacting the compound represented by the general formula (4c) with acetyl chloride, acetyl bromide, or acetic anhydride in a solvent such as THF, 1,4-dioxane, or methylene chloride at 0°C to room temperature in the presence of an organic base such as triethylamine, diisopropylethylamine, or pyridine. In this case, the reaction may be carried out in pyridine or the like which also serves as a base.
In the synthesis route C, the compound represented by the general formula (9c) can be produced by subjecting the compound represented by the general formula (8c) to commonly used conversion reaction of an acetal group to a formyl group (step C-3).

The reaction may be carried out in an acetone solvent using an acid catalyst such as p-toluenesulfonic acid monohydrate or pyridinium p-toluenesulfonate at room temperature or under heating and reflux. Alternatively, the reaction may be carried out at 0°C to room temperature using methanol, ethanol, ethyl acetate, diethyl ether, or the like each containing hydrogen chloride.

In the synthesis route C, the compound represented by the general formula (11c) can be produced by subjecting the compound represented by the general formula (9c) to fluorination (step C-4).

The reaction may be carried out in a solvent such as dichloromethane at 0°C to room temperature using a fluorinating agent such as dimethylaminosulfur trifluoride or diethylaminosulfur trifluoride.

In the synthesis route C, the compound represented by the general formula (5c) can be produced by subjecting the compound represented by the general formula (11c) to commonly used deprotection reaction of alcohol-protecting groups and to commonly used hydrolysis reaction of esters (step C-5).

When the protecting group is a methoxymethyl group or a tetrahydropyranyl group, the deprotection reaction of the alcohol-protecting group may be carried out in a solvent such as methanol, ethanol, ethyl acetate, or diethyl ether each containing hydrogen chloride at 0°C to room temperature. When the protecting group is a silyl group such as a t-butyldimethylsilyl group, a t-butyldiphenylsilyl group, or a triisopropylsilyl group, the reaction may be carried out in a solvent such as acetonitrile or THF at 0°C to room temperature using potassium fluoride, cesium fluoride, or tetrabutylammonium fluoride. When the protecting group is an acetyl group, the reaction may be carried out in a solvent such as THF, CPME, methanol, ethanol, or 1,4-dioxane at 0°C to room temperature using an aqueous sodium, potassium, or lithium hydroxide solution.

The ester hydrolysis reaction may be carried out in a solvent such as methanol, ethanol, THF, CPME, DMSO, DMF, or 1,4-dioxane by adding an aqueous potassium, sodium, or lithium hydroxide solution, preferably an aqueous sodium hydroxide solution at room temperature or under heating and reflux.

In the synthesis route C, the compound represented by the general formula (6c) can be produced by decarboxylation of the compound represented by the general formula (5c) (step C-6). The reaction may be carried out as in the step A-4.

In the synthesis route C, the compound represented by the general formula (7c) can be produced by oxidation of the compound represented by the general formula (6c) (step C-7).

The reaction may be carried out using any method commonly used to oxidize alcohols to aldehydes or ketones and may be carried out as in, for example, the step A-5.

In the synthesis route B, when R¹ is a difluoromethyl group, the compound of general formula (13) is represented by the general formula (13c):

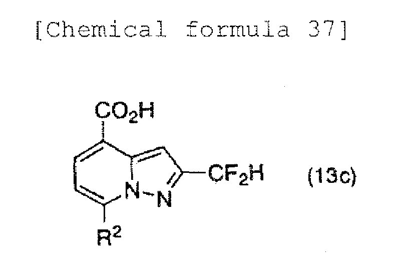

[wherein R² is as defined above] and this compound can also be produced via the synthesis route C' described below.

<Synthesis route C'>

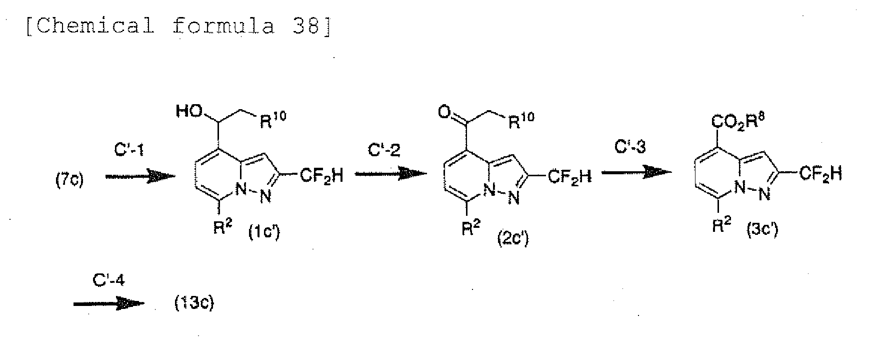

In the synthesis route C', the compound represented by the general formula (1c'):

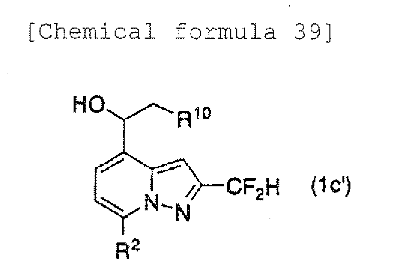

[wherein R¹⁰ is a hydrogen atom or a lower alkyl group having 1 to 5 carbon atoms, and R² is as defined above] can be produced by reacting the compound represented by the general formula (7c) with the compound represented by the general formula (17):

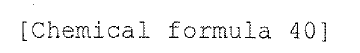

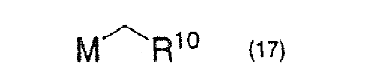

[wherein R¹⁰ and M are as described above] (step C'-1).

Preferably, the reaction is carried out by mixing the compounds represented by the general formulas (7c) and (17) in a solvent such as THF, 1,4-dioxane, or ether at -78°C to 0°C and slowly heating the mixture to room temperature.

In the synthesis route C', the compound represented by the general formula (2c') can be produced by oxidizing the compound represented by the general formula (1c') (step C'-2).

The reaction may be carried out using any method commonly used to oxidize alcohols to aldehydes or ketones and may be carried out as in, for example, the step A-5.

In the synthesis route C', the compound represented by the general formula (3c') can be produced by allowing the compound represented by the general formula (2c') and the compound represented by the general formula (18):

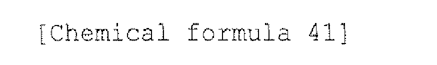

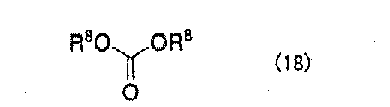

[wherein R⁸ is as defined above] to act in the presence of a base (step C'-3).

Preferably, the reaction is carried out using a solvent amount of the compound represented by the general formula (18) at an elevated temperature of 80°C to 120°C in the presence of a base such as sodium hydride, sodium alkoxide, potassium alkoxide, or potassium hydride, preferably sodium hydride.

In the synthesis route C', the compound represented by the general formula (13c) can be produced by subjecting the compound represented by the general formula (3c') to hydrolysis (step C'-4). The reaction may be carried out as in the step A-3.

In the synthesis routes A and B, when R¹ is a hydroxymethyl group, the compound represented by the general formula (7) is represented by the general formula (7d):

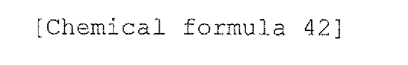

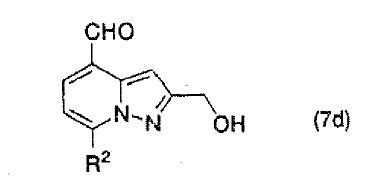

[wherein R² is as defined above], and the compound represented by the general formula (13) is represented by the general formula (13d):

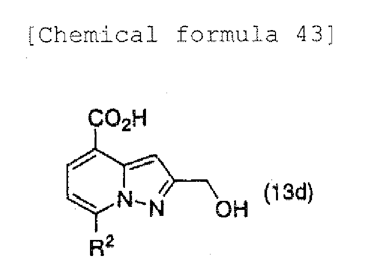

[wherein R² is as defined above]. These compounds can also be produced via the synthesis route D described below.

<Synthesis route D>

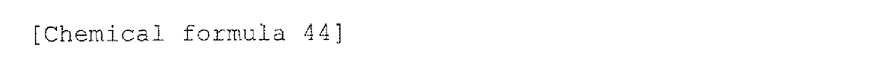

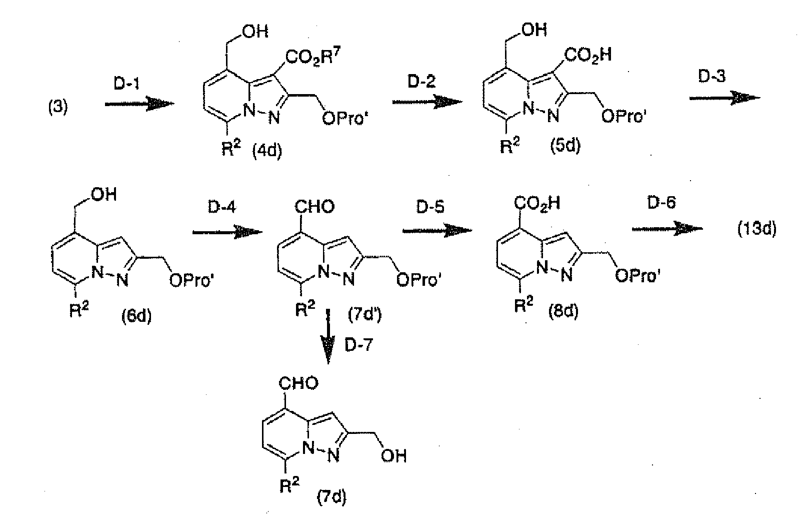

In the synthesis route D, the compound represented by the general formula (4d):

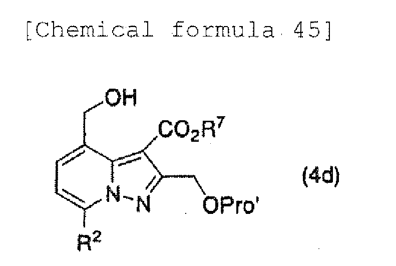

[wherein Pro' is an alcohol protecting group such as a methoxymethyl group, a t-butyldimethylsilyl group, a t-butyldiphenylsilyl group, a triisopropylsilyl group, or a tetrahydropyranyl group, and R² and R⁷ are as described above] can be produced by allowing the compound represented by the general formula (3) and the compound represented by the general formula (10d):

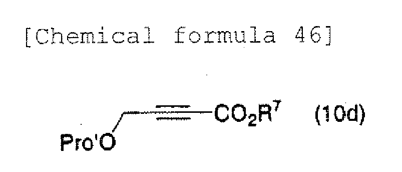

[wherein R⁷ and Pro' are as described above] to act in the presence of a base (step D-1).

The reaction may be carried out as in the step A-2.

In the synthesis route D, the compound represented by the general formula (5d) can be produced by subjecting the compound represented by the general formula (4d) to commonly used hydrolysis reaction of esters (step D-2). The reaction may be carried out as in the step A-3.

In the synthesis route D, the compound represented by the general formula (6d) can be produced by decarboxylation of the compound represented by the general formula (5d) (step D-3). The reaction may be carried out as in the step A-4.

In the synthesis route D, the compound represented by the general formula (7d') can be produced by oxidizing the compound represented by the general formula (6d) (step D-4).

The reaction may be carried out using any method commonly used to oxidize alcohols to aldehydes or ketones and may be carried out as in, for example, the step A-5.

In the synthesis route D, the compound represented by the general formula (8d) can be produced by oxidizing the compound represented by the general formula (7d') (step D-5) or by oxidizing the compound represented by the general formula (6d).

The oxidation reaction in the step D-5 may be carried out using any method commonly used to oxidize aldehydes to carboxylic acids and may be carried out as in, for example, the step B-1-1.

When the compound represented by the general formula (6d) is oxidized, any method commonly used to oxidize alcohols to carboxylic acids may be used, and the reaction may be carried out as in, for example, the step B-1-2.

In the synthesis route D, the compound represented by the general formula (13d) can be produced by subjecting the compound represented by the general formula (8d) to commonly used deprotection reaction of alcohol-protecting groups (step D-6).

When the protecting group is a methoxymethyl group or a tetrahydropyranyl group, the deprotection reaction may be carried out in a solvent such as methanol, ethanol, ethyl acetate, or diethyl ether each containing hydrogen chloride at 0°C to room temperature. When the protecting group is a silyl group such as a t-butyldimethylsilyl group, a t-butyldiphenylsilyl group, or a triisopropylsilyl group, the reaction may be carried out in a solvent such as acetonitrile or THF at 0°C to room temperature using potassium fluoride, cesium fluoride, or tetrabutylammonium fluoride.

In the synthesis route D, the compound represented by the general formula (7d) can be produced by subjecting the compound represented by the general formula (7d') to commonly used deprotection reaction of alcohol-protecting groups (step D-7). The deprotection reaction may be carried out as in the step D-6.

In the synthesis routes A and B, when R¹ is a cyano group, the compound represented by the general formula (6) is represented by the general formula (6e):

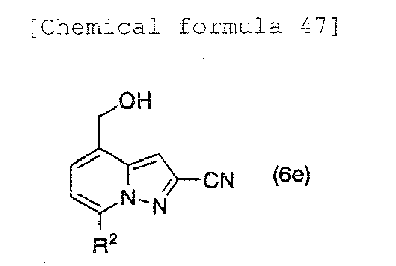

[wherein R² is as defined above]. When R¹ is an oxime group, the compound represented by the general formula (6) is represented by the general formula (6e'):

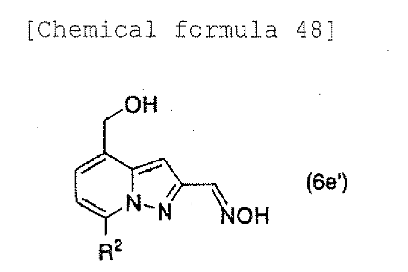

[wherein R² is as defined above]. These compounds can also be produced via synthesis route E described below.

<Synthesis route E>

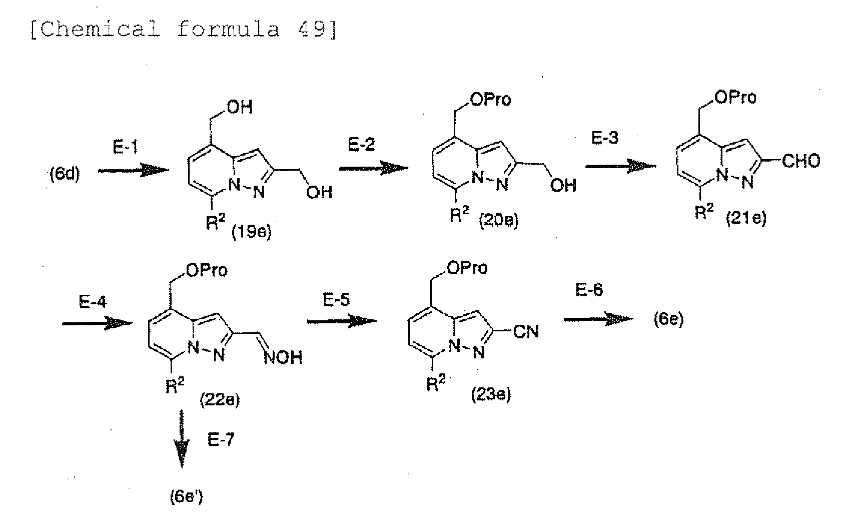

In the synthesis route E, the compound represented by the general formula (19e) can be produced by subjecting the compound represented by the general formula (6d) to commonly used deprotection reaction of alcohol-protecting groups (step E-1).

The deprotection reaction may be carried out as in, for example, the step D-6.

In the synthesis route E, the compound represented by the general formula (20e) can be produced by subjecting the compound represented by the general formula (19e) to various alcohol protecting group-introducing reactions (step E-2), or by subjecting the compound represented by the general formula (6d) to various alcohol protecting group-introducing reactions and then subjecting the resultant product to commonly used deprotection reaction of alcohol-protecting groups.

The various alcohol protecting group-introducing reactions in step E-2 may be carried out as in, for example, the step C-2.

The deprotection reaction of alcohol-protecting groups may be carried out as in, for example, the step D-6.

In the synthesis route E, the compound represented by the general formula (21e) can be produced by oxidizing the compound represented by the general formula (20e) (step E-3).

The reaction may be carried out using any method commonly used to oxidize alcohols to aldehydes or ketones and may be carried out as in, for example, the step A-5.

In the synthesis route E, the compound represented by the general formula (22e) can be produced by reacting the compound represented by the general formula (21e) with hydroxylamine or hydroxylamine hydrochloride in the presence or absence of a base (step E-4).

The reaction may be carried out in a solvent such as water, methanol, or ethanol at 0°C to 100°C using, as a base, sodium acetate, sodium carbonate, or the like.

In the synthesis route E, the compound represented by the general formula (23e) can be produced by subjecting the compound represented by the general formula (22e) to dehydration reaction (step E-5).
The reaction may be carried out in a solvent such as toluene, ether, THF, CPME, 1,4-dioxane, dichloromethane, chloroform, or pyridine at 0°C to 100°C using a dehydrating agent such as diphosphorus pentaoxide, phosphorus pentachloride, thionyl chloride, acetic anhydride, trifluoroacetic anhydride, DCC, N,N'-carbonyldiimidazole, or triphenylphosphine-carbon tetrachloride in the presence or absence of a base such as triethylamine, diisopropylethylamine, or pyridine.

The compound represented by the general formula (23e) may also be produced by converting the compound represented by the general formula (21e) to the compound represented by the general formula (22e) using the method of the step E-4 and subjecting the unisolated product to dehydration reaction according to the method of the step E-5.

In the synthesis route E, the compound represented by the general formula (6e) can be produced by subjecting the compound represented by the general formula (23e) to commonly used deprotection reaction of alcohol-protecting groups (step E-6).

The deprotection reaction may be carried out as in the step C-5.

In the synthesis route E, the compound represented by the general formula (6e') can be produced by subjecting the compound represented by the general formula (22e) to commonly used deprotection reaction of alcohol-protecting groups (step E-7).

The deprotection reaction may be carried out as in, for example, the step C-5.

In the synthesis route B, when R¹ is a methyl group optionally substituted with an alkoxy group having 1 to 6 carbon atoms, the compound represented by the general formula (13) is represented by the general formula (13f):

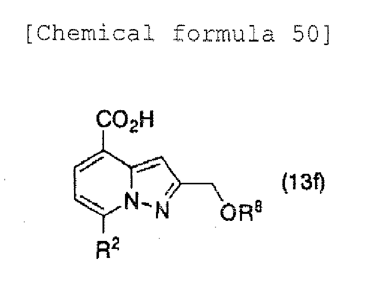

[wherein R² and R⁸ are as described above], and this compound can also be produced via the synthesis route F described below.

<Synthesis route F>

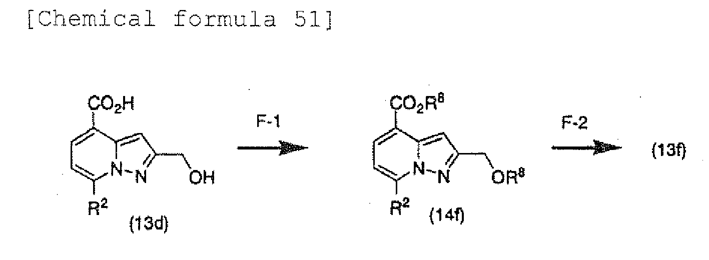

In the synthesis route F, the compound represented by the general formula (14f):

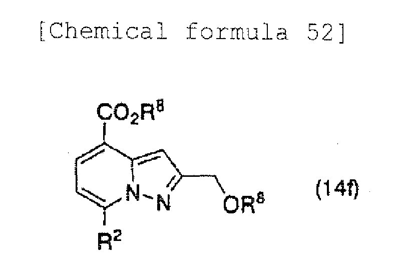

[wherein R² and R⁸ are as described above] can be produced by allowing the compound represented by the general formula (13d) and the compound represented by the general formula (16) to act in the presence of a base (step F-1). The reaction may be carried out in a solvent such as toluene, THF, CPME, acetonitrile, DMF, or DMSO at 0°C to 100°C using a base such as sodium hydride, potassium hydride, lithium hydroxide, sodium hydroxide, potassium hydroxide, sodium carbonate, potassium carbonate, silver carbonate, or silver oxide.

In the synthesis route F, the compound represented by the general formula (13f) can be produced by subjecting the compound represented by the general formula (14f) to commonly used hydrolysis reaction of esters (step F-2).

The reaction may be carried out as in the step A-3.

In the synthesis routes A and B, when R² is an alkoxy group having 1 to 6 carbon atoms or an alkylsulfanyl group having 1 to 6 carbon atoms, the compound represented by the general formula (7) is represented by the general formula (7g):

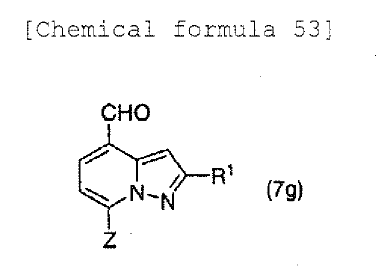

[wherein Z is an alkoxy group having 1 to 6 carbon atoms or an alkylsulfanyl group having 1 to 6 carbon atoms, and R¹ is as defined above], and this compound can also be produced via the synthesis route G described below.

<Synthesis route G>

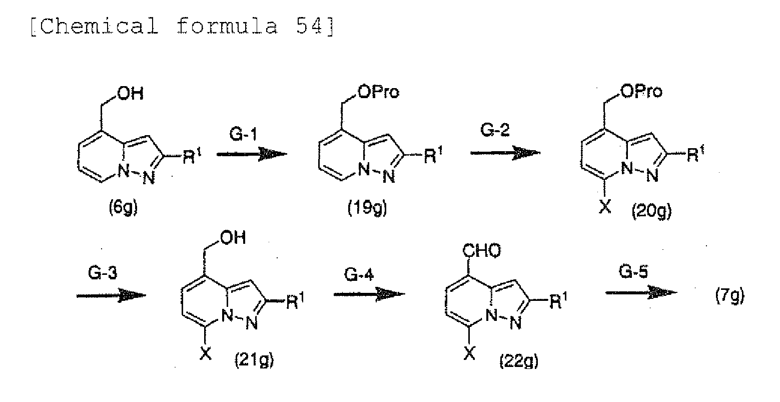

In the synthesis route G, the compound represented by the general formula (19g) can be produced by subjecting the compound represented by the general formula (6g), or the compound represented by the general formula (6) with R² being a hydrogen atom, to alcohol protecting group-introducing reaction (step G-1). The reaction may be carried out as in the step C-2.

In the synthesis route G, the compound represented by the general formula (20g) can be produced by halogenation of the compound represented by the general formula (19g) (step G-2).

The reaction may be carried out by reacting the compound represented by the general formula (19g) with a base such as butyllithium, lithium diisopropylamide, or lithium bis (trimethylsilyl) amide in a solvent such as THF or CPME at -78°C to 0°C and subsequently reacting the resultant product with a halogenating agent such as N-fluorobenzenesulfonimide, N-chlorosuccinimide, N-bromosuccinimide, 1,2-dibromoethane, bromine, N-iodosuccinimide, iodine, or 1,2-diiodoethane at -78°C to room temperature.

In the synthesis route G, the compound represented by the general formula (21g) can be produced by subjecting the compound represented by the general formula (20g) to commonly used deprotection reaction of alcohol-protecting groups (step G-3).

The reaction may be carried out as in step C-5.

In the synthesis route G, the compound represented by the general formula (22g) can be produced by oxidizing the compound represented by the general formula (21g) (step G-4).

The reaction may be carried out using any method commonly used to oxidize alcohols to aldehydes or ketones and may be carried out as in, for example, the step A-5.

In the synthesis route G, the compound represented by the general formula (7g) can be produced by alkoxylation or sulfanylation of the compound represented by the general formula (22g) (step G-5).

The reaction may be carried out by adding a base such as sodium hydride or potassium hydride to a corresponding alcohol or thiol (ZH) compound in a solvent such as DMF, THF, CPME, or DMSO, preferably DMF, at room temperature to 60°C.

In the synthesis routes A and B, when R² is an amino group or an alkylamino group having 1 to 6 carbon atoms, the compound represented by the general formula (7) is represented by the general formula (7h):

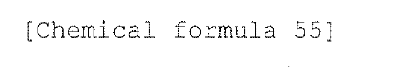

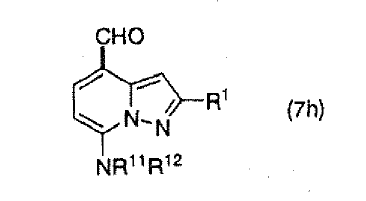

[wherein R¹¹ and R¹² are each independently a hydrogen atom, an alkyl group having 1 to 6 carbon atoms, or an amino protecting group, and R¹ is as defined above], and this compound can be produced by using the compound represented by the general formula (22g) and the compound represented by the general formula (24):

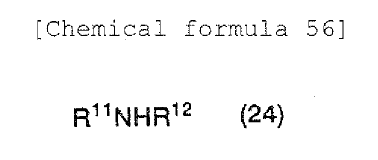

[wherein R¹¹ and R¹² are as described above].

The reaction may be carried out in a solvent such as THF, CPME, DMSO, or DMF at 0°C to 100°C in the presence or absence of butyllithium, sodium hydride, potassium hydride, or the like. When one or both of R¹¹ and R¹² in the compound represented by the general formula (7h) are amino-protecting groups, this compound may also be produced by first producing the compound represented by the general formula (7h) in which one or both of R¹¹ and R¹² are hydrogen atoms and subsequently subjecting the produced compound to general amino protection reaction. Examples of the general amino-protecting group include amino-protecting groups described in "PROTECTIVE GROUPS IN ORGANIC SYNTHESIS THIRD EDITION (Theodora W. Greene, Peter G. M. Wats, JOHN WILEY & SONS, INC.)." Preferred examples include a t-butoxycarbonyl group. When a t-butoxycarbonyl group is introduced as the amino-protecting group, the reaction may be carried out using di-t-butyldicarbonate in a solvent such as THF, CPME, DMSO, DMF, or acetonitrile at a reaction temperature of 0°C to 100°C in the presence or absence of 4-dimethylaminopyridine or the like.

In the synthesis routes A and B, when R² is a hydroxymethyl group, the compound represented by the general formula (14) is represented by the general formula (14j-1):

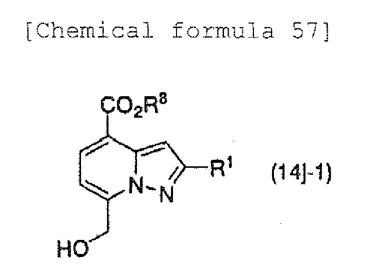

[wherein R¹ and R⁸ are as described above] . When R² is a formyl group, the compound represented by the general formula (14) is represented by the general formula (14j-2):

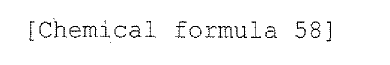

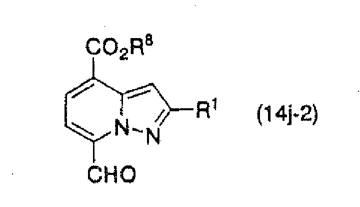

[wherein R¹ and R⁸ are as described above]. When R² is a 1-hydroxyalkyl group having 2 to 6 carbon atoms, the compound represented by the general formula (14) is represented by the general formula (14j-3):

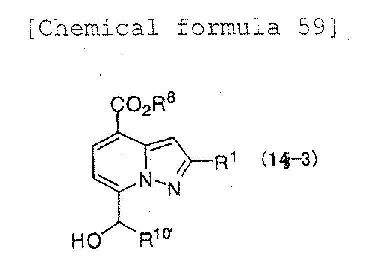

[wherein R^{10'} is an alkyl group having 1 to 5 carbon atoms and R¹ and R⁸ are as described above]. When R² is an alkanoyl group having 2 to 6 carbon atoms, the compound represented by the general formula (14) is represented by the general formula (14j-4):

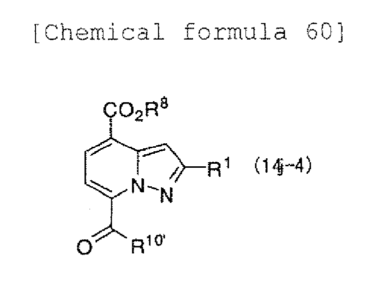

[wherein R¹, R⁸, and R^{10'} are as described above]. The compounds represented by the general formulas (14j-1) to (14j-4) can also be produced via the synthesis route J described below.

<Synthesis route J>

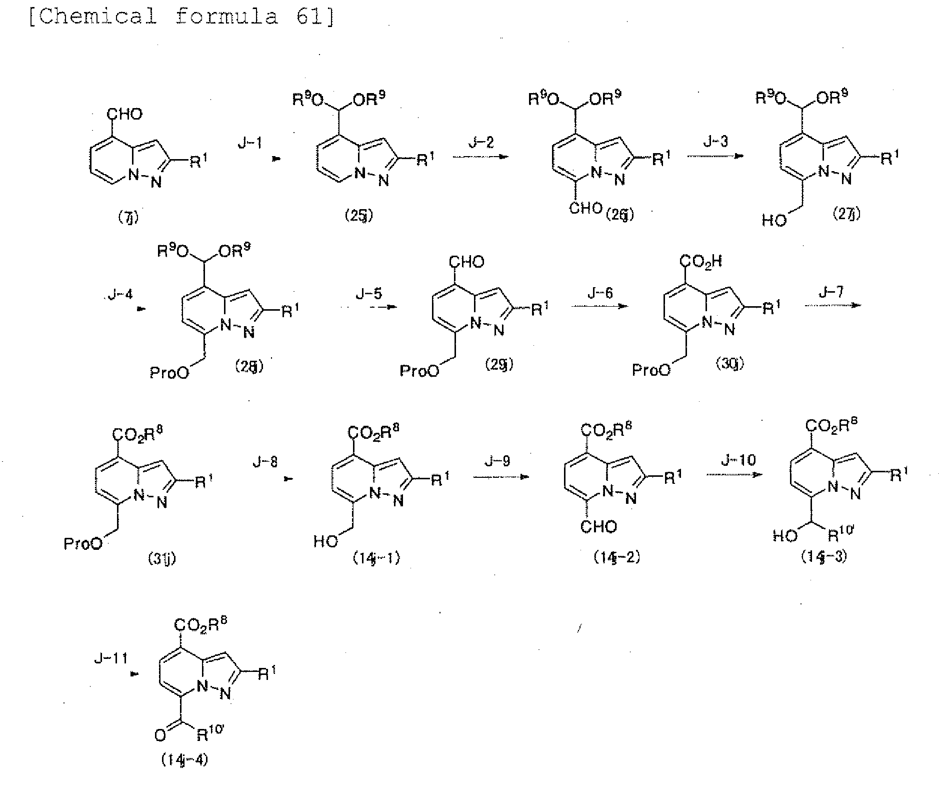

In the synthesis route J, the compound represented by the general formula (25j) can be produced by acetalization of the compound represented by the general formula (7j), or the compound represented by the general formula (7) with R² being a hydrogen atom, with the compound represented by the general formula (32):

[Chemical formula 62] **V-OR⁹ (32)**

[wherein V is a hydrogen atom or a trialkylsilyl group, and R⁹ is as defined above] (step J-1).

The reaction may be carried out in a solvent such as benzene, toluene, xylene, or methylene chloride at 0°C to 150°C in the presence of a catalyst such as hydrogen chloride, sulfuric acid, p-toluenesulfonic acid, pyridinium p-toluenesulfonate, camphorsulfonic acid, trimethylsilyl methanesulfonate, montmorillonite K-10, or acidic ion-exchange resin.

In the synthesis route J, the compound represented by the general formula (26j) can be produced by formylation of the compound represented by the general formula (25j) (step J-2).

The reaction may be carried out by reacting with a base such as butyllithium, lithium diisopropylamide, or lithium bis (trimethylsilyl) amide, preferably lithium diisopropylamide, in a THF solvent at -78°C and subsequently reacting the resultant product with ethyl formate or DMF at -78°C to room temperature.

In the synthesis route J, the compound represented by the general formula (27j) can be produced by reduction of the compound represented by the general formula (26j) (step J-3).

The reaction may be carried out by reacting with a reducing agent such as sodium borohydride, lithium borohydride, DIBAL, or lithium aluminum hydride at 0°C to room temperature. The reaction is carried out in a reaction solvent. When sodium borohydride is used, an ether-based solvent such as THF, CPME, or 1,4-dioxane or an alcohol-based solvent such as ethanol or methanol is preferably used as the reaction solvent. When lithium borohydride is used, THF or a solvent prepared by adding an alcohol-based solvent such as ethanol to THF is preferably used as the reaction solvent. When DIBAL is used, THF, toluene, methylene chloride, or the like is preferably used as the reaction solvent. When lithium aluminum hydride is used, an ether-based solvent such as THF or diethyl ether is preferably used as the reaction solvent.

In the synthesis route J, the compound represented by the general formula (28j) can be produced by subjecting the compound represented by the general formula (27j) to various alcohol protecting group-introducing reactions (step J-4).

The reaction may be carried out as in the step C-2.

In the synthesis route J, the compound represented by the general formula (29j) can be produced by deacetalization of the compound represented by the general formula (28j) (step J-5).

The reaction may be carried out in acetone solvent with an acid catalyst such as p-toluenesulfonic acid monohydrate or pyridinium p-toluenesulfonate at room temperature or under heating and reflux or may be carried out by using methanol, ethanol, ethyl acetate, or diethyl ether each containing hydrogen chloride at 0°C to room temperature.

In the synthesis route J, the compound represented by the general formula (30j):

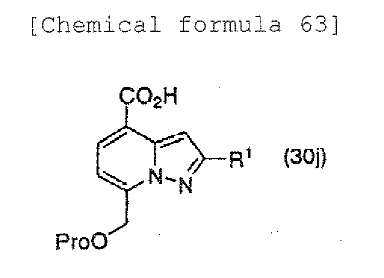

[wherein R¹ and Pro are as described above] can be produced by oxidizing the compound represented by the general formula (29j) (step J-6).

The reaction may be carried out using any method commonly used to oxidize aldehydes to carboxylic acids and may be carried out as in, for example, the step B-1-1.

In the synthesis route J, the compound represented by the general formula (31j) can be produced by esterification of the compound represented by the general formula (30j) (step J-7).

The reaction may be carried out as in the step B-2.

In the synthesis route J, the general formula (14j-1) can be produced by subjecting the compound represented by the general formula (31j) to commonly used deprotection reaction of alcohol-protecting groups (step J-8).

The reaction may be carried out as in the step C-5.

In the synthesis route J, the compound represented by the general formula (14j-2) can be produced by oxidizing the compound represented by the general formula (14j-1) (step J-9).

The reaction may be carried out using any method commonly used to oxidize alcohols to aldehydes or ketones and may be carried out as in, for example, the step A-5.

In the synthesis route J, the compound represented by the general formula (14j-3) can be produced by reacting the compound represented by the general formula (14j-2) with the compound represented by the general formula (33):

[Chemical formula 64] M-R^{10'} (33)

[wherein R^{10'} and M are as described above] (step J-10).

The reaction may be carried out in a reaction solvent such as THF, CPME, ether, or 1,4-dioxane at a reaction temperature of -78°C to room temperature.

In the synthesis route J, the compound represented by the general formula (14j-4) can be produced by oxidizing the compound represented by the general formula (14j-3) (step J-11).

The reaction may be carried out using any method commonly used to oxidize alcohols to aldehydes or ketones and may be carried out as in, for example, the step A-5.

In the synthesis routes A and B, when R² is an alkoxymethyl group having 1 to 6 carbon atoms, the compound represented by the general formula (6) is represented by the general formula (6k):

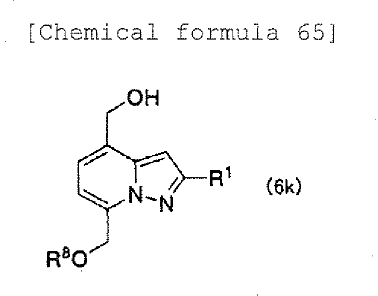

[wherein R¹ and R⁸ are as described above], and this compound can also by produced via the synthesis route K described below.

<Synthesis route K>

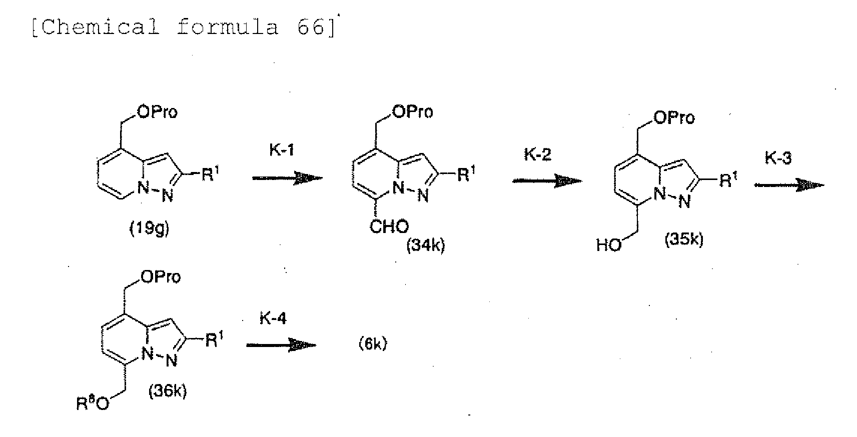

In the synthesis route K, the compound represented by the general formula (34k) can be produced by formylation of the compound represented by the general formula (19g) (step K-1).

The reaction may be carried out as in the step J-2.

In the synthesis route K, the compound represented by the general formula (35k) can be produced by reduction of the compound represented by the general formula (34k) (step K-2).

The reaction may be carried out as in the step J-3.

In the synthesis route K, the compound represented by the general formula (36k) can be produced by reacting the compound represented by the general formula (35k) with the compound represented by the general formula (16) in the presence of a base (step K-3).

The reaction may be carried out in a solvent such as toluene, THF, CPME, acetonitrile, DMF, or DMSO at 0°C to 100°C using, as a base, sodium hydride, potassium hydride, lithium hydroxide, sodium hydroxide, potassium hydroxide, sodium carbonate, potassium carbonate, silver carbonate, silver oxide, or the like.

In the synthesis route K, the compound represented by the general formula (6k) can be produced by subjecting the compound represented by the general formula (36k) to commonly used deprotection reaction of alcohol-protecting groups (step K-4).

The reaction may be carried our as in the step C-5.

When R¹³ is a hydrogen atom and n is 1, the compound represented by the general formula (1) is represented by the general formula (1k):

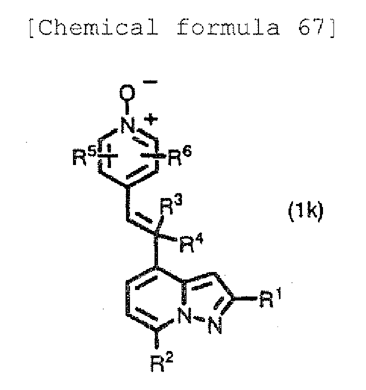

[wherein R¹, R², R³, R⁴, R⁵, R⁶, and

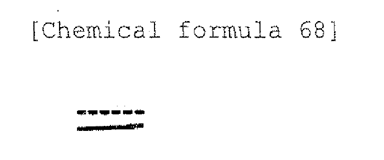

are as described above]. This compound can be produced from the compound represented by the general formula (1) with R¹³ being a hydrogen atom, i. e. , the compound represented by the general formula (1m):

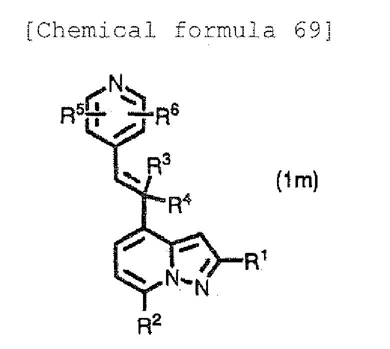

[wherein R¹, R², R³, R⁴, R⁵, R⁶, and

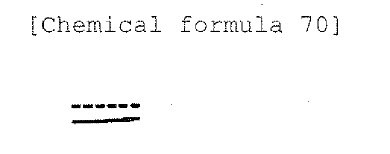

are as described above].

The reaction may be carried out in a solvent such as water, acetic acid, methylene chloride, chloroform, or 1,2-dichloroethane at a reaction temperature of 0°C to 150°C using hydrogen peroxide, m-chloroperbenzoic acid, peracetic acid, peroxymaleic acid, magnesium monoperoxyphthalate, sodium peroxyborate, or the like.
When R¹³ is a halogen atom, the compound represented by the general formula (1) is represented by the general formula (1n):

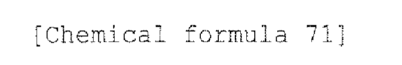

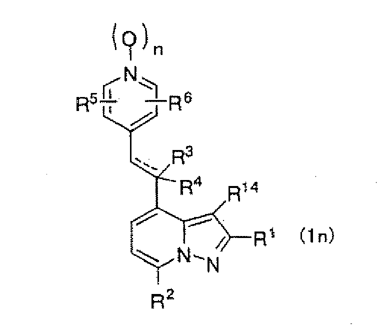

[wherein R¹⁴ is a halogen atom and R¹, R², R³ R⁴, R⁵, R⁶, n, and

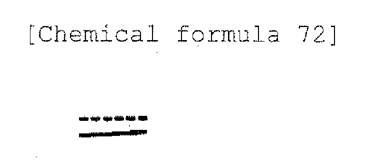

are as described above]. This compound can be produced from the compound represented by the general formula (1) with R¹³ being a hydrogen atom, i.e., the compound represented by the general formula (1p):

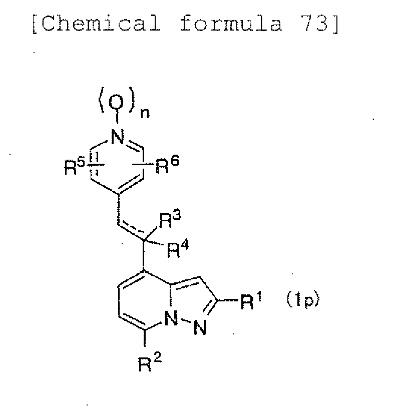

[wherein R¹, R², R³, R⁴, R⁵, R⁶, n, and

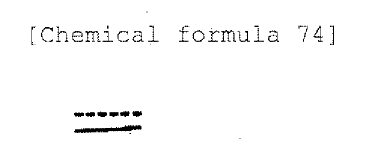

are as described above].

The reaction may be carried out by reacting the compound represented by the general formula (1p) with sodium hypochlorite, sodium chlorite, bleaching powder, chlorine, N-chlorosuccinimide, bromine, N-bromosuccinimide, iodine, N-iodosuccinimide, or the like in a solvent such as DMF at 0°C to 100°C.

### Examples

The present invention will next be described by way of Examples, but the invention is not limited thereto.

### <Example 1>

5-Hydroxymethyl-2-methoxypyridine

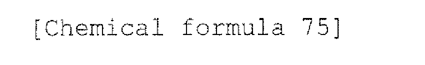

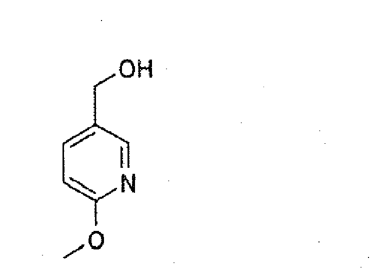

A solution of 6-methoxynicotinic acid methyl ester (50.0 g) in THE (300 mL) was added to a suspension of lithium aluminum hydride (11.4 g) in THF (600 mL) at 0°C, and the mixture was stirred at 0°C for 1 hour. A 10% aqueous sodium hydroxide solution (25.0 mL) was added to the reaction mixture, and then the resultant mixture was dried by adding anhydrous sodium sulfate. Subsequently, insoluble material was removed by filtration through Celite. The solvent of the filtrate was evaporated under reduced pressure to obtain the target product (41.1 g) as a colorless oil.
¹H-NMR (400 MHz, CDCl₃): δ 1.75 (1H, brs), 3.94 (3H, s), 4.62 (2H, s), 6.75 (1H, d, J = 8.0 Hz), 7.62 (1H, dd, J = 2.4, 8.0 Hz), 8.12 (1H, d, J= 2.4 Hz).

### <Example 2>

1-Amino-5-hydroxymethyl-2-methoxypyridinium 2,4,6-trimethylbenzenesulfonate

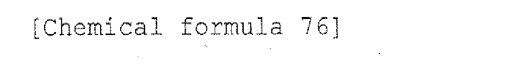

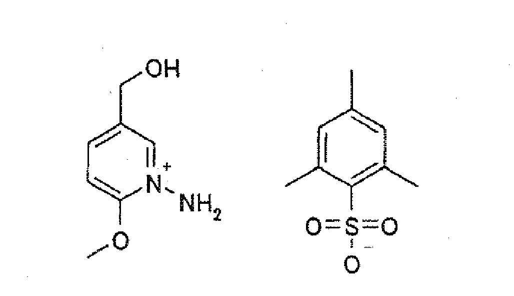

Ethyl O-mesitylsulfonylacetohydroxamate (87.8 g) was dissolved in 1,4-dioxane (70 mL). A 70% aqueous perchloric acid solution (31.0 mL) was added to the prepared solution under cooling with ice, and the mixture was stirred. Iced water was added to the mixture, and the precipitated solid was collected by filtration. The collected solid was dissolved in dichloromethane, and the dichloromethane layer was dried over anhydrous magnesium sulfate. The resultant product was added dropwise to a solution of the compound of Example 1 (35.7 g) in dichloromethane (20 mL), and the mixture was stirred at room temperature for 1 hour. The solvent of the reaction mixture was evaporated under reduced pressure, and the precipitated solid was collected by filtration. The collected solid was washed with diethyl ether to obtain the target product (58.7 g) as a white solid.
¹H-NMR (400 MHz, DMSO-d₆): δ 2.15 (3H, s), 2.49 (6H, s), 4.24 (3H, s), 4.57 (2H, s), 6.74 (2H, s), 7.70 (1H, d, J = 9.2 Hz), 7.71 (2H, br s), 8.16 (1H, dd, J = 9.2, 1.4 Hz), 8.46 (1H, d, J = 1.4 Hz).

### <Example 3>

1-Amino-3-hydroxymethylpyridinium 2,4,6-trimethylbenzenesulfonate

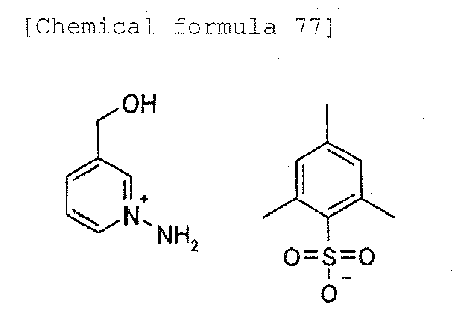

The same procedure as in Example 2 was followed using ethyl O-mesitylsulfonylacetohydroxamate (33.5 g) and 3-hydroxymethylpyridine (11.2 g) to obtain the target product (38.2 g) as a yellow oil.
¹H-NMR (400 MHz, DMSO-d₆): δ 2.33 (3H, s), 2.50 (6H, s), 4.69 (2H, s), 5.86 (1H, brs), 6.74 (2H, s), 7.96 (1H, dd, J = 8.0, 6.1 Hz), 8.15 (1H, d, J = 8.0 Hz), 8.50 (2H, s), 8.66 (1H, d, J = 6.1Hz), 8.71 (1H, s).

### <Example 4>

2-Ethyl-4-hydroxymethyl-7-methoxypyrazolo[1,5-a]pyridine-3-car boxylic acid ethyl ester

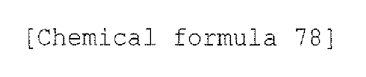

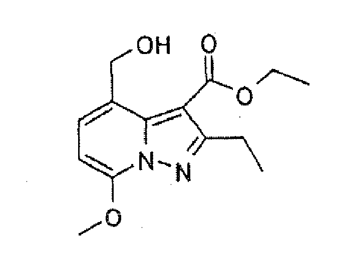

The compound of Example 2 (66.2 g) was dissolved in DMF (300 mL), and 2-pentynoic acid ethyl ester (16.4 mL) and potassium carbonate (51. 4 g) were added to the prepared solution. The mixture was stirred at room temperature for 23 hours. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1 -> ethyl acetate) to obtain the target product (6.70 g) as a white solid.
MS (EI⁺): 278 [M⁺]
¹H-NMR (400 MHz, CDCl₃): δ 1.34 (3H, t, J = 8.0 Hz), 1.44 (3H, t, J= 6.7 Hz), 3.12 (2H, q, J = 8.0 Hz), 4.16 (3H, s), 4.41 (2H, q, J = 6.7 Hz), 4.81 (2H, d, J = 7.3 Hz), 4.94 (1H, t, J = 7.3 Hz), 6.22 (1H, d, J = 7.3 Hz), 7.30 (1H, d, J = 7.3 Hz).

### <Example 5>

4-Hydroxymethyl-7-methoxy-2-trifluoromethylpyrazolo[1,5-a]pyri dine-3-carboxylic acid ethyl ester

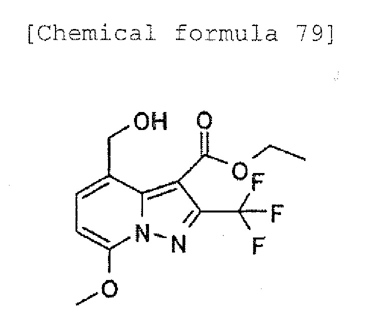

The same procedure as in Example 4 was followed using the compound of Example 2 and 4,4,4-trifluoro-2-butynoic acid ethyl ester to obtain the target product as a yellow powder.
¹H-NMR (400 MHz, CDCl₃): δ 1.42 (3H, t, J = 7.0 Hz), 4.20 (3H, s), 4.43 (2H, q, J = 7.0 Hz), 4.62 (1H, t, J = 7.6 Hz), 4.83 (2H, d, J = 7.6 Hz), 6.36(1H, d, J = 7.6 Hz), 7.44 (1H, d, J = 7.6 Hz).

### <Example 6>

2-Cyclopropyl-4-hydroxymethyl-7-methoxypyrazolo[1,5-a]pyridine -3-carboxylic acid benzyl ester

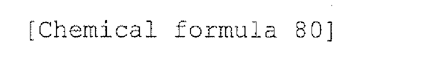

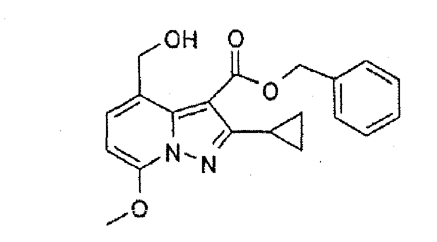

The same procedure as in Example 4 was followed using the compound of Example 2 (21.3 g) and cyclopropyl propynoic acid benzyl ester (8.01 g) to obtain the target product (5.07 g) as a white solid.
MS (EI⁺): 352 [M⁺]
¹H-NMR (440 MHz, CDCl₃): δ 0.82 (2H, m), 1.06-1.10 (2H, m), 2.49-2.56 (1H, m), 4.11 (3H, s), 4.81 (2H, s), 5.42 (2H, s), 6.19 (1H, d, J = 7.9 Hz), 7.29 (1H, d, J = 7.9 Hz), 7.33-7.41 (3H, m), 7.45-7.49 (2H, m).

### <Example 7>

4-Hydroxymethyl-2-isopropyl-7-methoxypyrazolo[1,5-a]pyridine-3 -carboxylic acid benzyl ester

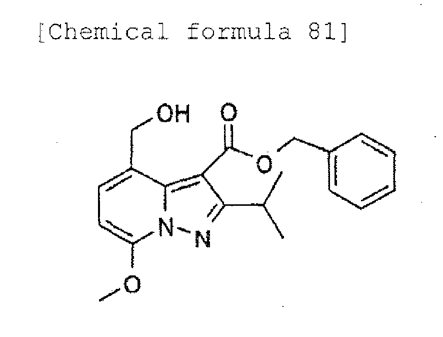

The compound of Example 2 (16.3 g) was dissolved in DMF (224 mL). Then, 4-methyl-2-pentynoic acid benzyl ester (6.21 g) and potassium carbonate (12.7 g) were added to the prepared solution, and the mixture was stirred at room temperature for 8 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane : ethyl acetate = 1:1 -> 1:2) to obtain the target product (6.10 g) as a white solid.
¹H-NMR (400 MHz, CDCl₃):δ 1.34 (6H, d, J = 6.7 Hz), 3.65-3.73 (1H, m), 4.14 (3H, s), 4.72 (1H, brs), 4.80 (2H, s), 5.40 (2H, s), 6.20 (1H, d, J = 7.3 Hz ), 7.30 (1H, d, J = 7.3 Hz ), 7.34-7.42 (3H, m), 7.46-7.49 (2H, m).

### <Example 8>

4-Hydroxymethyl-7-methoxypyrazolo[1,5-a]pyridine-3-carboxylic acid ethyl ester

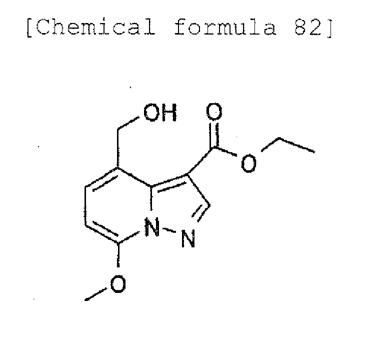

The compound of Example 2 (16.3 g) was dissolved in DMF (224 mL). Then, propiolic acid ethyl ester (3.13 mL) and potassium carbonate (12.7 g) were added to the prepared solution, and the mixture was stirred at room temperature for 8 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane : ethyl acetate = 1:1 -> 1:5) to obtain the target product (4.54 g) as a white solid.
¹H-NMR (400 MHz, CDCl₃): δ 1.42 (3H, t, J = 7.3 Hz), 4.18 (3H, s), 4.39 (2H, q, J = 7.3 Hz), 4.86 (2H, d, J = 6.7 Hz), 5.05 (1H, t , J = 6.7 Hz), 6.28 (1H, d, J = 7.3 Hz), 7.35 (1H, d, J = 7.3 Hz), 8.51 (1H, s).

### <Example 9>

2-Ethyl-4-hydroxymethylpyrazolo[1,5-a]pyridine-3-carboxylic acid ethyl ester

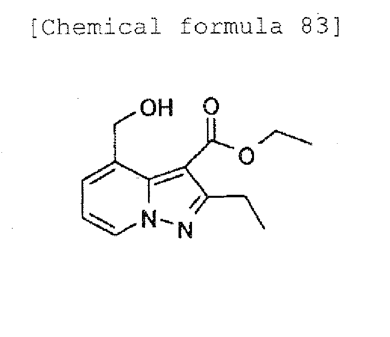

The same procedure as in Example 4 was followed using the compound of Example 3 (38.2 g) and 2-pentynoic acid ethyl ester (6.97 g) to obtain the target product (7.33 g) as a yellow solid. MS (EI⁺): 248 [M⁺]
¹H-NMR (400 MHz, CDCl₃): δ 1.35 (3H, t, J = 7.3 Hz), 1.44 (3H, t, J = 7.3 Hz), 3.08 (2H, q, J = 7.3 Hz), 4.41 (2H, q, J = 7.3 Hz), 4.86 (2H, d, J = 7.3 Hz), 5.02 (1H, t, J = 7.3 Hz), 6.87 (1H, t, J = 6.7 Hz), 7.30 (1H, d, J = 6.7 Hz), 8.40 (1H, d, J = 6.7 Hz).

### <Example 10>

2-Diethoxymethyl-4-hydroxymethyl-7-methoxypyrazolo[1,5-a]pyrid ine-3-carboxylic acid ethyl ester

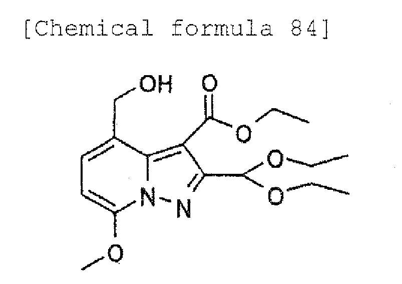

The compound of Example 2 (56.6 g) was dissolved in DMF (320 mL). 4,4-Diethoxy-2-butynoic acid ethyl ester (21.2 g) and potassium carbonate (43.9 g) were added to the prepared solution in that order, and the mixture was stirred at room temperature for 30 hours. Insoluble material was removed by filtration through Celite, and the filtrate was diluted with water and extracted with ethyl acetate. The extract layer was washed with water and then saturated brine and dried over anhydrous sodium sulfate. The solvent was evaporated under reduced pressure to obtain the target product (2.01 g) as a yellow oil.
MS (FAB⁺): 353 [M+H⁺]
¹H-NMR (400 MHz, CDCl₃): δ 1.25 (6H, t, J = 7.3 Hz), 1.44 (3H, t, J = 7.3 Hz), 3.66-3.74 (4H, m), 4.12 (3H, s), 4.42 (2H, q, J = 7.3 Hz), 4.77-4.81 (2H, m), 6.19 (1H, s), 6.22 (1H, d, J = 7.3 Hz), 7.31 (1H, d, J = 7.3 Hz).

### <Example 11>

4-Hydroxymethyl-7-methoxy-2-(tetrahydropyran-2-yloxymethyl)pyr azolo[1,5-a]pyridine-3-carboxylic acid ethyl ester

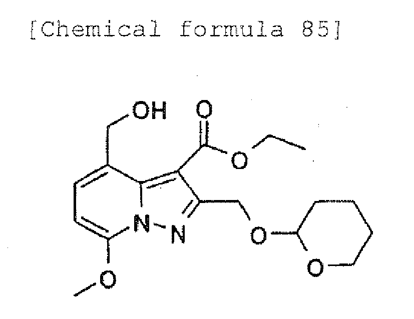

The compound of Example 2 (44.9 g) was dissolved in DMF (500 mL). Then, 4-(tetrahydropyran-2-yloxy)-2-butynoic acid ethyl ester (17.8 g) and potassium carbonate (34.8 g) were added to the prepared solution, and the mixture was stirred at room temperature for 17 hours. Water was added to the reaction mixture, and the resultant mixture was extracted with ethyl acetate. The extract layer was washed with saturated brine and dried over anhydrous sodium sulfate. The solvent was evaporated under reduced pressure, and the residue was purified by silica gel chromatography (hexane : ethyl acetate = 1:1 -> ethyl acetate) to obtain the target product (17.4 g) as a white solid.
¹H-NMR (400 MHz, CDCl₃): δ 1.44 (3H, t, J = 7.3 Hz), 1.51-1.73 (6H, m), 3.54-3.58 (1H, m), 3.93-3.99 (1H, m), 4.16 (3H, s), 4.42 (2H, q, J = 7.3 Hz), 4.78-4.85 (3H, m), 4.92 (1H, d, J = 12.2 Hz), 5.19 (1H, d, J = 12.2 Hz), 6.24 (1H, d, J = 8.0 Hz), 7.33 (1H, d, J = 8.0 Hz).

### <Example 12>

4-Hydroxymethyl-2-trifluoromethylpyrazolo[1,5-a]pyridine-3-car boxylic acid ethyl ester

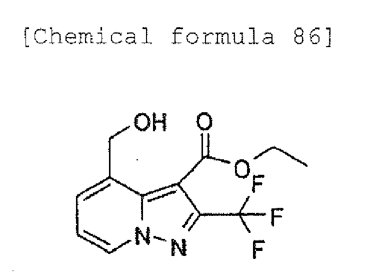

Ethyl 4,4,4-trifluoro-2-butynoate (22.1 g) and ground potassium carbonate (55.3 g) were added to a solution of the compound of Example 3 (64.9 g) in ethanol (750 mL), and the mixture was stirred at room temperature for 12 hours. Insoluble material was removed by filtration through Celite. The filtrate was concentrated under reduced pressure, and the residue was extracted with ethyl acetate. The extract layer was washed with water and saturated brine and dried over anhydrous sodium sulfate. The solvent was evaporated under reduced pressure, and the residue was purified by silica gel column chromatography (hexane : ethyl acetate = 2:1) to obtain the target product (18.9 g) as a yellow solid.
¹H-NMR (400 MHz, CDCl₃): δ 1.43 (3H, t, J = 7.3 Hz), 4.43 (2H, q, J = 7.3 Hz), 4.65 (1H, t, J = 7.3 Hz), 4.89 (2H, d, J = 7.3 Hz), 7.06 (1H, t, J = 7.3 Hz), 7.45 (1H, d, J = 7.3 Hz), 8.50 (1H, d, J = 7.3 Hz).

### <Example 13>

2-Ethyl-4-hydroxymethyl-7-methoxypyrazolo[1,5-a]pyridine-3-car boxylic acid

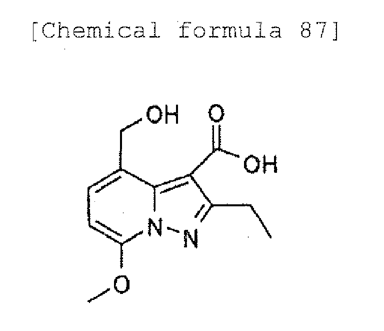

The compound of Example 4 (6.22 g) was dissolved in ethanol (150 mL), and a 10% aqueous potassium hydroxide solution (37 mL) was added to the prepared solution. The mixture was heated to reflux for 2 hours. The solvent was evaporated under reduced pressure, and the residue was dissolved in water and washed with ether. Concentrated hydrochloric acid was added to the aqueous layer to make it acidic, and the resultant product was extracted with ethyl acetate. The extract layer was washed with water and then saturated brine and dried over anhydrous sodium sulfate. The solvent was evaporated under reduced pressure to obtain the target product (4.58 g) as a gray solid.
¹H-NMR (400 MHz, CDCl₃): δ 1.37 (3H, t, J = 7.4 Hz), 3.19 (2H, q, J = 7.4 Hz), 4.18 (3H, s), 4.88 (2H, s), 6.29 (1H, d, J = 7.9 Hz), 7.38 (1H, d, J = 7.9 Hz) .

### <Example 14>

2-Cyclopropyl-4-hydroxymethyl-7-methoxypyrazolo[1,5-a]pyridine -3-carboxylic acid

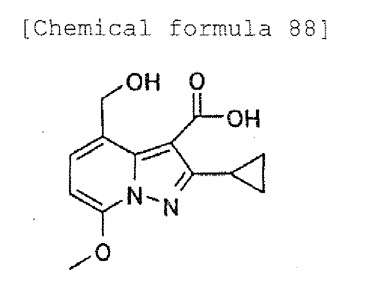

The compound of Example 6 (4.63 g) was dissolved in ethanol (70 mL). Then, potassium hydroxide (2.82 g) and water (30 mL) were added to the prepared solution at room temperature, and the mixture was stirred under heating and reflux for 2.5 hours. The solvent of the reaction mixture was evaporated under reduced pressure. The resultant mixture was diluted with water (100 mL), and concentrated hydrochloric acid (6.0 mL) was added to the mixture. The precipitated solid was collected by filtration to obtain the target product (3.32 g) as a white solid.
¹H-NM (400 MHz, CDCl₃): δ 1.05-1.11 (2H, m), 1.14-1.18 (2H, m), 2.70-2.76 (1H, m), 4.13 (3H, s), 4.86 (2H, s), 6.25 (1H, d, J = 8.0 Hz), 7.35 (1H, d, J = 8.0 Hz).

### <Example 15>

4-Hydroxymethyl-2-isopropyl-7-methoxypyrazolo[1,5-a]pyridine-3 -carboxylic acid

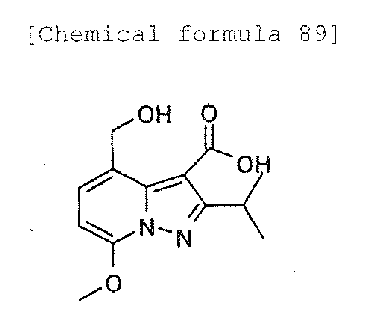

The compound of Example 7 (6.10 g) was dissolved in ethanol (91 mL), and potassium hydroxide (3.37 g) and water (39 mL) were added to the prepared solution at room temperature. The mixture was stirred under heating and reflux for 4 hours. The solvent of the reaction mixture was evaporated under reduced pressure. The resultant mixture was diluted with water (100 mL), and concentrated hydrochloric acid (5.0 mL) was added to the mixture. The precipitated solid was collected by filtration to obtain the target product (4.45 g) as a white solid.
¹H-NMR (400 MHz, CDCl₃): δ 1.44 (6H, d, J = 6.7 Hz), 3.84-3.95 (1H, m), 4.16 (3H, s), 4.90 (2H, s), 6.27 (H, d, J= 8.0 Hz), 7.38 (1H, d, J = 8.0 Hz).

### <Example 16>

4-Hydroxymethyl-7-methoxypyrazolo[1,5-a]pyridine-3-carboxylic acid

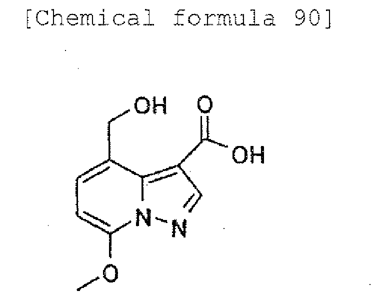

The compound of Example 8 (4.54 g) was dissolved in ethanol (96 mL), and potassium hydroxide (3.54 g) and water (41 mL) were added to the prepared solution at room temperature. The mixture was stirred under heating and reflux for 1 hour. The solvent of the reaction mixture was evaporated under reduced pressure. The resultant mixture was diluted with water (100 mL), and concentrated hydrochloric acid (8.3 mL) was added to the mixture. The precipitated solid was collected by filtration to obtain the target product (3.86 g) as a white solid.
¹H-NMR (400 MHz, CDCl₃): δ 4.20 (3H, s), 4.89 (2H, s), 6.34 (1H, d, J = 8.0 Hz), 7.42 (1H, d, J = 8.0 Hz), 8.59 (1H, s).

### <Example 17>

4-Hydroxymethyl-7-methoxy-2-(tetrahydropyran-2-yloxymethyl)pyr azolo[1,5-a]pyridine-3-carboxylic acid

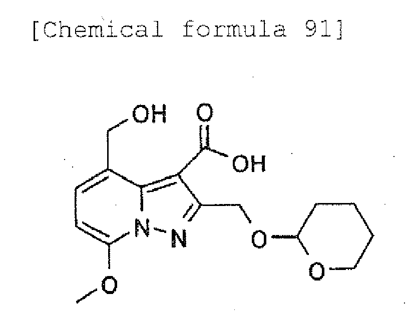

The compound of Example 11 (4.64 g) was dissolved in ethanol (60 mL). Then, potassium hydroxide (2.51 g) and water (19.2 mL) were added to the prepared solution at room temperature, and the mixture was stirred for 1.5 hours under heating and reflux. The solvent of the reaction mixture was evaporated under reduced pressure. The resultant mixture was diluted with water (70 mL), and dilute hydrochloric acid (35 mL) was added to the mixture. The precipitated solid was collected by filtration to obtain the target product (3.60 g) as a white solid.
¹H-NMR (400 MHz, CDCl₃): δ 1.57-1.81 (6H, m), 3.61-3.64 (1H, m), 3.88-3.93 (1H, m), 4.18 (3H, s), 4.81-4.86 (2H, m), 4.81-4.86 (2H, m), 4.92-4.94 (1H, m), 4.99 (1H, d, J = 12.2 Hz), 5.24 (1H, d, J = 12.2 Hz), 6.32 (1H, d, J = 7.3 Hz), 7.41 (1H, d, J = 7.3 Hz).

### <Example 18>

2-Ethyl-4-hydroxymethyl-7-methoxypyrazolo[1,5-a]pyridine

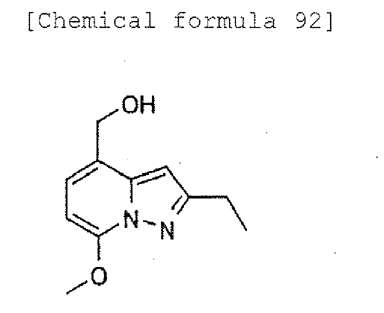

The compound of Example 13 (4.10 g) was suspended in bromobenzene (150 mL), and the suspension was heated to reflux for 5 hours. The solvent was evaporated under reduced pressure, and the residue was purified by silica gel column chromatography (hexane : ethyl acetate = 1:2 -> 1:4) to obtain the target product (2.49 g) as a white solid.
MS (EI⁺) : 206 [M⁺]
¹H-NMR (400 MHz, CDCl₃): δ 1. 36 (3H, t, J= 8.8 Hz), 1.65 (1H, brs), 2.92 (2H, q, J = 8.0 Hz), 4.13 (3H, s), 4.81 (2H, s) 5.99 (1H, d, J = 7.3 Hz), 6.43 (1H, s), 7.08 (1H, d, J = 7.3 Hz).

### <Example 19>

2-Cyclopropyl-4-hydroxymethyl-7-methoxypyrazolo[1,5-a]pyridine

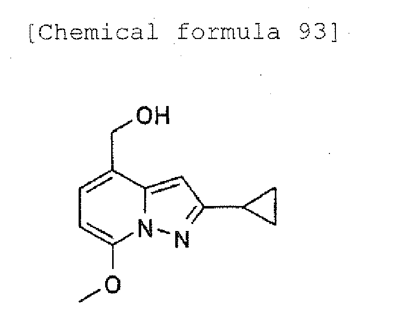

The compound of Example 14 (3.32 g) was suspended in o-dichlorobenzene (130 mL), and the suspension was stirred at 150°C for 22 hours. The solvent of the reaction mixture was evaporated under reduced pressure, and the residue was purified by silica gel chromatography (hexane : ethyl acetate = 1:3 -> 1:4) to obtain the target product (2.05 g) as a white solid.
¹H-NMR (400 MHz, CDCl₃) :δ 0.85-0.90 (2H, m), 1.01-1.06 (2H, m), 2.17-2.25 (1H, m), 4.12 (3H, s), 4.77 (2H, s), 5.96 (1H, d, J = 7.3 Hz), 6.19 (1H, s), 7.05 (1H, d, J = 7.3 Hz).

### <Example 20>

4-Hydroxymethyl-2-isopropyl-7-methoxypyrazolo[1,5-a]pyridine

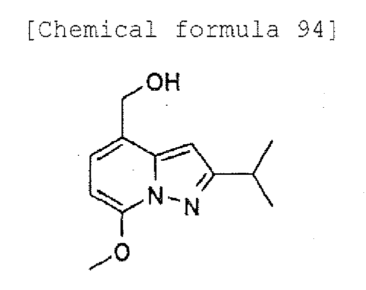

The compound of Example 15 (4.45 g) was suspended in o-dichlorobenzene, and the suspension was stirred at 150°C for 15 hours. The solvent was evaporated under reduced pressure, and the residue was purified by silica gel chromatography (hexane : ethyl acetate = 1:4 -> ethyl acetate) to obtain the target product (3.21 g) as a pale red solid.
¹H-NMR (400 MHz, CDCl₃): δ 1.38 (6H, d, J = 7.3 Hz), 3.23-3.32 (1H, m), 4.12 (3H, s), 4.81 (2H, s), 5.98 (1H, d, J = 8.0 Hz), 6.43 (1H, s), 7.07 (1H, d, J = 8.0 Hz).

### <Example 21>

4-Hydroxymethyl-7-methoxypyrazolo[1,5-a]pyridine

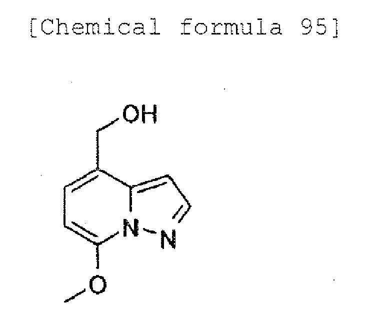

The compound of Example 16 (3.86 g) was suspended in o-dichlorobenzene, and the suspension was stirred at 150°C for 17 hours. The solvent of the reaction mixture was evaporated under reduced pressure, and the residue was purified by silica gel chromatography (ethyl acetate -> ethyl acetate : methanol = 50:1 -> 30:1) to obtain the target product (2.77 g) as a white solid.
¹H-NMR (400 MHz, CDCl₃): δ 4.14 (3H, s), 4.84 (2H, s), 6.05 (1H, d, J = 8.0 Hz), 6.61 (1H, d, J = 2.4 Hz), 7.12 (1H, d, J = 8.0 Hz), 8.01 (1H, d, J = 2.4 Hz).

### <Example 22>

4-Hydroxymethyl-7-methoxy-2-(tetrahydropyran-2-yloxymethyl)pyr azolo[1,5-a]pyridine

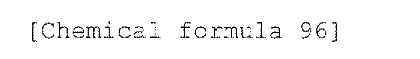

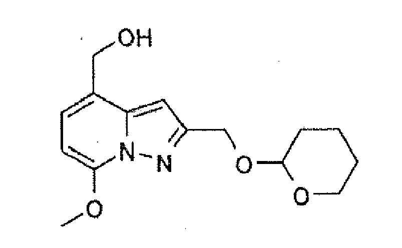

The compound of Example 17 (15.9 g) was suspended in o-dichlorobenzene (480 mL), and the suspension was stirred at 150°C for 13 hours. After completion of the reaction, the solvent was evaporated under reduced pressure, and the residue was purified by silica gel chromatography (ethyl acetate : methanol = 10:1) to obtain the target product (3.83 g) as a white solid and 2,4-dihydroxymethyl-7-methoxypyrazolo[1,5-a]pyridine (4.10 g).
¹H-NMR (400 MHz, CDCl₃): δ 1.53-1.80 (6H, m), 3.56-3.58 (1H, m), 3.93-3.99 (1H, m), 4. 13 (3H, s), 4.81 (1H, d, J = 12.8 Hz), 4.77-4.82 (3H, m), 5.02 (1H, d, J = 12.8 Hz), 6.04 (1H, d, J = 7.4 Hz), 6.61 (1H, s), 7.12 (1H, d, J = 7.4 Hz). 2,4-dihydroxymethyl-7-methoxypyrazolo[1,5-a]pyridine ¹H-NMR (400 MHz, CDCl₃): δ 4.15 (3H, s), 4.83 (2H, s), 4.94 (2H, s), 6.07 (1H, d, J = 7.4 Hz), 6.63 (1H, s), 7.14 (1H, d, J = 7.4 Hz).

### <Example 23>

2-Ethyl-4-hydroxymethylpyrazolo[1,5-a]pyridine

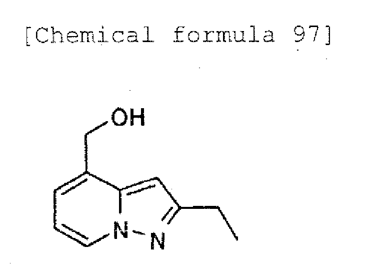

A 40% aqueous sulfuric acid solution (130 mL) was added to the compound of Example 9 (7.33 g), and the mixture was heated at 100°C for 1 hour. A 10% aqueous sodium hydroxide solution was added to the reaction mixture, and the precipitated solid was collected by filtration and purified by silica gel column chromatography (hexane : ethyl acetate = 1:1) to obtain the target product (4.52 g) as a white solid.
¹H-NMR (400 MHz, CDCl₃) : δ 1.36 (3H, t, J = 7.9 Hz), 2.11-2.13 (1H, brm), 2.86 (2H, q, J = 7.3 Hz), 4.85 (2H, d, J = 5.5 Hz), 6.37 (1H, s), 6.66 (1H, dd, J = 6.7, 7.4 Hz), 7.09 (1H, d, J = 6.7 Hz), 8.30 (1H, d, J = 7.4 Hz).

### <Example 24>

4-Hydroxymethyl-7-methoxy-2-trifluoromethylpyrazolo[1,5-a]pyri dine

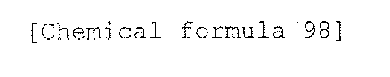

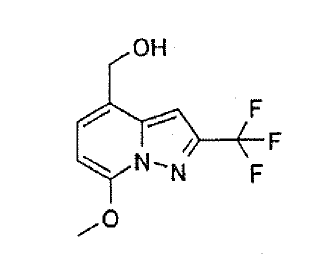

The same procedure as in Examples 13 and 18 was followed using the compound of Example 5 to obtain the target product as a white solid.
¹H-NMR (400 MHz, CDCl₃) : δ 1.56 (1H, brs), 4.18 (3H, s), 4.87 (2H, d, J = 0.9 Hz), 6.22 (1H, d, J = 7.6 Hz), 6.92 (1H, s), 7.24-7.27 (1H, m).

### <Example 25>

4-Hydroxymethyl-2-trifluoromethylpyrazolo[1,5-a]pyridine

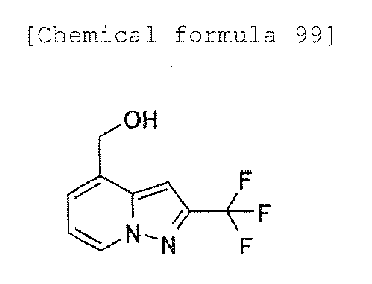

The compound of Example 12 (14.9 g) was dissolved in ethanol (250 mL), and a 10% aqueous potassium hydroxide solution (80 mL) was added to the prepared solution. The mixture was heated to reflux for 3 hours. The solvent was concentrated under reduced pressure, and the aqueous layer of the residue was washed with diethyl ether. Concentrated hydrochloric acid was added to the aqueous layer, and the precipitated solid was collected by filtration, washed with water, and dried. The obtained solid was suspended in o-dichlorobenzene (300 mL), and the suspension was heated at 150°C for 17 hours. After the suspension was allowed to cool, the solvent was evaporated under reduced pressure, and the residue was purified by silica gel column chromatography (hexane : ethyl acetate = 2:1) to obtain the target product (3.05 g) as a white solid.
MS (EI+): 216 [M⁺]
¹H-NMR (400 MHz, CDCl₃): δ 1.89 (1H, t, J = 6.1 Hz), 4.93 (2H, d, J = 6.1 Hz), 6.88 (1H, s), 6.94 (1H, t, J = 7.3 Hz), 7.27-7.29 (1H, m), 8.45 (1H, d, J = 7.3 Hz).

### <Example 26>

2-Ethyl-7-methoxypyrazolo[1,5-a]pyridine-4-carbaldehyde

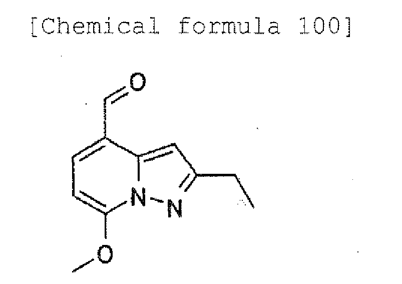

The compound of Example 18 (2.50 g) was dissolved in dichloromethane (60 mL), and activated manganese dioxide (10.5 g) was added to the prepared solution. The mixture was stirred at room temperature for 24 hours. Insoluble material was removed by filtration through Celite, and the solvent of the filtrate was evaporated under reduced pressure to obtain the target product (2.28 g) as a gray solid.
MS (EI⁺): 204 [M⁺]
¹H-NMR (400 MHz, CDCl₃) : δ 1.38 (3H, t, J = 8.0 Hz), 2.95 (2H, q, J = 8.0 Hz), 4.26 (3H, s), 6.20 (1H, d, J = 7.3 Hz), 7.18 (1H, s), 7.71 (1H, d, J = 7.3 Hz), 9.93 (1H, s).

### <Example 27>

7-Methoxy-2-trifluoromethylpyrazolo[1,5-a]pyridine-4-carbaldeh yde

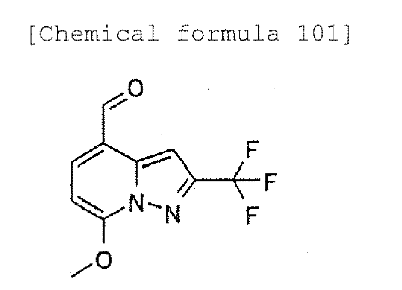

The same procedure as in Example 26 was followed using the compound of Example 24 to obtain the target product as a white solid.

LRMS (EI⁺) : 244 [M⁺]
¹H-NMR (400 MHz, CDCl₃): δ 4.31 (3H, s), 6.43 (1H, d, J = 7.9 Hz), 7.64 (1H, s), 7.87 (1H, d, J = 7.9Hz), 9.98 (1H, s).

### <Example 28>

2-Cyclopropyl-7-methoxypyrazolo[1,5-a]pyr4-dine-4-carbaldehyde

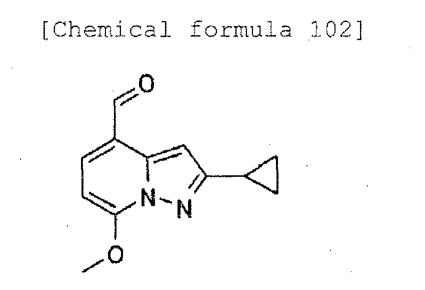

The compound of Example 19 (2. 04 g) was dissolved in chloroform (94 mL). Then, activated manganese dioxide (5.42 g) was added to the prepared solution at room temperature, and the mixture was stirred at 50°C for 5 hours. Insoluble material was removed by filtration through Celite, and the solvent of the filtrate was evaporated under reduced pressure. The residue was purified by silica gel chromatography (hexane : ethyl acetate = 1:3) to obtain the target product (1.94 g) as a yellow solid.
¹H-NMR (400 MHz, CDCl₃) : δ 0.91-0.95 (2H, m), 1.06-1.11 (2H, m), 2.21-2.27 (1H, m), 4.25 (3H, s), 6.18 (1H, d, J = 8.0 Hz), 6.92 (1H, s), 7.69 (1H, d, J = 8.0 Hz), 9.89 (1H, s).

### <Example 29>

2-Isopropyl-7-methoxy-pyrazolo[1,5-a]pyridine-4-carbaldehyde

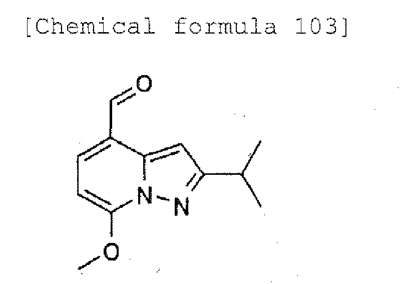

The compound of Example 20 (1.00 g) was dissolved in chloroform (45 mL), and activated manganese dioxide (2.63 g) was added to the prepared solution at room temperature. The mixture was stirred at 50°C for 3 hours. Insoluble material was removed by filtration through Celite, and the solvent of the filtrate was evaporated under reduced pressure to obtain the target product (938 mg) as a yellow solid.
¹H-NMR (400 MHz, CDCl₃) : δ 1.40 (6H, d, J = 7.3 Hz), 3.21-3.36 (1H, m), 4.26 (3H, s), 6.20 (1H, d, J= 8.0 Hz), 7.18 (1H, s), 7.71 (1H, d, J = 8.0 Hz), 9.92 (1H, s).

### <Example 30>

7-Methoxypyrazolo[1,5-a]pyridine-4-carbaldehyde

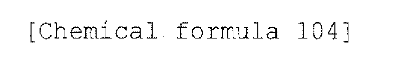

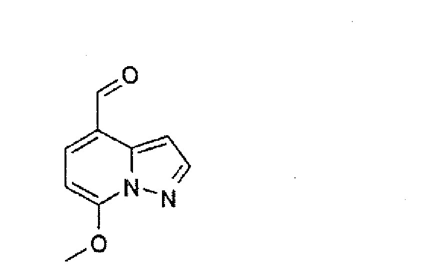

The compound of Example 21 (1.00 g) was dissolved in chloroform (56 mL), and activated manganese dioxide (3.25 g) was added to the prepared solution at room temperature. The mixture was stirred at 50°C for 3 hours. Insoluble material was removed by filtration through Celite, and the solvent of the filtrate was evaporated under reduced pressure to obtain the target product (966 mg) as a yellow solid.
¹H-NMR (400 MHz, CDCl₃) : δ 4.28 (3H, s), 6.80 (1H, d, J = 8.0 Hz), 7.35 (1H, d, J = 2.4 Hz), 7.67 (1H, d, J = 8.0 Hz), 8.16 (1H, d, J = 2.4 Hz), 9.96 (1H, s).

### <Example 31>

2-Ethylpyrazolo[1,5-a]pyridine-4-carbaldehyde

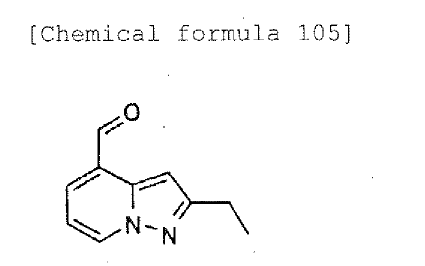

The same procedure as in Example 26 was followed using the compound of Example 23 (500 mg) to obtain the target product as a yellow solid.
¹H-NMR (400 MHz, CDCl₃): δ 1.39 (3H, t, J = 7.3 Hz), 2.92 (2H, q, J = 7.3 Hz), 6.85 (1H, dd, J = 6.7, 6.9 Hz), 7.13 (1H, s), 7.67 (1H, d, J = 6.9 Hz), 8.59 (1H, d, J = 6.7 Hz), 9.93 (1H, s).

### <Example 32>

7-Methoxy-2-(tetrahydropyran-2-yloxymethyl)pyrazolo[1,5-a]pyri dine-4-carbaldehyde

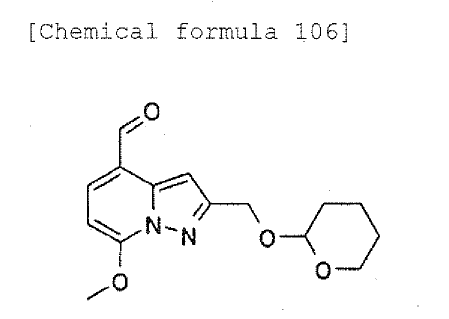

The compound of Example 22 (1. 02 g) was dissolved in chloroform (35 mL), and activated manganese dioxide (1.51 g) was added to the prepared solution at room temperature. The mixture was stirred at 50 °C for 4.5 hours. Insoluble material was removed by filtration through Celite, and the solvent of the filtrate was evaporated under reduced pressure to obtain the target product (876 mg) as a yellow solid.
¹H-NMR (400 MHz, CDCl₃): δ 1.52-1.90 (6H, m), 3.50-3.59 (1H, m), 3.92-3.98 (1H m), 4.26 (3H, s), 4.80 (1H, d, J = 12.2 Hz), 4.81-4.83 (1H, d, J = 12.2 Hz), 6.25 (1H, d, J = 7.3 Hz), 7.40 (1H, s), 7.74 (1H, d, J = 7.3 Hz), 9.95 (1H, s).

### <Example 33>

2-Ethyl-7-methoxypyrazolo[1,5-a]pyridine-4-carboxylic acid ethyl ester

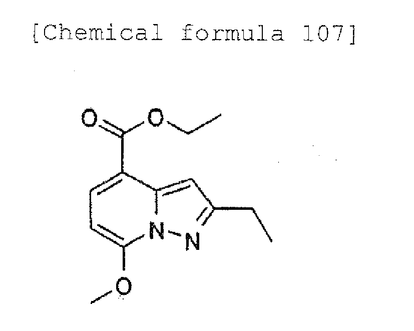

The compound of Example 26 (1.02 g) was suspended in water (100 mL), and potassium permanganate (3.16g) was added to the prepared solution. The mixture was stirred at room temperature for 21 hours. A 10% aqueous sodium hydroxide solution was added to the mixture to make it alkaline. Insoluble material was removed by filtration through Celite, and the filtrate was washed with ether. The aqueous layer was acidified with 10% hydrochloric acid and extracted with ethyl acetate. The extract layer was washed with water and then saturated brine and dried over anhydrous sodium sulfate. The solvent was evaporated under reduced pressure, and the residue (154 mg) was dissolved in DMF (7.0 mL). Ethyl iodide (0.0844 mL) and potassium carbonate (145 mg) were added to the prepared solution, and the mixture was stirred at room temperature for 1.5 hours. The reaction mixture was diluted with water and extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 1:1) to obtain the target product (55.6 mg) as a white solid.
MS (EI⁺) : 248 [M⁺]
¹H-NMR (400 MHz, CDCl₃) : δ 1.38 (3H, t, J = 8.6 Hz), 1.45 (3H, t, J = 6.7 Hz), 2.93 (2H, q, J = 8.6 Hz), 4.21 (3H, s), 4.42 (2H, q, J = 6.7 Hz), 6.10 (1H, d, J = 8.0 Hz), 6.94 (1H, s), 8.01 (1H, d, J = 8.0 Hz).

### <Example 34>

2-Ethyl-7-methoxypyrazolo[1,5-a]pyridine-4-carboxylic acid

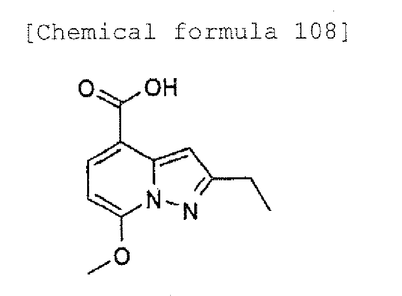

The compound of Example 33 (286 mg) was dissolved in methanol (3.0 mL), and a 10% aqueous potassium hydroxide solution (2.0 mL) was added to the prepared solution. The mixture was stirred at room temperature for 17 hours. The reaction mixture was washed with ether, and the aqueous layer was acidified with 10% hydrochloric acid and was extracted with ethyl acetate. The extract layer was washed with water and then saturated brine and dried over anhydrous sodium sulfate, and the solvent was evaporated under reduced pressure to obtain the target product (121 mg).
¹H-NMR (400 MHz, CDCl₃) : δ 1.39 (3H, t, J = 7.3 Hz), 2.85 (2H, q, J = 7.3 Hz), 4.24 (3H, s), 6.15 (1H, d, J= 7.9 Hz), 7.01 (H, s), 8.12 (1H, d, J = 7.9 Hz).

### <Example 35>

7-Methoxy-2-trifluoromethylpyrazolo[1,5-a]pyridine-4-carboxyli c acid

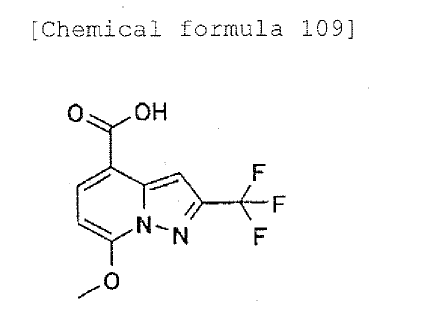

The compound of Example 27 (1.23g) was dissolved in t-butanol (36 mL) and water (12 mL). Sodium dihydrogenphosphate dihydrate (787 mg), 2-methyl-2-butene (2.4 mL), and sodium chlorite (2.00 g) were added to the prepared solution, and the mixture was stirred at room temperature for 5 hours. A 10% aqueous sodium hydroxide solution was added to the reaction mixture to make it alkaline and was washed with ether. Then, 10% hydrochloric acid was added to the aqueous layer, and the precipitated crystal was collected by filtration and washed with water to obtain the target product (885 mg) as a white solid.
¹H-NMR (400 MHz, CDCl₃) : δ 4.29 (3H, s), 6.37 (H, d, J = 8.0 Hz), 7.49 (1H, s), 8.25 (1H, d, J = 8.0 Hz).

### <Example 36>

2-Cyclopropyl-7-methoxypyrazolo[1,5-a]pyridine-4-carboxylic acid

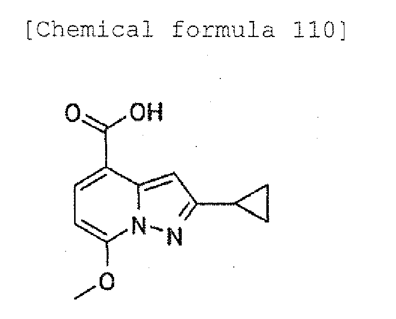

Silver nitrate (3.60 g), sodium hydroxide (1.75 g), and water (85 mL) were added to the compound of Example 28 (1.83 g), and the mixture was stirred at room temperature for 73 hours. Insoluble material was removed by filtration through Celite, and the filtrate was washed with diethyl ether. Dilute hydrochloric acid was added to the aqueous layer to make it acidic, and the resultant product was extracted with ethyl acetate. The organic layer was washed with water and then saturated brine and dried over anhydrous sodium sulfate. The solvent was evaporated under reduced pressure to obtain the target product (1.06 g) as a white solid.
¹H-NMR (400 MHz, CDCl₃) : δ 0.92-0.96 (2H, m), 1.06-1.11 (2H, m), 2.22-2.29 (1H, m), 4.23 (3H, s), 6.13 (1H, d, J = 8.0 Hz), 7.27 (1H, s), 8.10 (1H, d, J = 8.0 Hz).

### <Example 37>

2-Isopropyl-7-methoxypyrazolo[1,5-a]pyridine-4-carboxylic acid

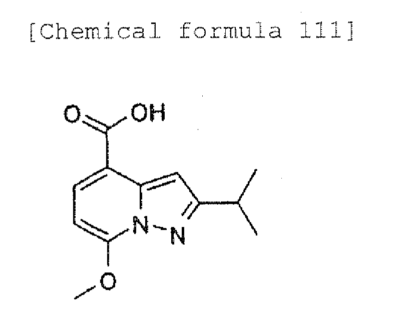

Silver nitrate (1.82 g), sodium hydroxide (859 mg), and water (43 mL) were added to the compound of Example 29 (938 mg), and the mixture was stirred at room temperature for 2 hours. Insoluble material was removed by filtration through Celite, and the filtrate was washed with diethyl ether. Dilute hydrochloric acid was added to the aqueous layer to make it acidic, and the resultant product was extracted with ethyl acetate and chloroform: methanol = 9:1. The extract layer was washed with saturated brine and dried over anhydrous sodium sulfate. The solvent was evaporated under reduced pressure to obtain the target product (887 mg) as a white solid. ¹H-NMR (400 MHz, CDCl₃): δ 1.41 (6H, d, J = 7.3 Hz), 3.30-3.37 (1H, m), 4.25 (3H, s), 6.17 (1H, d, J = 8.0 Hz), 7.02 (1H, s), 8.13 (1H, d, J = 8.0 Hz).

### <Example 38>

7-Methoxypyrazolo[1,5-a]pyridine-4-carboxylic acid

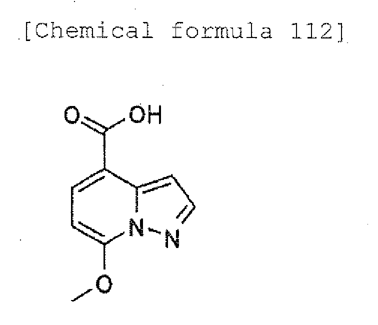

A suspension of silver nitrate (2.33 g) and sodium hydroxide (1.10 g) in water (55 mL) was added to the compound of Example 30 (965 mg), and the mixture was stirred at room temperature for 1.5 hours. Insoluble material was removed by filtration through Celite, and the filtrate was washed with diethyl ether. Dilute hydrochloric acid was added to the aqueous layer to make it acidic, and the resultant product was extracted with ethyl acetate and chloroform : methanol = 9:1. The extract layer was washed with saturated brine and dried over anhydrous sodium sulfate. The solvent was evaporated under reduced pressure to obtain the target product (769 mg) as a white solid.
¹H-NMR (400 MHz, CDCl₃) : δ 4.26 (3H, s), 6.22 (1H, d, J = 8.0 Hz), 7.19 (1H, s), 8.13 (1H, d, J = 1.8 Hz), 8.16 (1H, d, J = 8.0 Hz).

### <Example 39>

2-Ethylpyrazolo[1,5-a]pyridine-4-carboxylic acid

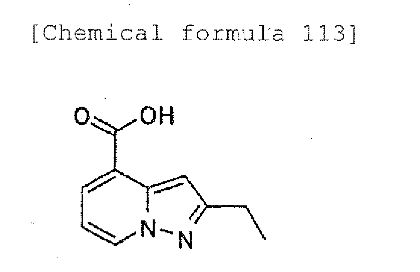

The same procedure as in Example 35 was followed using the compound of Example 31 to obtain the target product as a white solid. ¹H-NMR (400 MHz, CDCl₃): δ 1.40 (3H, t, J = 7.3 Hz), 2.92 (2H, q, J = 7.3 Hz), 6.78 (1H, dd, J = 6. 7, 7.3 Hz), 6.98 (1H, s), 8.03 (1H, dd, J = 1.2, 7.3 Hz), 8.63 (1H, dd, J = 1.2, 6.7 Hz).

### <Example 40>

7-Methoxy-2-(tetahydropyan-3-yloxymethyl)pyrazolo[1,5-a]pyri dine-4-carboxylic acid

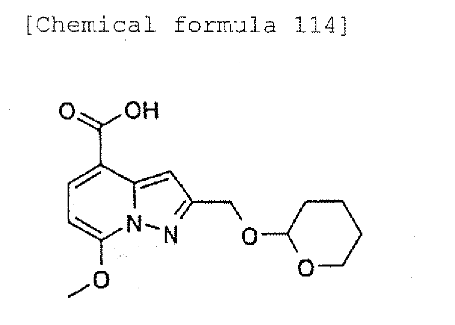

Silver nitrate (1.36 g), sodium hydroxide (623 mg), and water (30 mL) were added to the compound of Example 32 (876 mg), and the mixture was stirred at room temperature for 4 hours. Insoluble material was removed by filtration through Celite, and the filtrate was washed with diethyl ether. The aqueous layer was acidified with dilute hydrochloric acid and was extracted with ethyl acetate. The extract layer was washed with water and then saturated brine and dried over anhydrous sodium sulfate, and the solvent was evaporated under reduced pressure to obtain the target product (789 mg) as a white solid.
¹H-NMR (400 MHz, CDCl₃) : δ 1.54-1.91 (6H, m), 3.56-3.60 (1H, m), 3.94-4.00 (1H, m), 4.23 (3H, s), 4.80 (2H, m), 5.05 (1H, d, J = 12.8 Hz), 6.20 (1H, d, J = 8.0 Hz), 7.24 (1H, s), 8.15 (1H, d, J = 8.0 Hz).

### <Example 41>

4-Acetoxymethyl-2-diethoxymethyl-7-methoxypyrazolo[1,5-a]pyrid ine-3-carboxylic acid ethyl ester

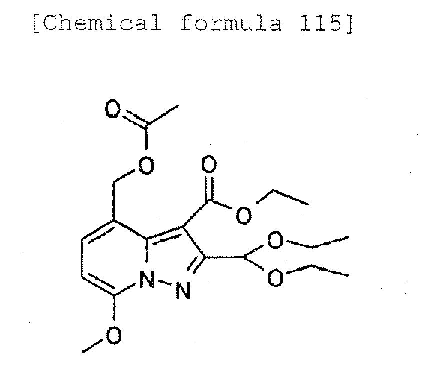

The compound of Example 10 (2.10 g) was dissolved in pyridine (20 mL), and acetic anhydride (1.12 mL) was added to the prepared solution. The mixture was stirred at room temperature for 6 hours. The reaction mixture was diluted with water and then extracted with ethyl acetate, and the extract layer was washed with water and then saturated brine and dried over anhydrous sodium sulfate. The solvent was evaporated under reduced pressure to obtain the target product (2.01 g) as a colorless oil.
MS (EI⁺): 394 [M⁺]
¹H-NMR (400 MHz), CDCl₃) :δ 1.25 (6H, t, J = 7.3 Hz), 1.41 (3H, t, J = 7.3 Hz), 2.04 (3H, s), 3.67-3.75 (4H, m), 4.13 (3H, s), 4.37 (2H, q, J = 7.3 Hz), 5.47 (2H, s), 6.17 (1H, s), 6.19 (1H, d, J = 8.0 Hz), 7.35 (1H, d, J = 8.0 Hz).

### <Example 42>

4-Acetoxymethyl-2-formyl-7-mhoxypyrazolo[1,5-a]pyridine-3-ca rboxylic acid ethyl ester

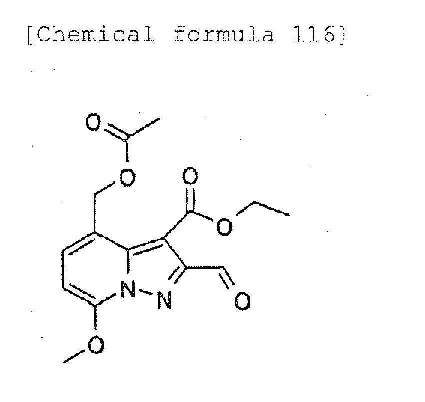

The compound of Example 41 (2.01 g) was dissolved in a mixed solvent (20 mL) of acetone and water (2:1), and p-toluenesulfonic acid monohydrate (97.3 mg) was added to the prepared solution. The mixture was stirred at 70°C for 2 hours. After allowed to cool, the reaction mixture was extracted with ethyl acetate, and the extract layer was washed with water and then saturated brine and dried over anhydrous sodium sulfate. The solvent was evaporated under reduced pressure, and the residue was purified by silica gel column chromatography hexane : ethyl acetate = 1:2) to obtain the target product (1.47 g) as a white solid.
MS (EI⁺) : 320 [M⁺]
¹H-NMR (400 MHz, CDCl₃) : δ 1.43 (3H, t, J = 7.3 Hz), 2.05 (3H, s), 4.21 (3H, s), 4.45 (2H, q, J = 7.3 Hz), 5.50 (2H, s), 6.36 (1H, d, J = 8.0 Hz), 7.46 (1H, d, J = 8.0 Hz), 10.49 (1H, s).

### <Example 43>

4-Acetoxymethyl-2-difluoromethyl-7-methoxypyrazolo[1,5-a]pyrid ine-3-carboxylic acid ethyl ester

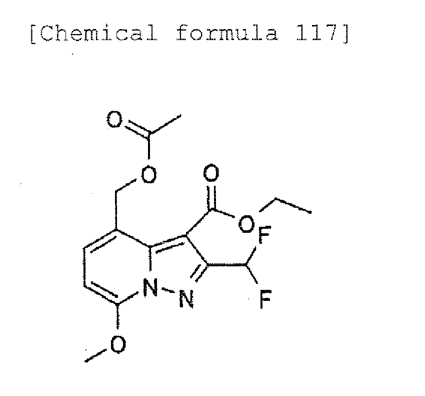

The compound of Example 42 (1.47 g) was dissolved in dichloromethane (23 mL) under argon atmosphere. Then, diethylaminosulfur trifluoride (1.52 mL) was added dropwise to the prepared solution under cooling with ice, and the mixture was stirred at room temperature for 1.5 hours. A saturated aqueous sodium hydrogen carbonate solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 2:3) to obtain the target product (1.21 g) as a white solid.
MS (EI⁺): 342 [M⁺]
¹H-NMR (400 MHz), CDCl₃): δ 1.42 (3H, t, J = 7.3 Hz), 2.06 (3H, s), 4.20 (3H, s), 4.40 (2H, q, J = 7.3 Hz), 5.60 (2H, s), 6.35 (¹H, d, J = 7.9 Hz), 7.26 (1H, t, J = 53.8 Hz), 7.49 (1H, d, J = 7.9 Hz).

### <Example 44>

2-Difluoromethyl-4-hydroxymethyl-7-methoxypyrazolo[1,5-a]pyrid ine

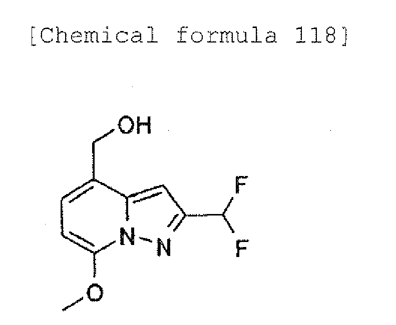

The compound of Example 43 (1.21 g) was dissolved in ethanol (10 mL), and a 10% aqueous potassium hydroxide solution (6.0 mL) was added to the prepared solution. The mixture was heated to reflux for 3.5 hours. After allowed to cool, the reaction mixture was washed with diethyl ether, acidified with 10% hydrochloric acid, and extracted with ethyl acetate. The extract layer was washed with water and then saturated brine and dried over anhydrous sodium sulfate, and the solvent was evaporated under reduced pressure. The obtained residue (871 mg) was suspended in bromobenzene (50 mL), and the resultant suspension was heated to reflux for 3.5 hours. The solvent was evaporated under reduced pressure, and the residue was purified by silica gel column chromatography (hexane : ethyl acetate = 1:1) to obtain the target product (583 mg) as a white solid.
MS (EI⁺): 228 [M⁺]
¹H-NMR (400 MHz, CDCl₃): δ 1.77 (1H, t, J = 5.5 Hz), 4.18 (3H, s), 4.87 (2H, d, J = 5.5 Hz), 6.17 (1H, d, J = 7.3 Hz), 6.86 (1H, s), 6.94 (1H, t, J = 55.4 Hz), 7.22 (1H, d, J = 7.3 Hz).

### <Example 45>

2-Difluoromethyl-7-methoxypyrazolo[1,5-a]pyridine-4-carbaldehy de

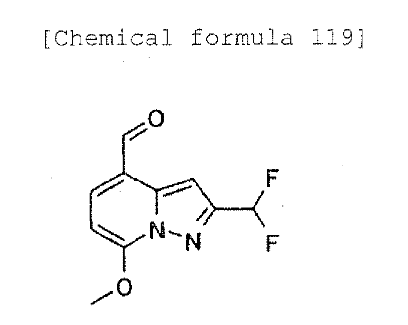

The compound of Example 44 (582 mg) was dissolved in dichloromethane (20 mL). Then, activated manganese dioxide (2.22 g) was added to the prepared solution, and the mixture was stirred at room temperature for 11 hours. Insoluble material was removed by filtration through Celite, and the solvent of the filtrate was evaporated under reduced pressure to obtain the target product (580 mg) as a white solid.
MS (EI⁺): 226 [M⁺]
¹H-NMR (400 MHz, CDCl₃): δ 4.30 (3H, s), 6.37 (1H, d, J = 8.0 Hz), 6.95 (1H, t, J = 55.4 Hz), 7.59 (1H, s), 7.83 (1H, d, J = 8.0 Hz), 9.98 (1H, s).

### <Example 46>

2-Difluoromethyl-4-(1-hydroxypropyl)-7-methoxypyrazolo[1,5-a]p yridine

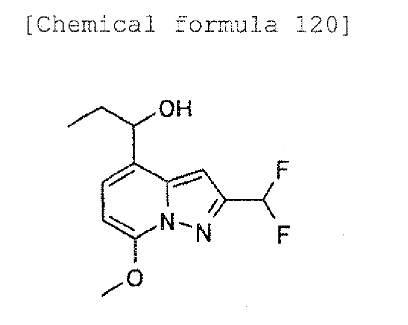

The compound of Example 45 (580 mg) was dissolved in THF (13 mL) under argon atmosphere. Ethylmagnesium bromide (a 1.0 mol/L THF solution, 3.1 mL) was added dropwise to the prepared solution at -78°C, and the mixture was stirred at room-temperature for 2 hours. A saturated aqueous ammonium chloride solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (602 mg) as a yellow solid.
MS (EI⁺): 256 [M⁺]
¹H-NMR (400 MHz, CDCl₃): δ 0.96 (3H, t, J = 7.3 Hz), 1.86-1.99 (3H, m), 4.17 (3H, s), 4.88 (1H, t, J = 6.7 Hz), 6.18 (1H, d, J = 7.9 Hz), 6.87 (1H, s), 6.99 (1H, t, J= 55.0 Hz), 7.22 (1H, d, J = 7.9 Hz).

### <Example 47>

2-Difluoromethyl-7-methoxy-4-propionylpyrazolo[1,5-a]pyridine

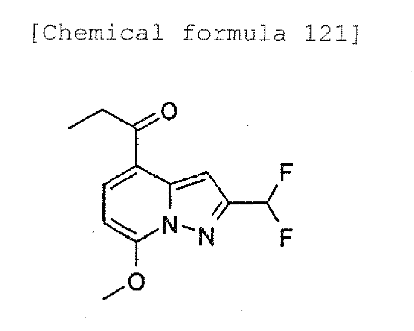

The compound of Example 46 (550 mg) was dissolved in chloroform (10 mL). Activated manganese dioxide (5.61 g (and additional 1.87 g at 24 hour intervals)) was added to the prepared solution, and the mixture was heated to reflux for 2 days. Insoluble material was removed by filtration through Celite, and the solvent of the filtrate was evaporated under reduced pressure to obtain the target product (415 mg).
MS (EI⁺): 254 [M⁺]
¹H-NMR (400 MHz, COCl₃): δ 1.27 (3H, t, J = 7.3 Hz), 3.03 (2H, q, J = 7.3 Hz), 4.27 (3H, s), 6.26 (1H, d, J = 8.0 Hz), 6.94 (1H, t, J = 55.0 Hz), 7.62 (1H, s), 7.98 (1H, d, J = 8.0 Hz).

### <Example 48>

2-Difluoromethyl-7-methoxypyrazolo[1,5-a]pyridine-4-carboxylic acid methyl ester

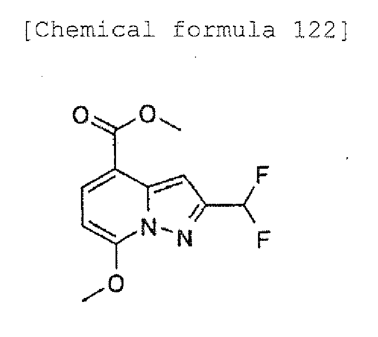

Dimethyl carbonate (10 mL), 60% sodium hydride (87.0 mg), and one drop of methanol were added to the compound of Example 47 (185 mg), and the mixture was heated to reflux for 1 hour and 40 minutes. A saturated aqueous ammonium chloride solution was added to the reaction mixture, and the resultant mixture was extracted with ethyl acetate. The extract layer was dried over anhydrous sodium sulfate, and the solvent was evaporated under reduced pressure. The residue was purified by silica gel chromatography (hexane : ethyl acetate = 3:2) to obtain the target product (33.2 mg) as a yellow solid.
MS (EI⁺): 256 [M⁺]
¹H-NMR (400 MHz, CDCl₃): δ 3.99 (3H, s), 4.26 (3H, s), 6.28 (1H, d, J = 8.4 Hz), 6.95 (1H, t, J= 55.2 Hz), 7.36 (1H, s), 8.13 (1H, d, J = 8.0 Hz).

### <Example 49>

7-Methoxy-4-(tetrahydropyran-2-yloxymethyl)pyrazolo[1,5-a]pyri dine-2-carbaldehyde

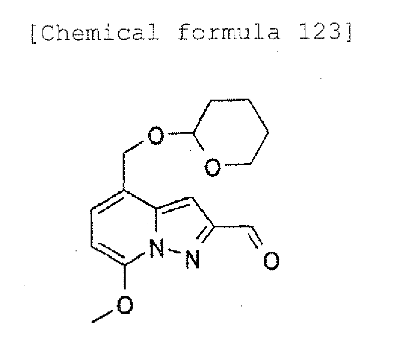

3,4-Dihydro-2H-pyran (707 mg) and p-toluenesulfonic acid monohydrate (79.9 mg) were added to a solution of the 2,4-dihydroxymethyl-7-methoxypyrazolo[1,5-a]pyridine (1.75 g) obtained in Example 22 in DMF (30 mL), and the mixture was stirred at room temperature for 4 days. A saturated aqueous sodium hydrogen carbonate solution was added to the reaction mixture, and the resultant mixture was extracted with ethyl acetate. The extract layer was dried over anhydrous sodium sulfate, and the solvent was evaporated under reduced pressure. The residue was subjected to silica gel column chromatography (ethyl acetate) to obtain [7-methoxy-4-(tetrahydropyran-2-yloxymethyl)pyrazolo[1,5-a]pyr idine-2-yl]methanol (0.59 g) as a pale yellow oil and 4-hydroxymethyl-7-methoxy-2-(tetrahydropyran-2-yloxymethyl)pyr azolo[1,5-a]pyridine (0.65 g) as a pale yellow oil.
Chloroform (40 mL) and activated manganese dioxide (1.38 g) were added to the obtained [7-methoxy-4-(tetrahydropyran-2-yloxymethyl)pyrazolo[1,5-a]pyr idine-2-yl]methanol (922 mg), and the mixture was heated to reflux for 15 hours. Insoluble material was removed by filtration through Celite, and the resultant mixture was washed with warm chloroform. The washings were combined with the filtrate, and the solvent was evaporated under reduced pressure. The residue was purified by silica gel column chromatography (ethyl acetate) to obtain the target product (738 mg) as a pale yellow oil.
LRMS (CI⁺): 291 [M+H⁺]

### <Example 50>

4-Hydroxymethyl-7-methoxypyrazolo[1,5-a]pyridine-2-carbonitril e

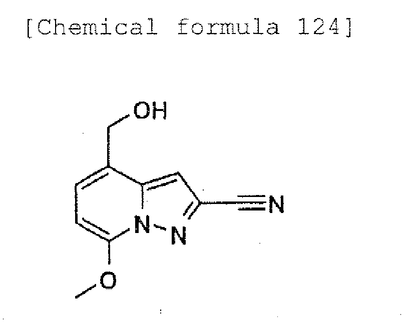

Sodium acetate (1.00 g) and hydroxylamine hydrochloride (450 mg) were added to a solution of the compound of Example 49 (627 mg) in methanol (22 mL) at room temperature, and the mixture was stirred at room temperature for 30 minutes. The solvent was evaporated under reduced pressure, and the residue was purified by silica gel column chromatography (ethyl acetate) to obtain a white solid (671 mg).
Triethylamine (1.52 mL) and trifluoroacetic anhydride (0.60 mL) were added to a solution of the obtained solid in methylene chloride (22 mL), and the mixture was stirred at room temperature for 1 hour. Water was added to the reaction mixture, and the resultant mixture was extracted with methylene chloride. The extract layer was dried over anhydrous magnesium sulfate, and the solvent was evaporated under reduced pressure.
p-Toluenesulfonic acid monohydrate (411 mg) was added to a solution of the obtained residue in methanol (22 mL), and the mixture was stirred at room temperature for 1 hour. A saturated aqueous sodium hydrogen carbonate solution was added to the reaction mixture, and the resultant mixture was extracted with ethyl acetate. The extract layer was dried over anhydrous magnesium sulfate, and the solvent was evaporated under reduced pressure. The residue was washed with isopropyl ether to obtain the target product (389 mg) as a pale yellow solid.
MS (EI⁺): 203 [M⁺]
¹H-NMR (400 MHz, CDCl₃): δ 1.84 (1H, t, J = 6.1 Hz), 4.19 (3H, s), 4.86 (2H, d, J = 6.1 Hz), 6.28 (1H, d, J = 7.9 Hz), 7.06 (1H, s), 7.29 (1H, d, J = 7.9 Hz).

### <Example 51>

4-Formyl-7-methoxypyrazolo[1,5-a]pyridine-2-carbonitrile

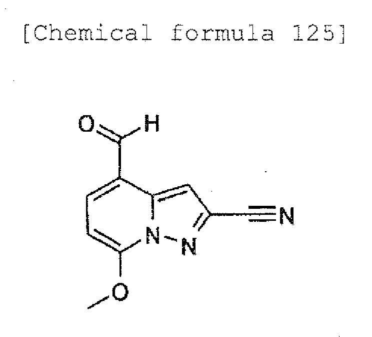

Chloroform (45 mL) and activated manganese dioxide (1.63 g) were added to the compound of Example 50 (380 mg), and the mixture was heated to reflux for 3 hours. Insoluble material was removed by filtration through Celite, and the resultant mixture was washed with warm chloroform. The washings were combined with the filtrate, and the solvent was evaporated under reduced pressure to obtain the target product (360 mg) as a pale yellow solid.
MS (EI⁺) : 201 [M⁺]
¹H-NMR (400 MHz, CDCl₃): δ 4.32 (3H, s), 6.48 (1H, d, J = 7.9 Hz), 7.67 (2H, s), 7.90 (1H, d, J = 7.9 Hz), 9.98 (1H, s).

### <Example 52>

2-Cyano-7-methoxypyrazolo[1,5-a]pyridine-4-carboxylic acid

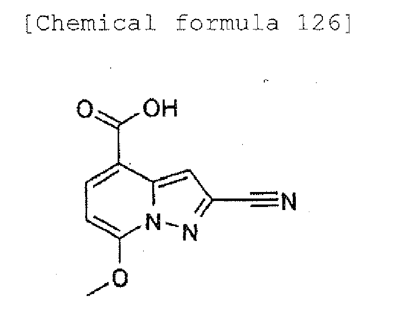

2-Methyl-2-butene (3 mL) and a solution of sodium chlorite (740 mg) and sodium dihydrogen phosphate (753 mg) in water (5 mL) were added to a solution of the compound of Example 51 (183 mg) in DMSO (9 mL), and the mixture was stirred at room temperature for 12 hours. After 1 mol/L sodium hydroxide (5 mL) was added to the reaction mixture, water (15 mL) and ethyl acetate (10 mL) were added to the resultant mixture, and the mixture was stirred. After the organic layer was separated, the pH of the aqueous layer was adjusted to 3 with concentrated hydrochloric acid, and the resultant product was extracted with chloroform : methanol = 7:1. The extract layer was dried over anhydrous magnesium sulfate, and the solvent was evaporated under reduced pressure. The residue was washed with water to obtain the target product (149 mg) as a white solid.
LRMS (EI⁺): 217 [M⁺]
¹H-NMR (400 MHz, DMSO-d₆): δ 4.22 (3H, s), 6.78 (1H, d, J = 8.6 Hz), 7.61 (1H, s), 8.15 (1H, d, J = 8.6 Hz).

### <Example 53>

2-Hydroxymethyl-7-methoxypyrazolo[1,5-a]pyridine-4-carboxylic acid

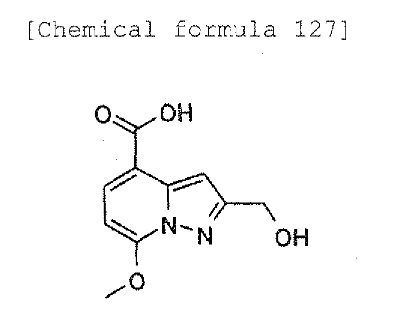

The compound of Example 40 (437 mg) was dissolved in methanol (14 mL). p-Toluenesulfonic acid monohydrate (27.0 mg) was added to the prepared solution at room temperature, and the mixture was stirred at 50°C for 30 minutes. The reaction mixture was cooled with ice, and the precipitate was collected by filtration to obtain the target product (254 mg) as a white solid.
¹H-NMR (400 MHz, DMSO-d₆): δ 4.15 (3H, s), 4.64 (2H, d, , J = 4.9 Hz), 5.31 (1H, t, J = 4.9 Hz), 6.44 (1H, d, J = 8.0 Hz), 6.98 (1H, d, J = 8.0 Hz), 7.95 (1H, s), 12.90 (1H, brs).

### <Example 54>

7-Methoxy-2-methoxymethylpyrazolo[1,5-a]pyridine-4-carboxylic acid methyl ester

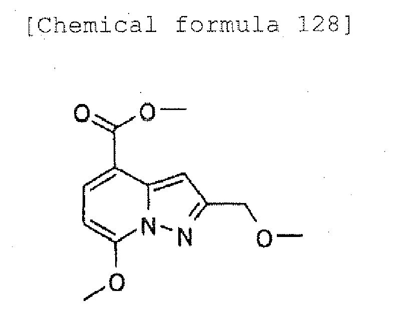

The compound of Example 53 (253 mg) was dissolved in DMF (11 mL), and silver oxide (2.64 g) and iodomethane (1.42 mL) were added to the prepared solution. The mixture was stirred at room temperature for 15 hours. Insoluble material was removed by filtration through Celite. The solvent of the filtrate was evaporated under reduced pressure, and the residue was purified by silica gel chromatography (ethyl acetate) to obtain the target product (224 mg) as a yellowish white solid.
¹H-NMR (400 MHz, CDCl₃): δ 3.47 (3H, s), 3.97 (3H, s), 4.23 (3H, s), 4.76 (2H, s), 6.16 (1H, d, J = 8.0 Hz), 7.14 (1H, s), 8.05 (1H, d, J = 8.0 Hz).

### <Example 55>

7-Methoxy-2-methoxymethylpyrazolo[1,5-a]pyridine-4-carboxylic acid

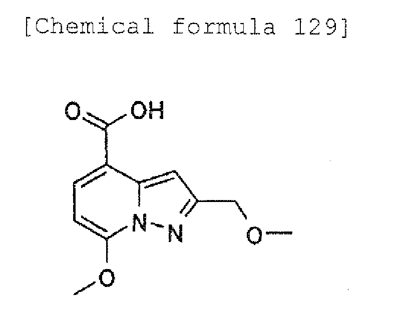

The compound of Example 54 (222 mg) was dissolved in methanol (4.20 mL), and potassium hydroxide (174 mg) and water (1.35 mL) were added to the prepared solution at room temperature. The mixture was stirred at room temperature for 4 hours. The solvent of the reaction mixture was evaporated under reduced pressure. Water was added to the residue, and the resultant mixture was washed with diethyl ether. The aqueous layer was acidified with dilute hydrochloric acid, and the resultant product was extracted with ethyl acetate. The extract layer was dried over anhydrous sodium sulfate. The solvent was evaporated under reduced pressure to obtain the target product (207 mg) as a white solid.
¹H-NMR (400 MHz, CDCl₃): δ 3.48 (3H, s), 4.25 (3H, s), 4.77 (2H, s), 6.20 (1H, d, J = 8.0 Hz), 7.22 (1H, s), 8.15 (1H, d, J = 8.0 Hz).

### <Example 56>

4-(t-Butyldimethylsilyloxymethyl)-2-trifluoromethylpyrazolo[1, 5-a]pyridine

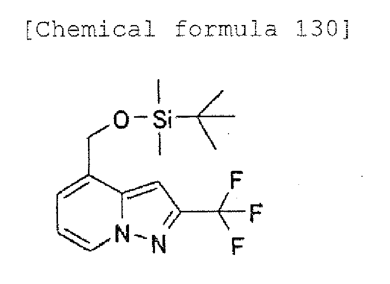

The compound of Example 25 (3.05 g) was dissolved in DMF (30 mL), and imidazole (1.92 g) and tert-butyldimethylsilyl chloride (3.19g) were added to the prepared solution. The mixture was stirred at room temperature for 2.5 hours. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:1) to obtain the target product (4.84 g) as a colorless oil.
MS (EI⁺): 330 [M⁺]
¹H-NMR (400 MHz), CDCl₃): δ 0.14 (6H, s), 0.96 (9H, s), 4.90 (2H, s), 6.77 (1H, s), 6.93 (1H, t, J = 7.3 Hz), (1H, m), 8.41 (1H, d, J = 7.3 Hz).

### <Example 57>

4-(t-Butyldimethylsilyloxymethyl)-7-iodo-2-trifluoromethylpyra zolo[1,5-a]pyridine

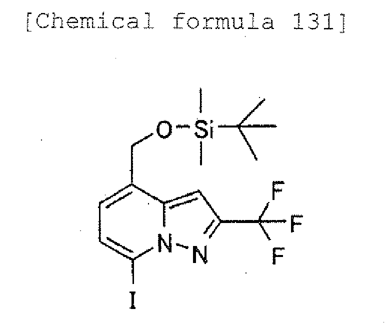

The compound of Example 56 (4.84 g) was dissolved in THF (20 ml) under argon atmosphere, and n-butyllithium (a 1.59 mol/M THF solution, 11.7 mL) was added dropwise to the prepared solution at -78°C. The mixture was stirred at -78°C for 2 hours. A solution of diiodoethane (4.77 g) in THF (10 mL) was added dropwise to the reaction mixture, and the resultant mixture was stirred at -78°C for 30 minutes. A saturated aqueous ammonium chloride solution was added to the reaction mixture, and the resultant mixture was extracted with ethyl acetate. The extract layer was washed with water and then saturated brine and dried over anhydrous sodium sulfate. The solvent was evaporated under reduced pressure, and the residue was purified by silica gel column chromatography (hexane : ethyl acetate = 4:1) to obtain the target product (6.44 g) as an orange solid. MS (EI⁺): 456 [M⁺]
¹H-NMR (400 MHz, CDCl₃) : δ 0.14 (6H, s), 0.95 (9H, s), 4.89 (2H, s), 7.01 (1H, s), 7.02-7.05 (1H, m), 7.48 (1H, d, J = 7.3 Hz).

### <Example 58>

4-Hydroxymethyl-7-iodo-2-trifluoromethylpyrazolo[1,5-a]pyridin e

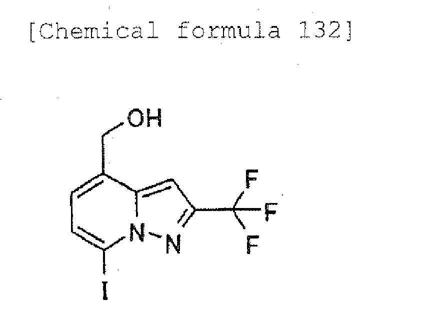

The compound of Example 57 (6.44 g) was dissolved in THF (50 mL), and tetrabutylammonium fluoride (a 1.0 mol/L THF solution, 17.0 mL) was added to the prepared solution at 0°C. The mixture was stirred at 0°C for 2 hours. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (4.47 g) as a white solid.
MS (EI⁺) : 342 [M⁺]
¹H-NMR (400 MHz, CDCl₃): δ 1.75 (1H, brs), 4. 92 (2H, d, J = 1.2 Hz), 7.04 (1H, d, J = 7.3 Hz), 7.11 (1H, s), 7.49 (1H, d, J = 7.3 Hz).

### <Example 59>

7-Iodo-2-trifluoromethylpyrazolo[1,5-a]pyridine-4-carbaldehyde

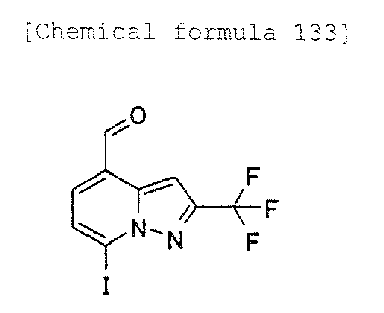

The compound of Example 58 (4.47 g) was dissolved in chloroform (60 mL), and activated manganese dioxide (8.54 g) was added to the prepared solution. The mixture was stirred at 50°C for 8 hours. Insoluble material was removed by filtration through Celite, and the solvent of the filtrate was evaporated under reduced pressure to obtain the target product (4.26 g) as a yellow solid.
MS (EI⁺): 340 [M⁺]
¹H-NMR (400 MHz, CDCl₃): δ 7.52 (1H, d, J = 7.3 Hz), 7.73 (1H, d, J = 7.3 Hz), 7.85 (1H, s), 10.10 (1H, s).

### <Example 60>

7-Methylsulfanyl-2-trifluoromethylpyrazolo[1,5-a]pyridine-4-ca rbaldehyde

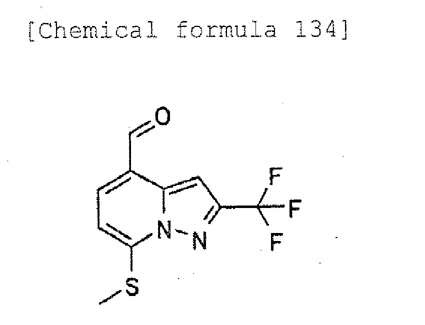

The compound of Example 59 (1.02 g) was dissolved in DMF (10 mL), and sodium thiomethoxide (252 mg) was added to the prepared solution. The mixture was stirred at 60°C for 2 hours. The reaction mixture was diluted with water and extracted with ethyl acetate, and the organic layer was washed with water and then saturated brine and dried over anhydrous sodium sulfate. The solvent was evaporated under reduced pressure, and the residue was purified by silica gel column chromatography (hexane : ethyl acetate = 2: 1) to obtain the target product (570 mg) as a yellow solid.
MS (EI⁺): 260 [M⁺]
¹H-NMR (400 MHz, CDCl₃): δ 2.72 (3H, s), 6.86 (1H, d, J= 8.0 Hz), 7.65 (1H, s), 7.80 (1H, d, J = 8.0 Hz), 10.05 (1H, s).

### <Example 61>

7-Methylsulfanyl-2-trifluoromethylpyrazolo[1,5-a]pyridine-4-ca rboxylic acid

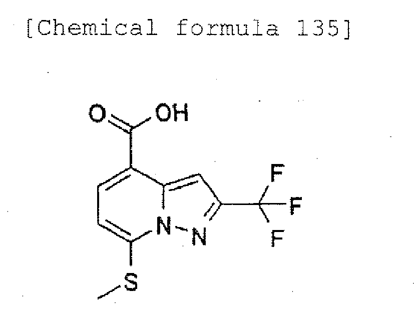

The compound of Example 60 (516 mg) was suspended in tert-butanol (6.0 mL) and water (2.0 mL). Sodium dihydrogenphosphate dihydrate (309 mg), 2-methyl-2-butene (0.94 mL), and sodium chlorite (448 mg) were added to the prepared solution, and the mixture was stirred at room temperature for 1.5 hours. A 10% aqueous sodium hydroxide solution was added to the resultant mixture to make it alkaline. The aqueous layer was washed with diethyl ether, and concentrated hydrochloric acid was added to the resultant aqueous layer to make it acidic. The precipitated solid was collected by filtration, washed with water, and dried to obtain the target product (210 mg) as a pale yellow solid.
¹H-NMR (400 MHz, DMSOd₆): δ 3.09 (3H, s), 7.54 (1H, s), 7.64 (1H, d, J = 7.3 Hz), 8.32 (1H, d, J = 7.3 Hz).

### <Example 62>

7-Methylamino-2-trifluoromethylpyrazolo[1,5-a]pyridine-4-carba ldehyde

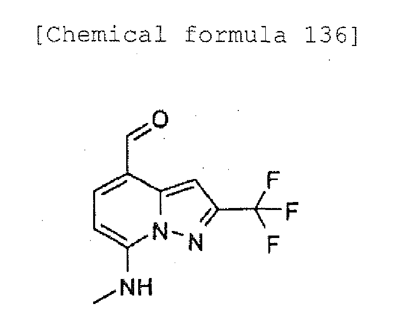

The compound of Example 59 (680 mg) was added to methylamine (a 2.0 mol/L THF solution, 20 mL), and the mixture was stirred in a sealed tube at 60°C for 16 hours. The solvent was evaporated, and the residue was purified by silica gel column chromatography (hexane : ethyl acetate = 3:1) to obtain the target product (460 mg) as a yellow solid.
MS (EI⁺) 243 [M⁺]
¹H-NMR (400 MHz), CDCl₃): δ 3.22 (3H, s), 6.12 (1H, d, J = 8.0 Hz), 6.75 (1H, brs), 7.55 (1H, s), 7.80 (1H, d, J = 8.0 Hz), 9.84 (1H, s).

### <Example 63>

7-(t-Butoxycarbonylmethyl-amino)-2-trifluoromethylpyrazolo[1,5-a]pyridine-4-carba ldehyde

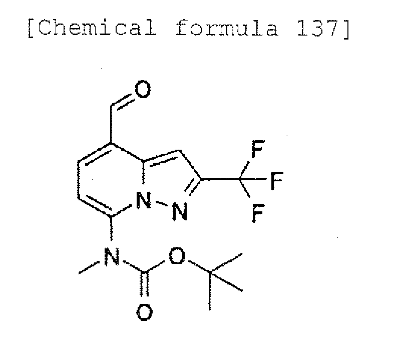

The compound of Example 62 (410 mg) was dissolved in acetonitrile (10 mL), and di-tert-butyl-dicarbonate (736 mg) and dimethylaminopyridine (8.4 mg) were added to the prepared solution. The mixture was stirred at room temperature for 3 days. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:1) to obtain the target product (561 mg) as an orange oil.
MS (EI⁺): 343 [M⁺]
¹H-NMR (400 MHz, CDCl₃) : δ 1.34 (9H, s), 3.40 (3H, s), 7.02 (1H, d, J = 8.0 Hz), 7.67 (1H, s), 7.84 (1H, d, J = 8.0 Hz), 10.08 (1H, s).

### <Example 64>

7-(t-Butoxycarbonyl-methyl-amino)-2-trifluoromethylpyrazolo[1, 5-a]pyridine-4-carboxylic acid

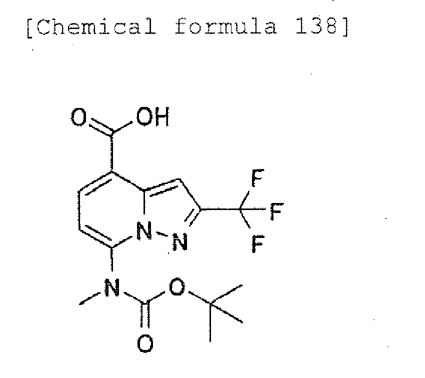

The compound of Example 63 (562 mg) was suspended in tert-butanol (9.0 mL) and water (3.0 mL). Sodium dihydrogenphosphate dihydrate (264 mg), 2-methyl-2-butene (0.81 mL), and sodium chlorite (535 mg) were added to the suspension, and the mixture was stirred at room temperature for 6 hours. A 10% aqueous sodium hydroxide solution was added to the mixture to make it alkaline. The aqueous layer was washed with diethyl ether, and concentrated hydrochloric acid was added to make the aqueous layer acidic. The precipitated solid was collected by filtration, washed with water, and dried to obtain the target product (84.7 mg) as a white solid.
MS (EI⁺): 359 [M⁺]
¹H-NMR (400 MHz, DMSO-d₆): δ 1.20 (9H, s), 3.28 (3H, s), 7.32 (1H, d, J = 8.0Hz), 7.46 (1H, s), 8.12 (1H, d, J = 8.0 Hz).

### <Example 65>

4-[1,3]Dioxan-2-yl -7-iodo-2-trifluoromethylpyrazolo[1,5-a]pyridine

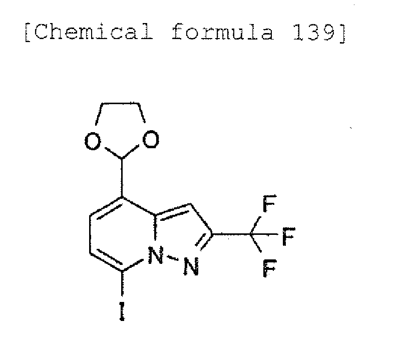

The compound of Example 59 (2.55 g) was dissolved in toluene (70 mL). Then, p-toluenesulfonic acid monohydrate (142 mg) and ethylene glycol (2.51 mL) were added to the prepared solution, and the mixture was heated to reflux for 18 hours with a Dean-Stark apparatus. After allowed to cool, the reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:1) to obtain the target product (2.75 g) as a yellow solid.
MS (EI⁺): 384 [M⁺]
¹H-NMR (400 MHz, CDCl₃): δ 4.11-4.15 (4H, m), 6.07 (1H, s), 7.11 (1H, d, J = 7.3 Hz), 7.20 (1H, s), 7.49 (1H, d, J = 7.3 Hz).

### <Example 66>

4-[1,3]Dioxan-2-yl -2-trifluoromethylpyrazolo[1,5-a]pyridine-7-carbaldehyde

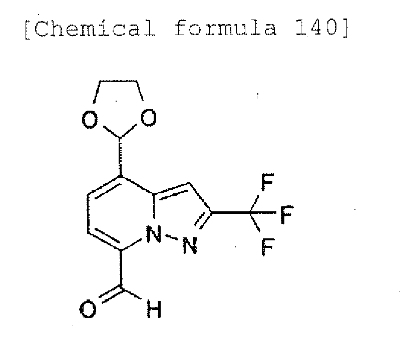

The compound of Example 65 (2.75 g) was dissolved in THF (30 mL) under argon atmosphere, and n-butyllithium (a 1.54 mol/L hexane solution, 5.6 mL) was added dropwise to the prepared solution at -78°C. The mixture was stirred at -78°C for 30 minutes. Ethyl formate (0.75 mL) was added to the reaction mixture, and the resultant mixture was stirred at room temperature for 30 minutes. A saturated aqueous ammonium chloride solution was added to the reaction mixture, and the resultant mixture was extracted with ethyl acetate. The organic layer was washed with water and then saturated brine and dried over anhydrous sodium sulfate. The solvent was evaporated under reduced pressure, and the residue was purified by silica gel column chromatography (hexane : ethyl acetate = 2:1) to obtain the target product (1.87 g) as a yellow solid.
MS (EI⁺): 286 [M⁺]
¹H-NMR (400 MHz, CDCl₃) δ 4.14 (4H, s), 6.12 (1H, s), 7.15 (1H, s), 7.49 (1H, d, J = 7.3 Hz), 7.63 (1H, d, J = 7.3 Hz), 10.94 (1H, s).

### <Example 67>

4-[1,3]Dioxan-2-yl-7-hydroxymethyl-2-trifluoromethylpyrazolo[1,5-a]pyridine

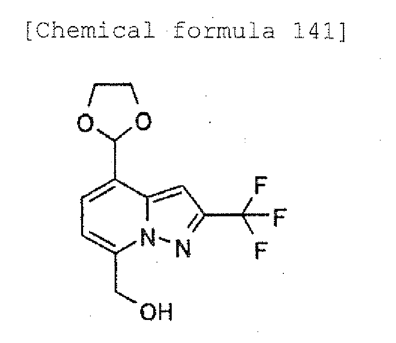

The compound of Example 66 (1.87 g) was dissolved in methanol (30 mL), and sodium borohydride (247 mg) was added to the prepared solution at 0°C. The mixture was stirred at 0°C for 1 hour. A saturated aqueous sodium hydrogen carbonate solution was added to the reaction mixture, and the resultant mixture was extracted with ethyl acetate. The extract layer was washed with water and then saturatedbrine and dried over anhydrous sodium sulfate. The solvent was evaporated under reduced pressure, and the residue was purified by silica gel column chromatography (hexane : ethyl acetate = 1:1) to obtain the target product (1.82 g) as a white solid.
MS (EI⁺): 288 [M⁺]
¹H-NMR (400 MHz, CDCl₃): δ 3.74 (1H, t, J = 6.7 Hz), 4.11-4.16 (4H, m), 5.08 (2H, d, J = 6.7 Hz), 6.08 (1H, s), 6.95 (1H, d, J = 6.7 Hz), 7.02 (1H, s), 7.40 (1H, d, J = 6.7 Hz).

### <Example 68>

7-Acetoxymethyl-4-[1,3]dioxan-2-yl-2-trifluoromethylpyrazolo[1,5-a]pyridine

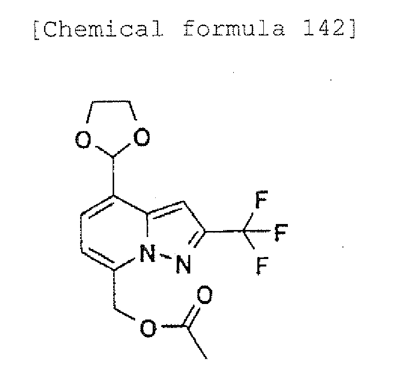

The compound of Example 67 (1.82 g) was dissolved in pyridine (20 mL), and acetic anhydride (1.2 mL) was added to the prepared solution. The mixture was stirred at room temperature for 30 minutes. The reaction mixture was diluted with water and extracted with ethyl acetate, and the organic layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (1.96 g) as a white solid.
MS (EI⁺): 330 [M⁺]
¹H-NMR (400 MHz, CDCl₃): δ 2.20 (3H, s), 4.12-4.16 (4H, m), 5.63 (2H, s), 6.08 (1H, s), 6.99 (1H, d, J = 7.3 Hz), 7.02 (1H, s), 7.39 (1H, d, J = 7.3 Hz).

### <Example 69>

7-Acetoxymethyl-2-trifluoromethylpyrazolo[1,5-a]pyridine-4-carbaldehyde

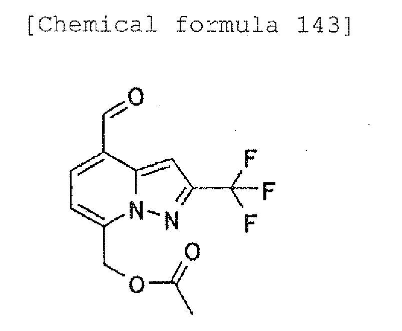

The compound of Example 68 (1.93 g) was dissolved in an acetone-water mixed solvent (2:1, 20 mL), and p-toluenesulfonic acid monohydrate (111 mg) was added to the prepared solution. The mixture was stirred at 700°C for 2 hours. The reaction mixture was diluted with water and extracted with ethyl acetate, and the extract layer was washed with water and then saturated brine and dried over anhydrous sodium sulfate. The solvent was evaporated under reduced pressure, and the residue was purified by silica gel column chromatography (hexane : ethyl acetate = 3:2) to obtain the target product (1.43 g) as a yellow solid.
MS (EI⁺): 286 [M⁺]
¹H-NMR (400 MHz, CDCl₃): δ 2.26 (3H, s), 5.72 (2H, s), 7.18 (1H, d, J = 7.3 Hz), 7.67 (1H, s), 7.85 (1H, d, J = 7.3 Hz), 10.11 (1H, s).

### <Example 70>

7-Acetoxymethyl-2-trifluoromethylpyrazolo[1,5-a]pyridine-4-carboxylic acid

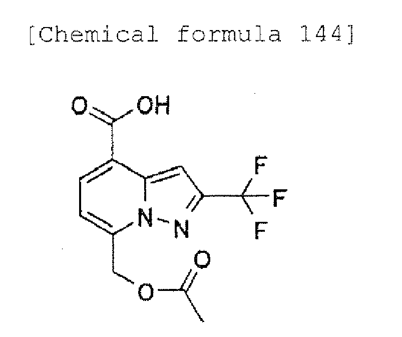

The compound of Example 69 (1.22 g) was dissolved in DMF (22 mL), and pyridinium dichromate (12.9 g) and Celite (200 mg) were added to the prepared solution. The mixture was stirred at room temperature for 2 days. Insoluble material was removed by filtration through Celite, and the filtrate was diluted with water and extracted with ethyl acetate. The extract layer was washed with water and then saturated brine and dried over anhydrous sodium sulfate, and the solvent was evaporated under reduced pressure to obtain the target product (1.08 g) as a brown solid.
MS (EI⁺): 302 [M⁺]
¹H-NMR (400 MHz, DMSO-d₆): δ 2.18 (3H, s), 5.60 (2H, s), 7.34 (1H, d, J = 7.3 Hz), 7.47 (1H, s), 8.13 (1H, d, J = 7.3 Hz), 13.78 (1H, brs).

### <Example 71>

4-(t-Butyldimethylsilyloxymethyl)-2-trifluoromethylpyrazolo[1, 5-a]pyridine-7-carbaldehyde

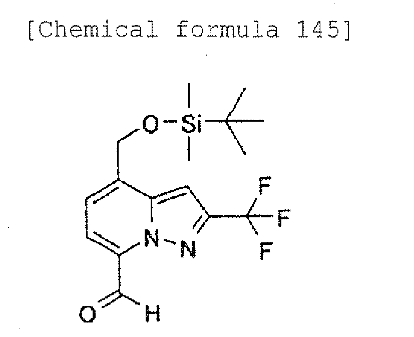

n-Butyllithium (a 2.67 mol/L hexane solution, 14.0 mL) was added dropwise to a solution of the compound of Example 56 (12.4 g) in THF (200 mL) at -78°C under argon atmosphere, and the mixture was stirred at -780°C for 30 minutes. The prepared solution was added dropwise to a solution of ethyl formate (9.06 mL, 113 mmol) in THF (100 mL) at -78°C. After the mixture was stirred at room temperature for 30 minutes, a saturated aqueous ammonium chloride was added thereto, and the resultant mixture was extracted with ethyl acetate (400 mL). The extract layer was washed with water and saturated brine and dried over anhydrous sodium sulfate. The solvent was evaporated under reduced pressure, and the residue was purified by silica gel column chromatography (hexane : ethyl acetate = 15:1) to obtain the target product (12.3 g, crude product) as a yellow solid.

### <Example 72>

4-(t-Butyldimethylsilyloxymethyl)-7-hydroxymethyl-2-trifluorom ethylpyrazolo[1,5-a]pyridine

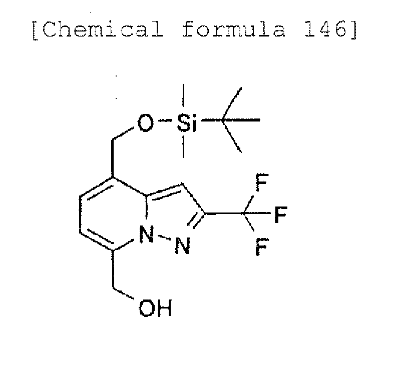

Sodium borohydride (1.56 g) was added to a solution of the compound of Example 71 (12.3 g) in methanol (200 mL) at 0°C, and the mixture was stirred at 0°C for 1 hour. A saturated aqueous ammonium chloride solution was added to the reaction mixture, and the resultant mixture was concentrated under reduced pressure and was extracted with ethyl acetate (700 mL). The extract layer was washed with water and saturated brine and dried over anhydrous sodium sulfate. The solvent was evaporated under reduced pressure, and the residue was purified by silica gel column chromatography (hexane : ethyl acetate = 9:1) to obtain the target product (9.86 g) as a white solid.
¹H-NMR (400 MHz, CBCl₃): δ 0.14 (6H, s), 0.96 (9H, s), 4.91 (2H, d, J = 1.2 Hz), 5.06 (2H, s), 6.85 (1H, s), 6.94 (1H, d, J = 7.3 Hz), 7.30 (1H, dt, J = 7.3, 1.2 Hz).

### <Example 73>

4-(t-Butyldimethylsilyloxymethyl)-7-methoxymethyl-2-trifluorom ethylpyrazolo[1,5-a]pyridine

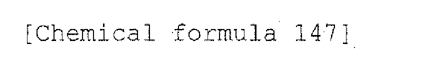

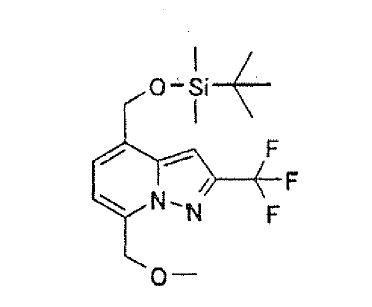

Silver oxide (30.0 g) and iodomethane (16.1 mL) were added to a solution of the compound of Example 72 (9.53g) in acetonitrile (300 mL), and the mixture was stirred at room temperature for 85 hours. Insoluble material was removed by filtration through Celite. The filtrate was concentrated under reduced pressure, and the residue was purified by silica gel column chromatography (hexane : ethyl acetate = 15:1) to obtain the target product (8.76 g) as a fluorescent pale yellow solid.
¹H-NMR (400 MHz, CDCl₃): δ 0.13 (6H, s), 0.95 (9H, s), 3.60 (3H, s), 4.91 (2H, s), 4.97 (2H, s), 6.83 (1H, s), 7.07 (1H, d, J = 7.3 Hz), 7.32 (1H, d, J = 7.3 Hz).

### <Example 74>

4-Hydroxymethyl-7-methoxymethyl-2-trifluoromethylpyrazolo[1,5-a]pyridine

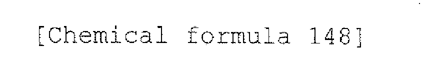

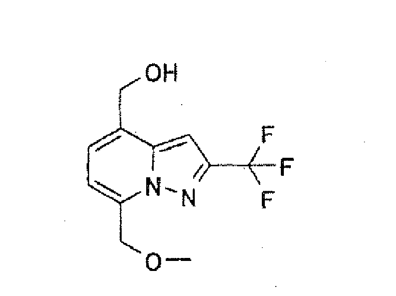

Tetrabutylammonium fluoride, (at 1 mol/L THF, solution, 35.1 mL) was added dropwise to a solution of the compound of Example 73 (8.76 g) in THF (120 mL) at 0°C, and the mixture was stirred at 0°C for 30 minutes. Water was added to the reaction mixture, and the resultant mixture was extracted with ethyl acetate (300 mL). The extract layer was washed with water and saturated brine and dried over anhydrous sodium sulfate. The solvent was evaporated under reduced pressure, and the residue was purified by silica gel column chromatography (hexane : ethyl acetate = 3:1) to obtain the target product (6.00 g) as a white solid.
¹H-NMR (400 MHz), CDCl₃): δ 1.79 (1H, br, s), 3.61 (3H, s), 4.93 (2H, s), 4.98 (2H, s), 6.93 (1H, s), 7.07 (1H, d, J = 7.3 Hz), 7.31 (1H, d, J = 7.3 Hz).

### <Example 75>

7-Methoxymethyl-2-trifluoromethylpyrazolo[1,5-a]pyridine-4-carbaldehyde

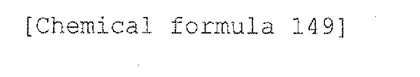

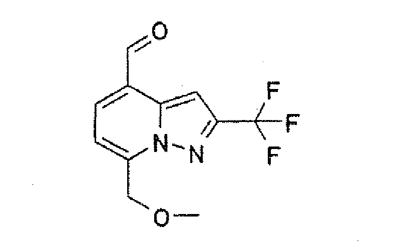

Activated manganese dioxide (20.0 g) was added to a solution of the compound of Example 74 (6.00 g) in chloroform (120 mL), and the mixture was stirred at 50°C for 5 hours. Insoluble material was removed by filtration through Celite, and the filtrate was concentrated under reduced pressure to obtain the target product (5.74 g) as a pale yellow solid.
¹H-NMR (400 MHz, CDCl₃): δ 3.65 (3H, s), 5.06 (2H, s), 7.32 (1H, d, J = 7.3 Hz), 7.65 (1H, s), 7.89 (1H, d, J = 7.3 Hz), 10.10 (1H, s).

### <Example 76>

7-Methoxymethyl-2-trifluoromethylpyrazolo[1,5-a]pyridine-4-carboxylic acid

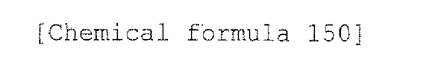

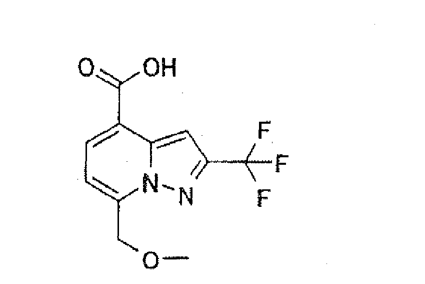

A solution of sodium hydroxide (310 mg) in water (5 mL) was added to a solution of silver nitrate (658 mg) in water (5 mL), and the compound of Example 75 (500 mg) was added to the mixture at 0°C under stirring. The resultant mixture was stirred at room temperature for 1 hour. Insoluble material was removed by filtration through Celite, and the resultant mixture was washed with hot water. The washings were combined with the filtrate, and the combined solution was acidified with a 1 mol/L aqueous hydrochloric acid solution. Ethyl acetate (100 mL) was added to the resultant solution, and insoluble material was removed by filtration through Celite. The organic layer of the filtrate was collected, washed with water and saturated brine, and dried over anhydrous sodium sulfate. The solvent was evaporated under reduced pressure to obtain the target product (470 mg) as a pale yellow solid.
¹H-NMR (400 MHz, DMSO-d₆): δ 3.53 (3H, s), 4.99 (2H, s), 7.30 (1H, d, J = 7.3 Hz), 7.46 (1H, s), 8.17 (1H, d, J = 7.3 Hz), 13.72 (1H, br s).

### <Example 77>

1-(7-Methoxy-2-trifluoromethylpyrazolo[1,5-a]pyridin-4-yl )-2-(pyridin-4-yl )ethanol

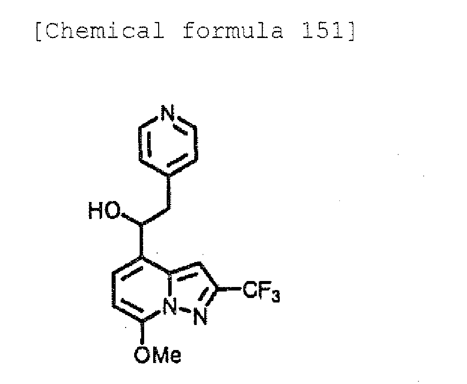

Diisopropylamine (257 µL) was dissolved in THF, (10 mL) under a stream of argon, and the solution was cooled to 0°C. A 1.54 M/L n-butyllithium-hexane solution (1.2 mL) was added dropwise to the prepared solution, and the mixture was stirred at 0°C for 30 minutes. After the mixture was cooled to -78°C, 4-picoline (160 µL) was added dropwise to the mixture, and the resultant mixture was stirred for 30 minutes and stirred at 0°C for 3 minutes. After the mixture was again cooled to -78°C, the compound of Example 27 (300 mg) was added thereto, and the resultant mixture was stirred for 30 minutes. A saturated aqueous ammonium chloride solution was added to the mixture. The resultant mixture was extracted with ethyl acetate, washed with water and saturated brine, and dried over anhydrous sodium sulfate, and the solvent was concentrated. The residue was purified by silica gel column chromatography (ethyl acetate -> ethyl acetate : methanol = 10:1) to obtain the target product (313 mg) as a white powder. Elemental Analysis (%): for C₁₆H₁₄F₃N₃O₂

| | C | H | N |
|---|---|---|---|
| Calcd. | 56.97 | 4.18 | 12.45 |
| Found | 56.62 | 4.08 | 12.28 |

MS(EI⁺): 337 [M+]
HRMS(EI⁺): 337.1024 (- 1.4mmu)
¹H-NMR (400 MHz, CDCl₃): δ 2.56 (1H, brs), 3.11-3.20 (2H, m), 4.17 (3H, s), 5.18 (1H, dd, J = 5.6, 7.2 Hz), 6.18 (1H, d, J = 8.0 Hz), 6.97 (H, s), 7.10 (2H, d, J = 5.5 Hz), 7.14 (1H, d, J = 8.0 Hz), 8.48 (2H, d, J = 5.5 Hz)

### <Example 78>

7-Methoxy-4-[(2-pyridin-4-yl )vinyl]-2-trifluoromethylpyrazolo[1,5-a]pyridine

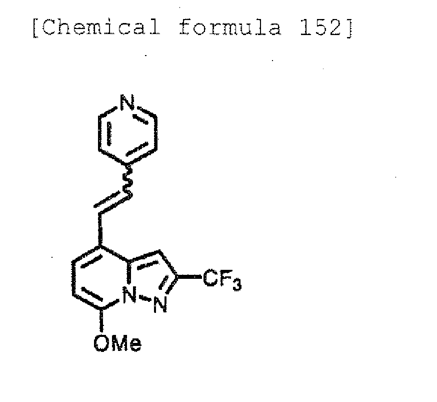

The compound of Example 77 (291 mg) was dissolved in toluene (8.0 mL). Methanesulfonic acid (280 µL) was added dropwise to the prepared solution, and the mixture was stirred at room temperature for 2 hours. A 10% aqueous solution of sodium hydroxide was added to the reaction mixture. The resultant mixture was extracted with ethyl acetate, washed with water and saturated brine, and dried over anhydrous sodium sulfate, and the solvent was concentrated. The residue was purified by silica gel column chromatography (ethyl acetate) to obtain the target product (243 mg) as an orange powder.
Elemental Analysis (%): for C₁₆H₁₂F₃N₃O

| | C | H | N |
|---|---|---|---|
| Calcd. | 60.19 | 3.79 | 13.16 |
| Found | 60.06 | 3.76 | 12.95 |

MS (EI⁺): 319 [M+]
¹H-NMR (400 MHz, COCl₃): δ 4.23 (3H, s), 6.33 (1H, d, J = 8.0 Hz), 7.08-7.12(2H, m), 7.39-7.43 (3H, m), 7.48 (1H, d, J = 8.0 Hz), 8.62 (2H, brs)

### <Example 79>

7-Methoxy-4-[(2-pyridin-4-yl )ethyl]-2-trifluoromethylpyrazolo[1,5-a]pyridine

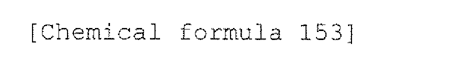

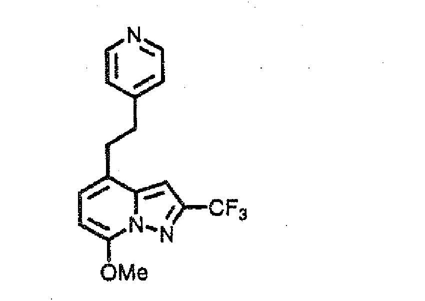

The compound of Example 78 (77.3 mg) was dissolved in ethanol (5.0 mL). 10% Pd/C (7.0 mg) was added to the prepared solution, which was vigorously stirred under hydrogen atmosphere at room temperature for 6 hours. After the catalyst was removed using Celite, the organic layer was concentrated, and the obtained powder was washed with diisopropyl ether and dried to obtain the target product (48.0 mg) as a white powder.
Elemental Analysis (%): for C₁₆H₁₄F₃N₃O

| | C | H | N |
|---|---|---|---|
| Calcd. | 59.81 | 4.39 | 13.08 |
| Found | 59.61 | 4.29 | 12.90 |

MS(EI⁺): 321 [M+]
¹H-NMR (400 MHz, CDCl₃): δ 3.00-3.13 (4H, m), 4.15 (3H, s), 6.12 (1H, d, J = 8.0 Hz), 6.81 (1H, s), 6.87 (1H, d, J = 8.0 Hz), 7.07 (2H, d , J = 6.1 Hz), 8.50 (2H, d, J = 6.1 Hz)

### <Example 80>

1-(7-Methoxy-2-trifluoromethylpyrazolo[1,5-a]pyridin-4-yl )-2-(pyridin-4-yl )ethanone

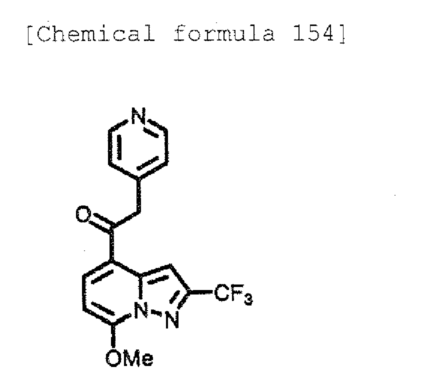

The compound of Example 77 (252 mg) was dissolved in acetone (12 mL). A solution of chromium oxide (149 mg) in sulfuric acid (130 µL) and water (0.8mL) was added dropwise to the prepared solution, and the mixture was stirred at room temperature for 3 hours. The reaction mixture was filtrated through Celite. The organic layer was separated, and the aqueous layer was extracted with ethyl acetate. The organic layer was washed with water and saturated brine and dried over anhydrous sodium sulfate, and the solvent was concentrated. The residue was purified by silica gel column chromatography (ethyl acetate) to obtain the target product (10.0 mg) as a white powder.
MS(EI⁺): 335 [M+]
HRMS(EI⁺): 335.0891 (+ 1.0mmu)
¹H-NMR (400 MHz, CDCl₃): δ 4.28 (3H, s), 4.31 (2H, s), 6.31 (1H, d, J = 8.0 Hz), 7.23 (2H, d, J = 6.1 Hz), 7.68 (H, s), 8.03 (1H, d, J = 8.0 Hz), 8.59 (2H, d, J = 6.1 Hz)

### <Example 81>

(7-Methoxy-2-trifluoromethylpyrazolo[1,5-a]pyridin-4-yl )phenylmethanol

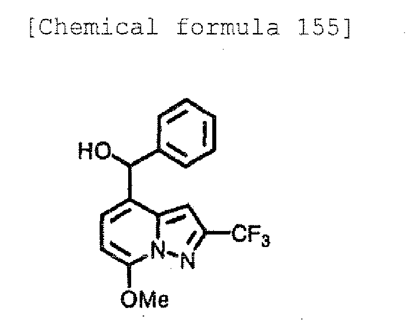

The compound of Example 27 (515 mg) was dissolved in THF (10 mL) under a stream of argon, and the solution was cooled to -78°C. Subsequently, a 1.02 mol/L phenylmagnesium bromide-THF solution (2.48 mL) was added dropwise to the prepared solution, and the mixture was stirred at room temperature for 2 hours. A saturated aqueous ammonium chloride solution was added to the mixture. The resultant mixture was extracted with ethyl acetate, washed with water and saturated brine, and dried over anhydrous sodium sulfate, and the solvent was concentrated. The residue was purified by silica gel column chromatography (hexane : ethyl acetate = 1:1) to obtain the target product (646 mg) as a yellow powder.
MS (EI⁺): 322 [M+]
¹H-NMR (400 MHz, COCl₃): δ 2.31 (1H, brs), 4.17 (3H, s), 6.04( 1H, s), 6.24 (1H, d, J = 8.0 Hz), 6.73 (1H, s), 7.30-7.43 (6H, m)

### <Example 82>

(7-Methoxy-2-trifluoromethylpyrazolo[1,5-a]pyridin-4-yl )lmethanol

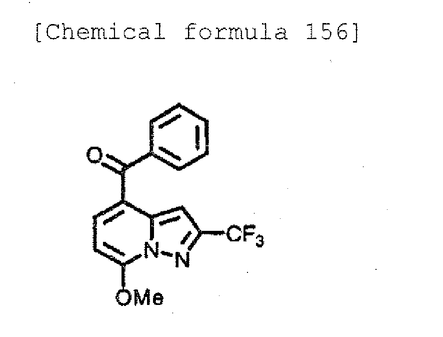

The compound of Example 81 (640 mg) was dissolved in toluene (10 mL). Activated manganese dioxide (1.73 g) was added to the prepared solution, and the mixture was stirred at 100°C for 1 hour. The reaction mixture was filtrated through Celite, and the filtrate was concentrated to obtain the target product (547 mg) as a white powder.
Elemental Analysis (%): for C₁₆H₁₁F₃N₂O₂

| | C | H | N |
|---|---|---|---|
| Calcd. | 60.00 | 3.46 | 8.74 |
| Found | 59.71 | 3.44 | 8.63 |

MS(EI⁺): 320 [M+]
¹H-NMR (400 MHz, CDCl₃): δ 4.28 (3H, s), 6.30 (1H, d, J= 7.9 Hz), 7.50-7.54 (3H, m), 7.60-7.64 (1H, m), 7.73-7.75 (3H, m)

### <Example 83>

1-(7-Methoxy-2-trifluoromethylpyrazolo[1,5-a]pyridin-4-yl )-1-phenyl-2-(pyridin-4-yl )ethanol

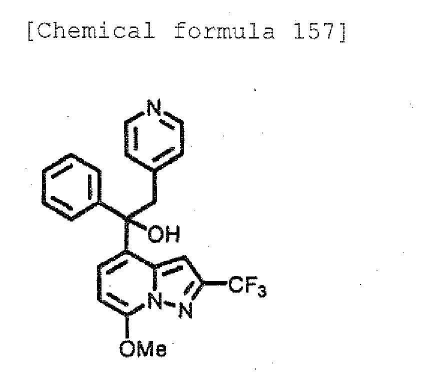

The compound of Example 82 was reacted in the same manner as in Example 77 to obtain the target product as a white powder.
Elemental Analysis (%): for C₂₂H₁₈F₃N₃O₂

| | C | H | N |
|---|---|---|---|
| Calcd. | 63.92 | 4.39 | 10.16 |
| Found | 63.51 | 4.35 | 9.98 |

MS (CI⁺) : 414 [M+]
HRMS(EI⁺): 414.4070 (+ 3.2mmu)
¹H-NMR (400 MHz, CDCl₃): δ 2.41 (1H, brs), 3.69 (2H, dd, J = 12.8, 20.8 Hz), 4.17 (3H, s), 6.17 (1H, d, J = 8.0 Hz), 6.42 (1H, s), 6.72-6.74 (2H, m), 7.31-7.34 (6H, m), 8.32-8.33 (2H, m)

### <Examples 84 and 85>

(E)-7-methoxy-4-[1-phenyl-2-(pyridin-4-yl ) vinyl]-2-trifluorom ethylpyrazolo[1,5-a]pyridine and (Z)-7-methoxy-4-[1-phenyl-2-(pyridin-4-yl )vinyl]-2-trifluorom ethylpyrazolo[1,5-a]pyridine

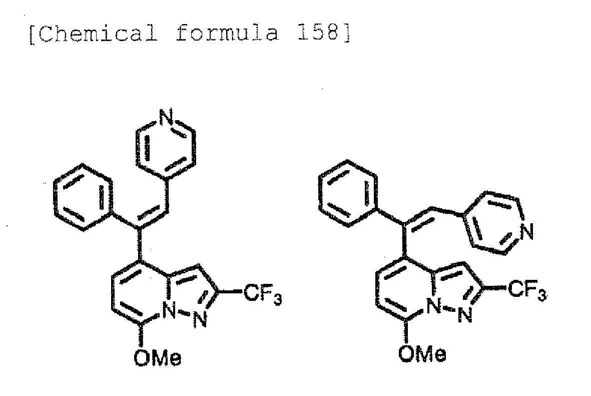

The compound of Example 83 (211 mg) was dissolved in toluene (5.0 ml). Methanesulfonic acid (0.33 mL) was added dropwise to the prepared solution, and the mixture was stirred at 65°C for 40 minutes. A 10% aqueous solution of sodium hydroxide was added to the reaction mixture. The resultant mixture was extracted with ethyl acetate, washed with water and saturated brine, and dried over anhydrous sodium sulfate, and the solvent was concentrated. The residue was purified by silica gel column chromatography (hexane : ethyl acetate = 1:2) to give the E-form (84.4 mg) as a white powder (Example 84) and the Z-form (12.3 mg) as a yellow powder (Example 85).

### Example 84

MS(EI⁺) : 395 [M+]
HRMS (EI⁺) : 395.1282 (+ 3.7 mmu)
¹H-NMR (400 MHz, CDCl₃): δ 4.20 (3H, s), 6.25 (1H, d, J = 8.0 Hz), 6.51 (1H, s), 6.92-6.95 (3H, m), 7.14 (1H, d, J = 8.0 Hz), 7.18-7.21 (2H, m), 7.32-7.34 (3H, m), 8.40 (2H, d, J = 5.5 Hz)

### Example 85

MS (EI⁺): 395 [M+]
HRMS (EI⁺): 395.1214 (- 3.1 mmu)
¹H-NMR (400 MHz, CDCl₃): δ 4.22 (3H, s), 6.23 (1H, d, J = 7.3 Hz), 6.32 (1H, s), 6.87 (2H, d, J = 5.5 Hz), 7.06-7.09 (2H, m), 7.33-7.37 (5H, m), 8.37 (2H, d, J = 5.5 Hz)

### <Example 86>

7-Methoxy-4-[1-phenyl-2-(pyridin-4-yl )ethyl]-2-trifluoromethylpyrazolo[1,5-a]pyridine

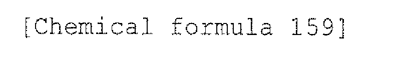

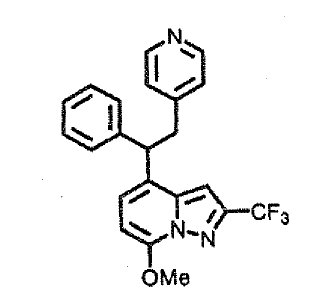

The compound of Example 84 (80.7 mg) was dissolved in ethanol (5.0 mL). 10% Pd/C (12.0 mg) was added to the prepared solution, which was vigorously stirred under hydrogen atmosphere at room temperature for 3 days. After the catalyst was removed using Celite, the organic layer was concentrated, and the residue was purified by silica gel column chromatography (hexane : ethyl acetate = 1:3) to obtain the target product (24.0 mg) as a colorless oil.
MS(EI⁺): 397 [M+]
HRMS (EI⁺): 397.11392 (- 1.0 mmu)
¹H-NMR (400 MHz, CDCl₃): δ 3.39-3.45 (2H, m), 4.15(3H, s), 4.44 (1H, t, J= 7.3 Hz), 6.21 (1H, d, J = 7.9 Hz), 6.62 (1H, s), 6.91-6.94 (2H, m), 7.14-7.28 (6H, m), 8.40 (2H, d, J = 6.1 Hz)

### <Example 87>

2-(3,5-Dichloropyridin-4-yl )-1-(7-methoxy-2-trifluoromethylpyrazolo[1,5-a]pyridin-4-yl )ethanol

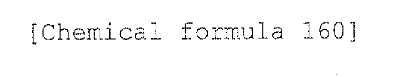

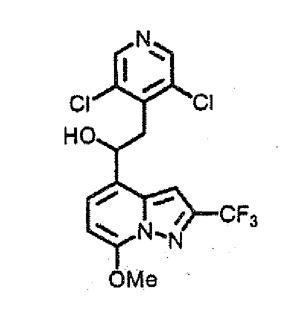

Diisopropylamine (86 µL) was dissolved in tetrahydrofuran (5.0 mL) under a stream of argon. A 1.54 mol/L n-butyllithium-hexane solution (0.40 mL) was added dropwise to the prepared solution at 0°C, and the mixture was stirred at 0°C for 30 minutes. After the mixture was cooled to -78°C, 3,5-dichloro-4-methylpyridine (100 mg) was added dropwise thereto, and the resultant mixture was stirred at -78°C for 30 minutes and further stirred at 0°C for 3 minutes. After the mixture was again cooled to -78°C, the compound of Example 27 (101 mg) was added thereto, and the resultant mixture was stirred for 30 minutes. A saturated aqueous ammonium, chloride solution was added to the mixture. The resultant mixture was extracted with ethyl acetate, washed with water and saturated brine, and dried over anhydrous sodium sulfate, and the solvent was concentrated. The residue was purified by silica gel column chromatography (hexane : ethyl acetate = 3:1) to obtain the target product (138 mg) as a white powder.
MS(EI⁺): 405 [M+]
HRMS(EI⁺): 405 0244 (- 1.4 mmu)
¹H-NMR (400 MHz, CDCl₃): δ 3.41 (1H, dd, J = 4.8, 13.5 Hz), 3.62 (1H, dd, J = 8.5, 13.5 Hz), 4.19 (3H, s), 5.37 (1H, dd, J = 4.8, 8.5 Hz), 6.21 (1H, d, J = 7.3 Hz), 7.07 (1H, s), 7.24 (1H, d, J = 7.3 Hz), 8.49 (2H, brs)

### <Example 88>

2-(3,5-Dichloropyridin-4-yl )-1-(7-methoxy-2-trifluoromethylpyrazolo[1,5-a]pyridin-4-yl )ethanone

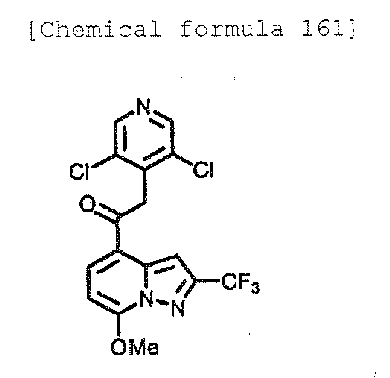

The compound of Example 87 (300 mg) was dissolved in acetone (10 mL). A solution of chromium oxide (148 mg) in sulfuric acid (130 µL) and water (2.0mL) was added dropwise to the prepared solution, and the mixture was stirred at room temperature for 2.5 hours. The reaction mixture was filtrated through Celite. The organic layer was separated, and the aqueous layer was extracted with ethyl acetate. The organic layer was washed with water and saturated brine and dried over anhydrous sodium sulfate, and the solvent was concentrated. The residue was purified by silica gel column chromatography (hexane : ethyl acetate = 1:1) to obtain the target product (142 mg) as a white powder.
Elemental Analysis (%): for C₁₆H₁₀Cl₂F₃N₃O₂

| | C | H | N |
|---|---|---|---|
| Calcd. | 47.55 | 2.49 | 10.40 |
| Found | 47.46 | 2.53 | 10.48 |

MS (EI⁺): 404 [M+]
¹H-NMR (400 MHz, CDCl₃): δ 4.32 (3H, s), 4.71 (2H, s), 6.40 (1H, d, J = 8.6 Hz), 7.64 (1H, s), 8.19 (1H, d, J = 8.6 Hz), 8.57 (2H, brs)

### <Example 89>

(E)-4-[2-(3,5-dichloropyridin-4-yl )vinyl]-7-methoxy-2-trifluoromethylpyrazolo[1,5-a]pyridine

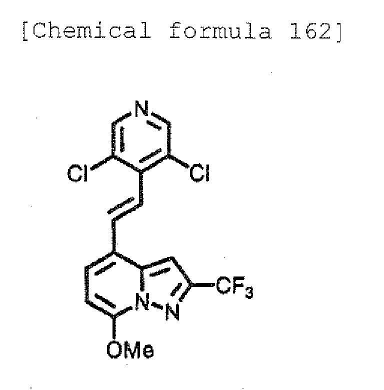

The compound of Example 87 (406 mg) was dissolved in tetrahydrofuran (15 mL). Methanesulfonic acid (0.32 mL) was added dropwise to the prepared solution, and the mixture was stirred at 70°C for 3 hours. A 10% aqueous solution of sodium hydroxide was added to the reaction mixture. The resultant mixture was extracted with ethyl acetate, washed with water and saturated brine, and dried over anhydrous sodium sulfate, and the solvent was concentrated. The residue was purified by silica gel column chromatography (hexane : ethyl acetate = 3:1) to obtain the target product (291 mg) as a yellow powder.
MS (EI⁺): 387 [M+]
HRMS(EI⁺): 387.0136 (- 1.7 mmu)
¹H-NMR (400 MHz, CDCl₃): δ 4.24 (3H, s), 6.35 (1H, d, J = 7.9 Hz), 7.10(1H, s), 7.20 (1H, d, J = 17.1 Hz), 7.51 (1H, d, J = 7.9 Hz), 7.63 (1H, d, J = 17.1 Hz), 8.55 (2h, brs)

### <Example 90>

1-(7-Methoxy-2-trifluoromethylpyrazolo[1,5-a]pyridin-4-yl )-2-(pyridin-4-yl )-ethanone

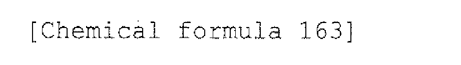

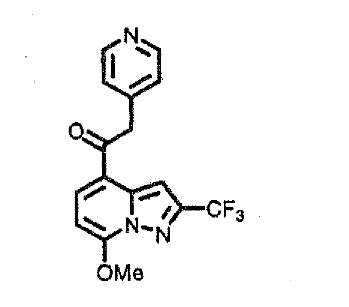

The compound of Example 35 (500mg) was dissolved in DMF (9. 60 mL) under argon atmosphere. Potassium carbonate (531 mg) and iodoethane (231 µL) were added to the prepared solution under cooling with ice, and the mixture was stirred at room temperature for 5 hours. Dilute hydrochloric acid was added to the reaction mixture under cooling with ice, and the resultant mixture was extracted with ethyl acetate. The organic layer was washed with saturated brine and dried over anhydrous sodium sulfate. The solvent was evaporated, and the residue was purified by silica gel chromatography (hexane : ethyl acetate = 2:1) to obtain an ester (481 mg) as a white powder. The obtained ester was used in the following reaction. Diisopropylamine (428 µL) was dissolved in tetrahydrofuran (20.0 mL) under argon atmosphere. A 1.58 mol/L n-butyllithium-hexane solution (1.94 mL) was added dropwise to the prepared solution at 0°C, and the mixture was stirred at 0°C for 30 minutes. Subsequently, 4-picoline (297 mg) was added dropwise to the mixture at -78°C, and the resultant mixture was stirred for 2 hours. The reaction mixture was added dropwise to a solution of the ester (294 mg) obtained by the above procedure in tetrahydrofuran (10 mL) at -78°C, and the mixture was stirred at -78°C for 1 hour. A saturated aqueous ammonium chloride solution was added to the reaction mixture, and the resultant mixture was extracted with ethyl acetate. The extract layer was washed with saturated brine and dried over anhydrous sodium sulfate. The solvent was evaporated, and the residue was purified by silica gel chromatography (ethyl acetate -> ethyl acetate : methanol = 20:1) to obtain the target product (66.6 mg) as a white powder.
MS (EI⁺): 335 [M+]
HRMS (EI⁺): 335.0852 (- 3.0 mmu)
¹H-NMR (400 MHz, CDCl₃): δ 4.29 (3H, s), 4.32 (2H, s), 6.33 (1H, d, J = 8.0 Hz), 7.25 (2H, d, J = 5.5 Hz), 7.63 (1H, s), 8.05 (1H, d, J = 8.0 Hz), 8.60 (2H, d, J = 5.5 Hz).

### <Example 91>

4-(2-(7-Methoxy-2-(trifluoromethyl)pyrazolo[1,5-a]pyridin-4-yl )ethyl)pyridine 1-oxide

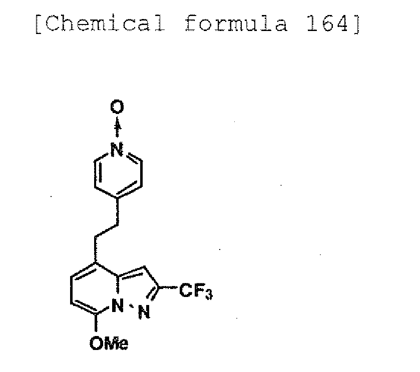

70% m-Chloroperbenzoic acid (163 mg) was added to a solution of the compound of Example 79 (177 mg) in chloroform (1.1 mL), and the mixture was stirred at room temperature for 1.5 hours. A saturated aqueous sodium thiosulfate solution was added to the reaction mixture, and the resultant mixture was extracted three times with ethyl acetate. The extract layer was washed with a saturated aqueous sodium hydrogen carbonate solution and then saturated brine and dried over anhydrous sodium sulfate. The solvent was evaporated under reduced pressure. Subsequently, the residue was purified by silica gel column chromatography (NH type, product of Fuji Silysia Chemical Ltd., ethyl acetate : methanol = 10 : 1), and the purified product was washed with diisopropyl ether to obtain the target product (127 mg) as a white solid.
Melting point: 203-205°C.
IR (KBr): 3397, 1636, 1546, 1489, 1352, 1246, 1165, 1120, 961, 800 cm⁻¹.
¹H NMR (CDCl₃, 400 MHz) d: 3.00-3.13 (4H, m), 4.15 (3H, s), 6.12 (1H, d, J = 7.9 Hz), 6.81 (1H, d, J = 7.9 Hz), 6.83 (1H, s), 6.99 (2H, d, J = 6.7 Hz), 8.09-8.12 (2H, m).
CIMS (+): 338 [M+H]⁺.
Elemental Analysis (%): Found C 56.76%, H 4.16%, N 12.31%, Calcd. for C₁₆H₁₄Cl₂F₃N₃O₂ C 56.87%, H 4.18%, N 12.46%.

### <Example 92>

3,5-Dichloro-4-(2-(7-methoxy-2-(trifluoromethyl)pyrazolo[1, 5-a]pyridin-4-yl )-2-oxoethyl)pyridine 1-oxide

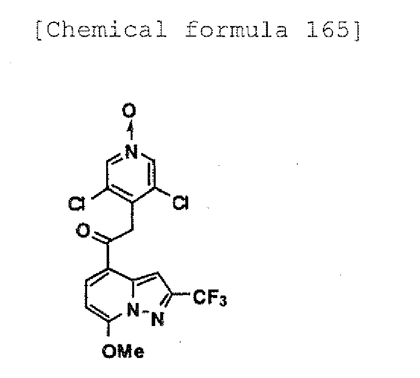

70% m-Chloroperbenzoic acid (730 mg) was added to a solution of the compound of Example 88 (1.00 g) in chloroform (5.00 mL), and the mixture was stirred at room temperature for 5.5 hours. A saturated aqueous sodium thiosulfate solution was added to the reaction mixture, and the resultant mixture was extracted three times with ethyl acetate. The combined extract layer was washed with a saturated aqueous sodium hydrogen carbonate solution and then saturated brine and dried over anhydrous sodium sulfate. The solvent was evaporated under reduced pressure, and the residue was purified by silica gel column chromatography (ethyl acetate : methanol = 10 : 1). The purified product was washed with diisopropyl ether to obtain the target product (630 mg) as a yellow solid. Melting point: 204-208°C (d).
¹H NMR (CDCl₃, 400 MHz) d: 4.33 (3H, s), 4.64 (2H, s), 6.40 (1H, d, J = 8.6 Hz), 7.62 (1H, s), 8.18 (1H, d, J = 8.6 Hz), 8.25 (2H, s).
CIMS (+): 420 [M+H]⁺.
Elemental Analysis (%): Found C 45.79%, H 2.47%, N 9.89%, Calcd. for C₁₆H₁₀Cl₂F₃N₃O₃ C 45.74%, H 2.40%, N 10.00%.

### <Example 93>

4-(2-(7-Methoxy-2-(trifluoromethyl)pyrazolo[1,5-a]pyridin-4-yl)-2-oxoethyl)pyridine 1-oxide

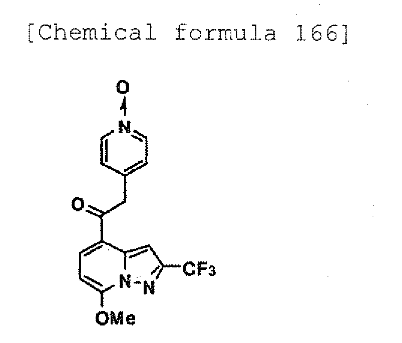

70% m-Chloroperbenzoic acid (478 mg) was added to a solution of the compound of Example 90 (325 mg) in chloroform (5.0 mL), and the mixture was stirred at room temperature for 1.5 hours. A saturated aqueous sodium thiosulfate solution was added to the reaction mixture, and the resultant mixture was extracted three times with ethyl acetate. The extract layer was washed with a saturated aqueous sodium hydrogen carbonate solution and then saturated brine and dried over anhydrous sodium sulfate. The solvent was evaporated under reduced pressure, and the residue was purified by silica gel column chromatography (chloroform : methanol = 5:1). The purified product was washed with diisopropyl ether to obtain the target product (43.0 mg) as a brown solid.
Melting point: 157-161°C (d).
IR (KBr): 3448, 1624, 1560, 1500, 1229, 1182, 1127, 964, 805 cm⁻¹. ¹H NMR (CDCl₃, 400 MHz) d: 4.306 (3H, s), 4.314 (2H, s), 6.35 (1H, d, J = 7.9 Hz), 7.21 (2H, d, J = 6.7 Hz), 7.66 (1H, s), 8.05 (1H, d, J = 7.9 Hz), 8.20 (2H, d, J = 6.7 Hz).
EIMS (+): 351 [M]⁺.
Elemental Analysis (%): Found C 53.83%, H 3.42%, N 11.45%, Calcd. for C₁₆H₁₂F₃N₃O₃·0.5H₂O C 53.74%, H 3.58%, N 11.75%.

### <Example 94>

4-(Chloromethyl)-7-methoxy-2-trifluoromethylpyrazolo[1,5-a]pyridine

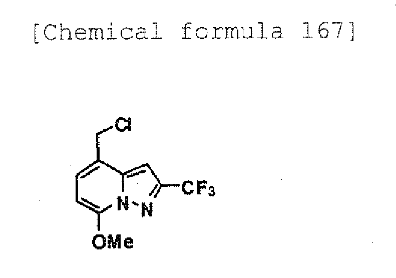

Thionyl chloride (1.42 mL) was added dropwise to a solution of the compound of Example 24 (2.40 g) in dichloromethane (80.0 mL) at 0°C, and the mixture was stirred at 0°C for 1 hour. The reaction mixture was added to a saturated aqueous sodium hydrogen carbonate solution. The organic layer was separated, washed with saturated brine, and dried over anhydrous sodium sulfate, and the solvent was evaporated under reduced pressure. The obtained residue was purified by silica gel column chromatography (hexane : ethyl acetate = 1:1) to obtain the target product (2.12 g) as a white solid. ¹H NMR (CDCl₃, 400 MHz) d: 4.19 (3H, s), 4.77 (2H, s), 6.22 (1H, d, J = 7.9 Hz), 6.96 (1H, s), 7.28 (1H, d, J = 7.9 Hz).
EIMS (+): 264 [M]⁺.

### <Example 95>

4-(2-(3,5-Dichloropyridin-4-yl )ethyl)-7-methoxy-2-(trifluoromethyl)pyrazolo[1,5-a]pyridine

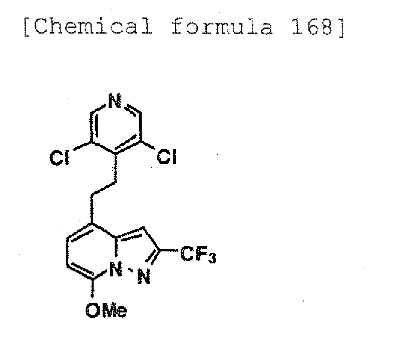

A 1.0 mol/L sodium bis(trimethylsilyl)amide-THF solution (0.907 mL) was added dropwise to a solution of 3,5-dichloro-4-picoline (122 mg) in THF (7.5 mL) at -78°C, and the mixture was stirred at 0°C for 10 minutes. A solution of the compound of Example 94 (100 mg) in THF (4.0 mL) was added dropwise to the mixture at -78°C, and the resultant mixture was stirred at room temperature for 1.5 hours. A saturated aqueous ammonium chloride solution was added to the reaction mixture, and the resultant mixture was extracted three times with ethyl acetate. The extract layer was washed with water and then saturated brine and dried over anhydrous sodium sulfate. The solvent was evaporated under reduced pressure, and the residue was purified by silica gel column chromatography (NH type, product of Fuji Silysia Chemical Ltd., ethyl acetate : hexane= 1:1). The purified product was washed with diisopropyl ether to obtain the target product (124 mg) as a white solid.
Melting point: 158-160°C (d).
IR (KBr): 1636, 1545, 1506, 1428, 1255, 1230, 1161, 1142, 1095 cm⁻¹. ¹H NMR (CDCl₃, 400 MHz) d: 3.00-3.07 (2H, m), 3.25-3.32 (2H, m), 4.17 (3H, s), 6.18 (H, d, J = 7.9 Hz), 6.90 (1H, s), 7.01 (1H, d, J = 7.9 Hz), 8.47 (2H, s).
EIMS (+): 389 [M]⁺.
Elemental Analysis (%): Found C 48.98%, H 3.04%, N 10.68%, Calcd. for C₁₆H₁₄Cl₂F₃N₃O C 49.25%, H 3.10%, N 10.77%.

### <Example 96>

3,5-Dichloro-4-(2-(7-methoxy-2-(trifluoromethyl)pyrazolo[1,5-a]pyridin-4-yl )ethyl)pyridine 1-oxide

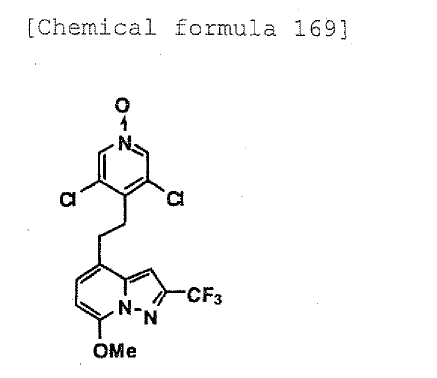

70% m-Chloroperbenzoic acid (394 mg) was added to a solution of the compound of Example 95 (312 mg) in chloroform (1.6 mL) at 0°C, and the mixture was stirred at room temperature for 10 hours. A saturated aqueous sodium thiosulfate solution was added to the reaction mixture, and the resultant mixture was extracted three times with ethyl acetate. The combined extract layer was washed with a saturated aqueous sodium hydrogen carbonate solution, water, and then saturated brine and dried over anhydrous sodium sulfate. The solvent was evaporated under reduced pressure, and the residue was purified by silica gel column chromatography (ethyl acetate : methanol = 10:1, and then chloroform: methanol = 10:1). The purified product was washed with diisopropyl ether to obtain the target product (40.0 mg) as a white solid.
Melting point: 187-189°C.
IR (KBr): 1635, 1544, 1505, 1427, 1268, 1230, 1166, 1094, 964 cm⁻¹.
¹H NMR (CDCl₃, 400 MHz) d: 3.01-3.07 (2H, m), 3.21-3.28 (2H, m), 4.17 (3H, s), 6.17 (1H, d, J = 7.4 Hz), 6.88 (1H, s), 6.95 (1H, d, J = 7.4 Hz), 8.17 (2H, s).
CIMS (+) : 406 [M+H]⁺.
Elemental Analysis (%): Found C 47.18%, H 2.95%, N 10.28%, Calcd. for C₁₆H₁₂Cl₂F₃N₃O₂ C 47.31%, H 2.98%, N 10.35%.

### <Example 97>

2-(3-Chloropyridin-4-yl )-1-(7-methoxy-2-(trifluoromethyl)pyrazolo[1,5-a]pyridin-4-yl )ethanone

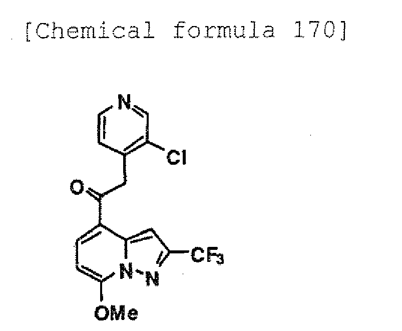

10% Palladium-carbon (50.0 mg) was added to a solution of the compound of Example 88 (500 mg) in acetic acid (25.0 mL) at room temperature, and the mixture was vigorously stirred at room temperature for 10 hours under hydrogen atmosphere. The reaction mixture was filtrated, and the organic solvent of the filtrate was evaporated under reduced pressure. The residue was purified by silica gel column chromatography (NH type, product of Fuji Silysia Chemical Ltd., ethyl acetate : hexane = 3:1). The purified product was washed with diisopropyl ether to obtain the target product (47.0 mg) as a yellow solid.
Melting point: 188-191°C.
IR (KBr): 3436, 1677, 1623, 1556, 1230, 1172, 1137, 964, 803 cm⁻¹. ¹H NMR (CDCl₃, 400 MHz) d: 4.30 (3H, s), 4.46 (2H, s), 6.37 (1H, d, J = 7.9 Hz), 7.24-7.27 (1H, m), 7.67 (1H, s), 8.12 (1H, d, J = 7.9 Hz), 8.48 (1H, d, J = 4.3 Hz), 8.64 (1H, s).
EIMS (+): 369 [M]⁺.
Elemental Analysis (%): Found C 51.25%, H 2.93%, N 11.17%, Calcd. for C₁₆H₁₁ClF₃N₃O₂·0.3H₂O C 51.23%, H 3.12%, N 11.20%.

### <Example 98>

1-(3-Chloro-7-methoxy-2-(trifluoromethyl)pyrazolo[1,5-a]pyridin-4-yl )-2-(3,5-dichloropyridin-4-yl )ethanone

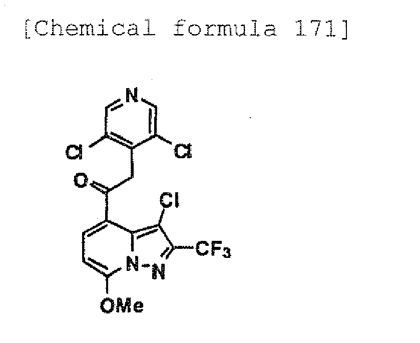

N-Chlorosuccinimide (102 mg) was added to a solution of the compound of Example 88 (205 mg) in DMF (5.00 mL) at room temperature, and the mixture was stirred at 60°C for 5 hours. A saturated aqueous sodium thiosulfate solution was added to the reaction mixture, and the resultant mixture was extracted twice with ethyl acetate. The combined extract layer was washed with water and then saturated brine and dried over anhydrous sodium sulfate. The solvent was evaporated under reduced pressure, and the residue was purified by silica gel column chromatography (ethyl acetate). The purified product was washed with diisopropyl ether to obtain the target product (132 mg) as a bluish white solid.
¹H NMR (CDCl₃, 400 MHz) d: 4.28 (3H, s), 4.65 (2H, s), 6.34 (1H, d, J = 7.9 Hz), 7.83 (1H, d, J = 7.9 Hz), 8.55 (2H, s).
EIMS (+): 437[M]⁺.
Elemental Analysis (%): Found C 43.52%, H 2.15%, N 9.54%, Calcd. for C₁₆H₉Cl₃F₃N₃O₂ C 43.81%, H 2.07%, N 9.58%.

### <Example 99>

2-(3,5-Dichloropyridin-4-yl )-1-(7-(methylamino)-2-(trifluoromethyl)pyrazolo[1,5-a]pyridin-4-yl )ethanone

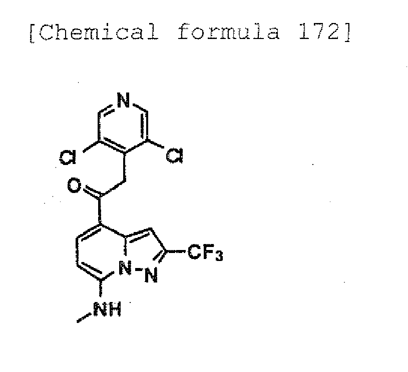

A 2.0 mol/L methylamine-THF solution (5.00 mL) was added to the compound of Example 88 (100 mg) at room temperature, and the mixture was stirred at room temperature for 30 minutes. The solvent and the like are evaporated under reduced pressure, and the residue was washed with diisopropyl ether to obtain the target product (80.0 mg) as a pale yellow solid.
Melting point: 220-223°C.
IR (KBr): 3260, 1664, 1621, 1593, 1546, 1237, 1178, 1123, 1099, 797 cm⁻¹.
¹H NMR (CDCl₃, 400 MHz) d: 3.24 (3H, d, J = 4.9 Hz), 4.67 (2H, s), 6.10 (1H, d, J = 7.9 Hz), 6.77 (1H, brd, J = 4.9 Hz), 7.56 (1H, s), 8.16 (1H, d, J = 7.9 Hz), 8.53 (2H, s).
EIMS (+): 402 [M]⁺.
Elemental Analysis (%): Found C 47.58%, H 2.94%, N 13.37%, Calcd. for C₁₆H₁₁Cl₂F₃N₄O C 47.66%, H 2.94%, N 13.37%.

### <Example 100>

2-(3,5-Dichloropyridin-4-yl )-1-(7-(dimethylamino)-2-(trifluoromethyl)pyrazolo[1,5-a]pyridin-4-yl )ethanone

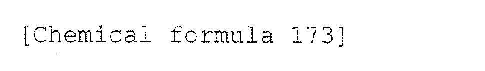

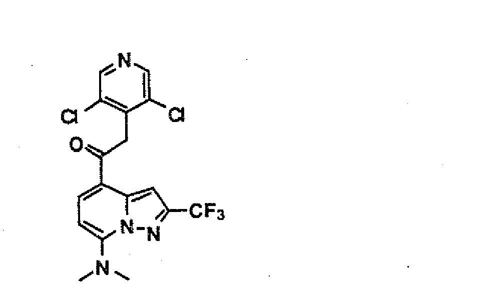

A 2.0 mol/L dimethylamine-THF solution (2.00 mL) was added to the compound of Example 88 (95.0 mg) at room temperature, and the mixture was stirred at room temperature for 35 minutes. The solvent and the like are evaporated under reduced pressure, and the residue was purified by silica gel column chromatography (ethyl acetate : hexane = 1:1). The purified product was washed with diisopropyl ether to obtain the target product (50.0 mg) as an orange solid.
Melting point: 234-238°C.
IR (KBr): 1596, 1566, 1551, 1364, 1233, 1173, 1120, 896 cm⁻¹.
¹H NMR (CDCl_{3,} 400 MHz) d: 3.38 (6H, s), 4.68 (2H, s), 6.25 (1H, d, J = 8.6 Hz), 7.62 (1H, s), 8.08 (1H, d, J = 8.6 Hz), 8.54 (2H, s).
EIMS (+): 416 [M]⁺.
Elemental Analysis (%): Found C 48.84%, H 3.13%, N 13.42%, Calcd. for C₁₇H₁₃Cl₂F₃N₄O C 48.94%, H 3.14%, N 13.43%.

### <Example 101>

4-(Chloromethyl)-2-(difluoromethyl)-7-methoxypyrazolo[1,5-a]py ridine

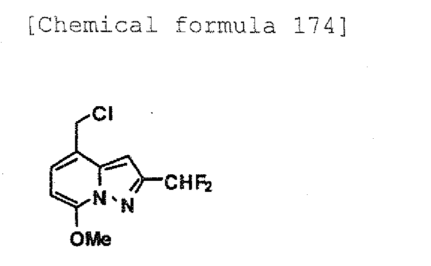

Thionyl chloride (0.191 mL) was added dropwise to a solution of the compound of Example 44 (300 mg) in dichloromethane (13.0 mL) at 0°C, and the mixture was stirred at 0°C for 1.5 hours. The reaction mixture was added to a saturated aqueous sodium hydrogen carbonate solution, and the mixture was extracted twice with ethyl acetate. The combined extract layer was washed with water and then saturated brine and dried over anhydrous sodium sulfate. The solvent was evaporated under reduced pressure to obtain the target product (316 mg) as a white solid.
¹H NMR (CDCl₃, 400 MHz) d: 4.18 (3H, s), 4.77 (2H, s), 6.16 (1H, d, J = 7.9 Hz), 6.91 (1H, s), 6.95 (1H, t, J = 55.0 Hz), 7.24 (1H, d, J = 7.9 Hz).
EIMS (+): 246 [M]⁺

### <Example 102>

4-(2-(3,5-Dichloropyridin-4-yl )ethyl)-2-(difluoromethyl)-7-methoxypyrazolo[1,5-a]pyridine

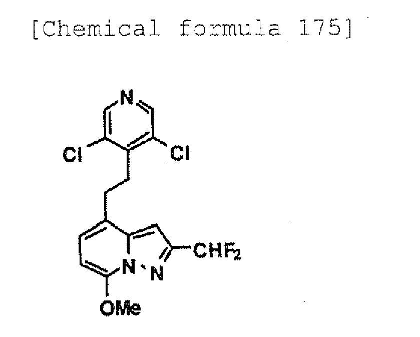

A 1.0 mol/L sodium bis(trimethylsilyl)amide-THF solution (1.51 mL) was added dropwise to a solution of 3, 5-dichloro-4-picoline (204 mg) in THF (2.00 mL) at -78°C, and the mixture was stirred at 0°C for 10 minutes. A solution of the compound of Example 101 (155 mg) in THF (6.00 mL) was added dropwise to the reaction mixture at -78°C, and the mixture was stirred at room temperature for 1 hour. A saturated aqueous ammonium chloride solution was added to the reaction mixture, and the resultant mixture was extracted twice with ethyl acetate. The combined extract layer was washed with water and then saturated brine and dried over anhydrous sodium sulfate. The solvent was evaporated under reduced pressure, and the residue was purified by silica gel column chromatography (NH type, product of Fuji Silysia Chemical Ltd., ethyl acetate: hexane= 1:1). The purified product was washed with diisopropyl ether to obtain the target product (246 mg) as a white solid.
Melting point: 153-156°C.
IR (KBr): 1637, 1574, 1542, 1498, 1453, 1370, 1297, 1156, 1088, 1033, 785 cm⁻¹.
¹H NMR (CDCl₃, 400 MHz) d: 3.01-3.07 (2H, m), 3.27-3.31 (2H, m), 4.16 (3H, s), 6.12 (1H, d, J = 7.9 Hz), 6.87 (1H, s), 6.95 (1H, t, J = 55.0 Hz), 6.97 (1H, d, J = 7.9 Hz), 8.47 (2H, s).
EIMS (+) : 371 [M]⁺.
Elemental Analysis (%): Found C 51.34%, H 3.38%, N 11.19%, Calcd. for C₁₆H₁₃Cl₂F₂N₃O C 51.63%, H 3.52%, N 11.29%.

### <Example 103>

3,5-Dichloro-4-(2-(2-(difluoromethyl)-7-methoxypyrazolo[1,5-a]pyridin-4-yl )ethyl)pyridine 1-oxide

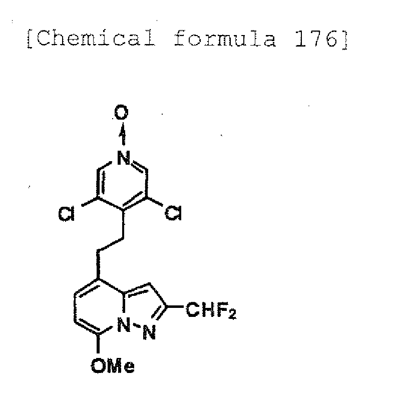

70% m-Chloroperbenzoic acid (45.6 mg) was added to a solution of the compound of Example 102 (27.5 mg) in chloroform (0.300 mL) at 0°C, the mixture was stirred at room temperature for 8.5 hours under protection from light. A saturated aqueous sodium thiosulfate solution was added to the reaction mixture, and the resultant mixture was extracted twice with ethyl acetate. The combined extract layer was washed with a saturated aqueous sodium hydrogen carbonate solution, water, and then saturated brine and dried over anhydrous sodium sulfate. The solvent was evaporated under reduced pressure, and the residue was purified on a silica gel plate (chloroform : methanol = 10:1) to obtain the target product (4.0 mg) as a brown solid.
Melting point: 165-170°C.
IR (KBr): 1637, 1543, 1437, 1430, 1264, 1090, 1037, 818 cm⁻¹.
¹H NMR (CDCl₃, 400 MHz) d: 3.01-3.07 (2H, m), 3.22-3.28 (2H, m), 4.16 (3H, s), 6.11 (1H, d, J = 7.3 Hz), 6.84 (1H, s), 6.91 (1H, d, J = 7.3 Hz), 6.95 (1H, t, J = 55.0 Hz), 8.20 (2H, s).
HRESIMS (+): 388.0439 (Calcd. for C₁₆H₁₄Cl₂F₂N₃O₂ 388.0431).

### <Example 104>

2-(Difluoromethyl)-7-methoxy-4-(2-(pyridin-4-yl )ethyl)pyrazolo[1,5-a]pyridine

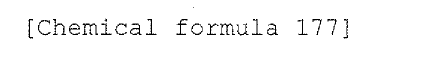

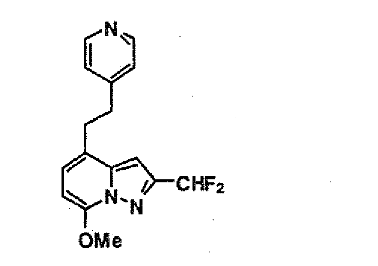

10% Palladium-carbon (8.00 mg) was added to a solution of the compound of Example 102 (80.0 mg) in acetic acid (4.00 mL) at room temperature, and the mixture was vigorously stirred at room temperature for 80 hours under hydrogen atmosphere. Insoluble material in the reaction mixture was removed by filtration, and the solvent of the filtrate was evaporated under reduced pressure. Triethylamine-water was added to the resultant mixture, and the resultant mixture was extracted twice with ethyl acetate. The combined extract layer was dried over anhydrous sodium sulfate, and the solvent was evaporated under reduced pressure. The residue was separated and purified on a silica gel plate (ethyl acetate : methanol = 10:1), and the resultant product was washed with diisopropyl ether to obtain the target product (25.6 mg) as a white solid.
Melting point: 121-123°C.
IR (KBr): 1635, 1601, 1575, 1538, 1376, 1316, 1290, 1182, 1094, 1043, 809 cm⁻¹.
¹H NMR (CDCl₃, 400 MHz) d: 3.00-3.04 (2H, m), 3.07-3.13 (2H, m), 4.14 (3H, s), 6.07 (1H, d, J = 7.3 Hz), 6.78 (1H, s), 6.83 (1H, d, J=7.3Hz), 6.95 (1H, t, J=55.1Hz), 7.06-7.08 (2H, m), 8.47-8.51 (2H, m).
EIMS (+): 303 [M]⁺.
Elemental Analysis (%): Found C 63.08%, H 4.98%, N 13.69%, Calcd. for C₁₆H₁₅F₂N₃O C 63.36%, H 4.98%, N 13.85%.

### <Example 105>

4-(2-(2-(Difluoromethyl)-7-methoxypyrazolo[1,5-a]pyridin-4-yl)ethyl)pyridine 1-oxide

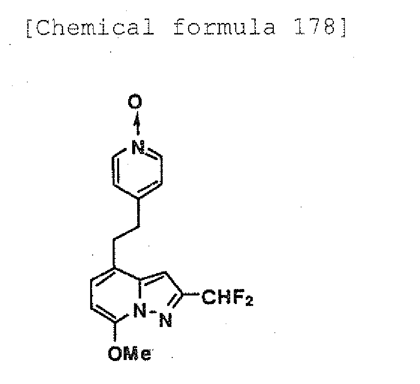

70% m-Chloroperbenzoic acid (81.4 mg) was added to a solution of the compound of Example 104 (40.0 mg) in chloroform (0.300 mL) at 0°C, and the mixture was stirred at room temperature for 1 hour under protection from light. A saturated aqueous sodium thiosulfate solution was added to the reaction mixture, and the resultant mixture was extracted twice with ethyl acetate. The extract layer was washed with a saturated aqueous sodium hydrogen carbonate solution, water, and then saturated brine and dried over anhydrous sodium sulfate. The solvent was evaporated under reduced pressure. The residue was purified on a silica gel plate (chloroform : methanol = 10:1) and further purified by column chromatography (NH type, product of Fuji Silysia Chemical Ltd., chloroform) to obtain the target product (12.0 mg) as a pale yellow solid.
Melting point: 150-152°C.
IR (KBr): 1639, 1577, 1543, 1491, 1452, 1296, 1220, 1184, 1080, 817 cm⁻¹.
¹H NMR (CDCl₃, 400 MHz) d: 3.01-3.12 (4H, m), 4.15 (3H, s), 6.06 (1H, d, J = 7.9 Hz), 6.77 (1H, d, J = 7.9 Hz), 6.79 (1H, s), 6.95 (1H, t, J = 55.0 Hz), 7.00 (2H, d, J = 7.0 Hz), 8.10 (2H, d, J = 7.0 Hz).
HRESIMS (+) : 320.1216 (Calcd. for C₁₆H₁₆F₂N₃O₂ 320.1211).

### <Example 106>

2-(3,5-Dichloropyridin-4-yl )-1-(2-(difluoromethyl)-7-methoxypyrazolo[1,5-a]pyridin-4-yl )ethanol

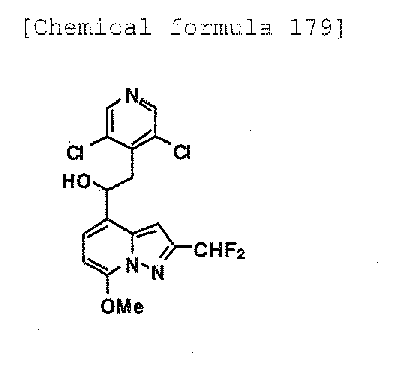

A1.58 mol/Ln-butyllithium-hexane solution (1.77mL) was added dropwise to a solution of diisopropylamine (0.392 mL) in THF (20.0 mL) at 0°C, and the mixture was stirred at 0°C for 30 minutes. A solution of 3,5-dichloro-4-picoline (452 mg) in THF (3.00 mL) was added dropwise to the reaction mixture at -78°C, and the resultant mixture was stirred at 0°C for 3 minutes. Subsequently, a solution of the compound of Example 45 (420 mg) in THF (6.00 mL) was added dropwise to the reaction mixture at -78°C, and the resultant mixture was stirred at -78°C for 40 minutes. A saturated aqueous ammonium chloride solution was added to the reaction mixture, and the resultant mixture was extracted twice with ethyl acetate. The extract layer was washed with water and then saturated brine and dried over anhydrous sodium sulfate. The solvent was evaporated under reduced pressure, and the residue was washed with diisopropyl ether to obtain the target product (668 mg) as a white solid.
¹H NMR (CDCl₃, 400 MHz) d: 3.41 (1H, dd, J = 13.1, 5.2 Hz), 3.61 (1H, dd, J = 13.1, 8.9 Hz), 4.18 (3H, s), 5.35-5.39 (1H, m), 6.16 (1H, d, J = 7.9 Hz), 6.95 (1H, t, J = 55.1 Hz), 7.00 (1H, s), 7.23 (1H, d, J = 7.9 Hz), 8.46 (2H, s).
EIMS (+): 387 [M]⁺.

### <Example 107>

2-(3,5-dichloropyridin-4-yl )-1-(2-(difluoromethyl)-7-methoxypyrazolo[1,5-a]pyrzdirn-4-yl )ethanone

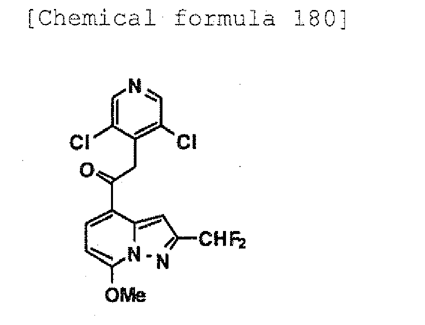

Dess-Martin periodinane (903 mg) was added to a solution of the compound of Example 106 (638 mg) in chloroform (16.0 mL) at 0°C, and the mixture was stirred at 0°C for 1.5 hours. A saturated aqueous sodium hydrogen carbonate solution was added to the reaction mixture, and the resultant mixture was extracted three times with ethyl acetate. The extract layer was washed with water and then saturated brine and dried over anhydrous sodium sulfate. The solvent was evaporated under reduced pressure, and the residue was recrystallized from methanol to obtain the target product (527 mg) as white needle crystals.
Melting point: 208-211°C (d).
IR (KBr): 1667, 1620, 1555, 1325, 1247, 1088, 1075, 1055, 780 cm⁻¹. ¹H NMR (CDCl₃, 400 MHz) d: 4.32 (3H, s), 4.71 (2H, s), 6.35 (1H, d, J = 7.9 Hz), 6.93 (H, t, J = 55.1 Hz), 7.57 (1H, s), 8.16 (1H, d, J = 7.9 Hz), 8.55 (2H, s).
EIMS (+): 385 [M]⁺.
Elemental Analysis (%) : Found C 49.95%, H 2.95%, N 10.86%, Calcd. for C₁₆H₁₁Cl₂F₂N₃O₂ C 49.76%, H 2.87%, N 10.88%.

### <Example 108>

3,5-Dichloro-4-(2-(2-(difluoromethyl)-7-methoxypyrazolo[1,5-a]pyridin-4-yl )-2-oxoethyl)pyridine 1-oxide

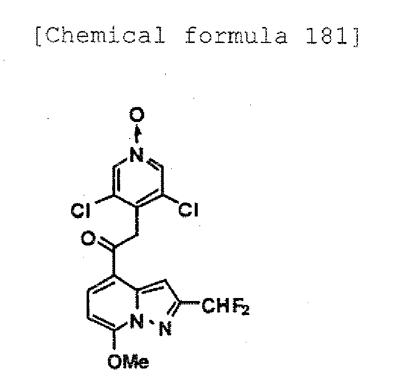

70% m-Chloroperbenzoic acid (76.7 mg) was added to a solution of the compound of Example 107 (100 mg) in chloroform (0.500 mL) at 0°C, and the mixture was stirred at room temperature for 19.5 hours under protection from light. A saturated aqueous sodium thiosulfate solution was added to the reaction mixture, and the resultant mixture was extracted twice with ethyl acetate. The extract layer was washed with a saturated aqueous sodium hydrogen carbonate solution, water, and then saturated brine and dried over anhydrous sodium sulfate. The solvent was evaporated under reduced pressure, and the residue was purified on a silica gel plate (chloroform : methanol = 10:1). The purified produced was washed with diisopropyl ether to obtain the target product (60.3 mg) as a pale yellow solid.
Melting point: 220-226°C (d).
IR (KBr): 1667, 1617, 1555, 1440, 1427, 1324, 1241, 1085, 1057, 784 cm⁻¹.
¹H NMR (CDCl₃, 400 MHz) d: 4.32 (3H, s), 4.63 (2H, s), 6.35 (1H, d, J = 7.9 Hz), 6.93 (1H, t, J = 54.4 Hz), 7.56 (1H, s), 8.14 (1H, d, J = 7.9 Hz), 8.24 (2H, s).
CIMS (+): 402 [M+H]⁺.
Elemental Analysis (%): Found C 46.28%, H 2.83%, N 9.98%, Calcd. for C₁₆H₁₁Cl₂F₂N₃O₃·0.7H₂O C 46.33%, H 3.01%, N 10.13%.

### <Example 109>

1-(2-(Difluoromethyl)-7-methoxypyrazolo[1,5-a]pyridin-4-yl )-2-(pyridin-4-yl )ethanone

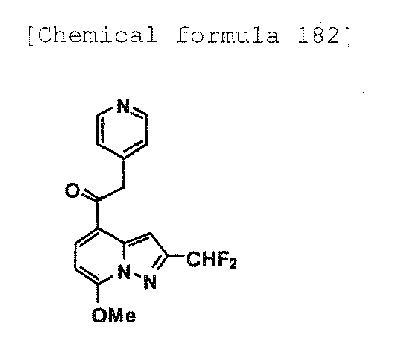

10% Palladium-carbon (33.8 mg) was added to a solution of the compound of Example 107 (338 mg) in acetic acid (18.0 mL) at room temperature, and the mixture was vigorously stirred at room temperature for 44 hours under hydrogen atmosphere. Insoluble material in the reaction mixture was removed by filtration, and the solvent of the filtrate was evaporated under reduced pressure. Triethylamine-water was added to the resultant mixture, and the mixture was extracted twice with ethyl acetate. The extract layer was dried over anhydrous sodium sulfate, and the solvent was evaporated under reduced pressure. The residue was separate and purified by silica gel column chromatography (ethyl acetate : methanol = 10:1) to obtain the target product (101 mg) as a pale yellow solid.
Melting point: 186-191°C (d).
IR (KBr): 1671, 1624, 1561, 1554, 1311, 1082, 1042, 782 cm⁻¹.
¹H NMR (CDCl₃, 400 MHz) d: 4.28 (3H, s), 4.31 (2H, s), 6.27 (1H, d, J = 7.9 Hz), 6.93 (1H, t, J = 54.4 Hz), 7.20-7.24 (2H, m), 7.63 (1H, s), 8.01 (1H, d, J = 7.9 Hz), 8.56-8.60 (2H, m).
EIMS (+): 317 [M]⁺.
Elemental Analysis (%): Found C 60.31%, H 4.10%, N 13.10%, Calcd. for C₁₆H₁₃F₂N₃O₂ C 60.57%, H 4.13%, N 13.24%.

### <Example 110>

4-(2-(2-(Difluoromethyl)-7-methoxypyrazolo[1,5-a]pyridin-4-yl)-2-oxoethyl)pyridine 1-oxide

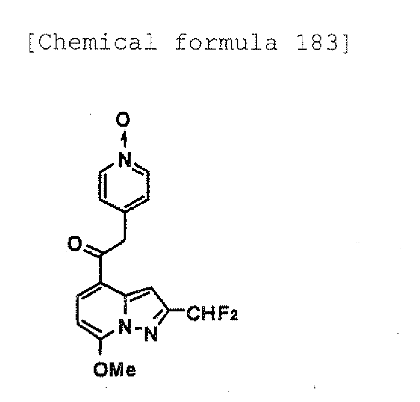

70% m-Chloroperbenzoic acid (60.2 mg) was added to a solution of the compound of Example 109 (31.0 mg) in chloroform (0.200 mL) at 0°C, and the mixture was stirred at room temperature for 40 minutes under protection from light. A saturated aqueous sodium thiosulfate solution was added to the reaction mixture, and the resultant mixture was extracted twice with ethyl acetate. The extract layer was washed with a saturated aqueous sodium hydrogen carbonate solution, water, and then saturated brine and dried over anhydrous sodium sulfate. The solvent was evaporated under reduced pressure, and the residue was purified on a silica gel plate (chloroform : methanol = 10:1). The purified product was washed with diisopropyl ether to obtain the target product (2.00 mg) as a yellow solid.
¹H NMR (CDCl₃, 400 MHz) d: 4.30 (3H, s), 4.34 (2H, s), 6.29 (1H, d, J = 8.6 Hz), 6.94 (1H, t, J = 55.0 Hz), 7.25-7.29 (2H, m), 7.59 (1H, s), 8.02 (1H, d, J = 8.6 Hz), 8.31 (2H, d, J = 6.7 Hz).
ESIMS (+): 334 [M+H]⁺.

### <Example 111>

2-(3-Chloropyridin-4-yl )-1-(2-(difluoromethyl)-7-methoxypyraz olo[1,5-a]pyridin-4-yl )ethanone

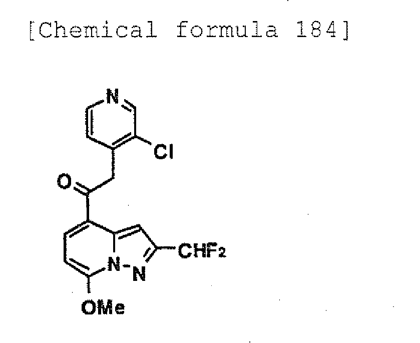

10% Palladium-carbon (33.8 mg) was added to a solution of the compound of Example 107 (338 mg) in acetic acid (18.0 mL) at room temperature, and the mixture was vigorously stirred at room temperature for 44 hours under hydrogen atmosphere. Insoluble material in the reaction mixture was removed by filtration, and the solvent of the filtrate was evaporated under reduced pressure. Triethylamine-water was added to the mixture, and the resultant mixture was extracted twice with ethyl acetate. The extract layer was dried over anhydrous sodium sulfate, and the solvent was evaporated under reduced pressure. The residue was separated and purified by silica gel column chromatography (ethyl acetate : methanol = 10:1) to obtain the target product (94.5 mg) as a yellow solid.
Melting point: 191-1940°C (d).
IR (KBr): 1676, 1620, 1557, 1492, 1452, 1323, 1246, 1151, 1090, 791 cm⁻¹.
¹H NMR (CDCl₃, 400 MHz) d: 4.30 (3H, s), 4.46 (2H, s), 6.31 (1H, d, J = 7.9 Hz), 6.93 (1H, t, J = 54.4 Hz), 7.25 (1H, s), 7.60 (1H, s), 8.09 (1H, d, J = 7.9 Hz), 8.47 (1H, d, J = 4.9 Hz), 8.64 (1H, s).
EIMS (+): 351 [M]⁺.
Elemental Analysis (%): Found C 54.41%, H 3.52%, N 11.86%, Calcd. for C₁₆H₁₂ClF₂N₃O₂ C 54.64%, H 3.44%, N 11.95%.

### <Example 112>

4-(Chloromethyl)-2-cyclopropyl-7-methoxypyrazolo[1,5-a]pyridine

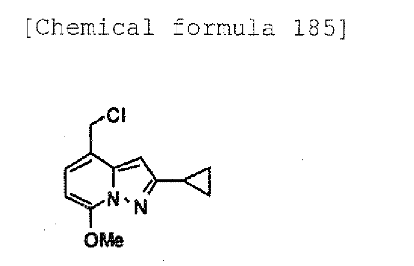

Thionyl chloride (0.0668 mL) was added dropwise to a solution of the compound of Example 19 (100 mg) in dichloromethane (4.50 mL) at 0°C, and the mixture was stirred at 0°C for 2 hours. The reaction mixture was added to a saturated aqueous sodium hydrogen carbonate solution, and the mixture was extracted twice with ethyl acetate. The combined extract layer was washed with water and then saturated brine and dried over anhydrous sodium sulfate, and the solvent was evaporated under reduced pressure to obtain the target product (82.0 mg) as a white solid.
¹H NMR (CDCl₃, 400 MHz) d: 0.88-0.93 (2H, m), 1.02-1.08 (2H, m), 2.18-2.27 (1H, m), 4.13 (3H, s), 4.71 (2H, s), 5.96 (1H, d, J = 7.3 Hz), 6.24 (1H, s), 7.09 (1H, d, J = 7.3 Hz).
EIMS (+) : 236 [M]⁺

### <Example 113>

2-Cyclopropyl-4-(2-(3,5-dichloropyridin-4-yl )ethyl)-7-methoxy pyrazolo[1,5-a]pyridine

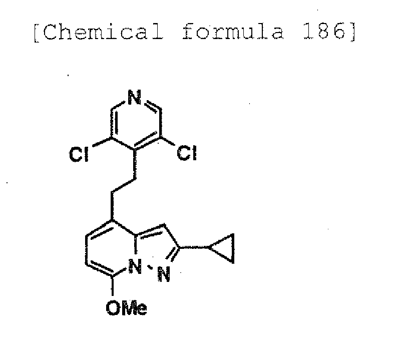

A 1.0 mol/L sodium bis(trimethylsilyl)amide-THF solution (1.01 mL) was added dropwise to a solution of 3, 5-dichloro-4-picoline (137 mg) in THF (8.50 mL) at -78°C, and the mixture was stirred at 0°C for 10 minutes. A solution of the compound of Example 112 (100 mg) in THF (4.00 mL) was added dropwise to the reaction mixture at -78°C, and the resultant mixture was stirred at room temperature for 1 hour. A saturated aqueous ammonium chloride solution was added to the reaction mixture, and the resultant mixture was extracted twice with ethyl acetate. The combined extract layer was washed with water and then saturated brine and dried over anhydrous sodium sulfate. The solvent was evaporated under reduced pressure, and the residue was purified by silica gel column chromatography (ethyl acetate: hexane = 2:1). The purified product was washed with diisopropyl ether to obtain the target product (126 mg) as a white solid.
¹H NMR (CDCl₃, 400 MHz) d: 0.84-0.90 (2H, m), 1.00-1.06 (2H, m), 2.17-2.26 (1H, m), 2.90-2.98 (2H, m), 3.22-3.29 (2H, m), 4.11 (3H, s), 5.92 (1H, d, J = 7.9 Hz), 6.20 (1H, s), 6.83 (1H, d, J = 7.9 Hz), 8.45 (2H, s).
ESIMS (+): 362 [M+H]⁺.
Elemental Analysis (%): Found C 59.51%, H 4.65%, N 11.55%, Calcd. for C₁₈H₁₇Cl₂N₃O C 59.68%, H 4.73%, N 11.60%.

### <Example 114>

4-(2-(2-Cyclopropyl-7-methoxypyrazolo[1,5-a]pyridin-4-yl )ethyl)-3,5-dichloropyridine 1-oxide

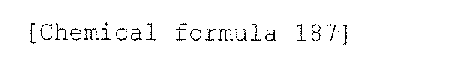

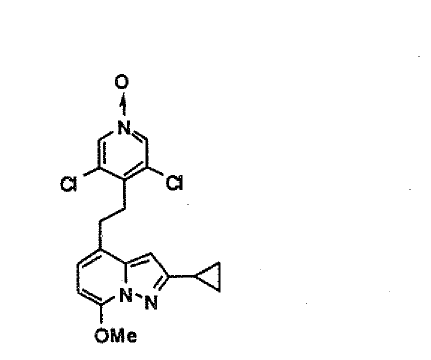

A 1.0 mol/L sodium bis(trimethylsilyl)amide-THF solution (1.01 mL) was added dropwise to a solution of the compound of Example 112 (154 mg) and 3,5-dichloro-4-picoline-N-oxide (347 mg) in THF (6.50 mL) at 0°C, and the mixture was stirred at 0°C for 1.5 hours. A saturated aqueous ammonium chloride solution was added to the reaction mixture, and the resultant mixture was extracted twice with ethyl acetate. The combined extract layer was washed with saturated brine and dried over anhydrous sodium sulfate. The solvent was evaporated under reduced pressure, and the residue was purified by silica gel column chromatography (ethyl acetate : methanol = 10:1). The purified product was washed with diisopropyl ether to obtain the target product (32.0 mg) as a brown solid.
¹H NMR (CDCl₃, 400 MHz) d: 0.86-0.90 (2H, m), 1.02-1.08 (2H, m), 2.18-2.26 (1H, m), 2.92-2.98 (2H, m), 3.18-3.24 (2H, m), 4.11 (3H, s), 5.91 (1H, d, J = 7.9 Hz), 6.17 (1H, s), 6.76 (1H, d, J = 7.9 Hz), 8.16 (2H, s).
ESIMS (+): 378[M+H]⁺.
Elemental Analysis (%): Found C 57.52%, H 4.64%, N 10.82%, Calcd. for C₁₈H₁₇Cl₂N₃O₂ C 57.16%, H 4.53%, N 11.11%.

### <Example 115>

1-(2-Cyclopropyl-7-methoxypyrazolo[1,5-a]pyridin-4-yl )-2-(3,5-dichloropyridin-4-yl )ethanol

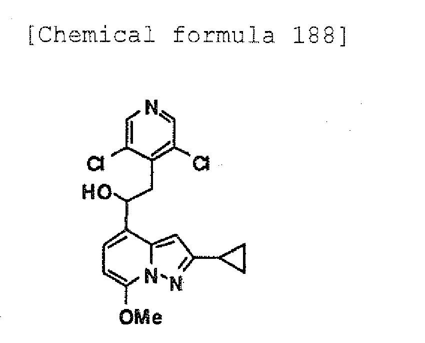

A 1.58 mol/Ln-butyllithium-hexane solution (1.76mL) was added dropwise to a solution of diisopropylamine (0.391 mL) in THF(20.0 mL) at 0°C, and the mixture was stirred at 0°C for 30 minutes. A solution of 3,5-dichloro-4-picoline (450 mg) in THF (2.00 mL) was added dropwise to the reaction mixture at -78°C, and the mixture was stirred at 0°C for 3 minutes. Subsequently, a solution of the compound of Example 28 (400 mg) in THF (4.00 mL) was added dropwise to the resultant mixture at -78°C, and the mixture was stirred at -78°C for 1 hour. A saturated aqueous ammonium chloride solution was added to the reaction mixture, and the resultant mixture was extracted twice with ethyl acetate. The extract layer was washed with water and then saturated brine and dried over anhydrous sodium sulfate. The solvent was evaporated under reduced pressure, and the residue was washed with diisopropyl ether to obtain the target product (627 mg) as a white solid.
¹H NMR (CDCl₃, 400 MHz) d: 0.84-0.89 (2H, m), 1.01-1.07 (2H, m), 2.09 (1H, brs), 2.16-2.25 (1H, m), 3.38 (1H, dd, J = 13.1, 5.2 Hz), 3.59 (1H, dd, J = 13.1, 8.9 Hz), 4.13 (3H, s), 5.25-5.30 (1H, m), 5.97 (1H, d, J = 7.3 Hz), 6.31 (1H, s), 7.09 (1H, d, J = 7.3 Hz), 8.45 (2H, s).
EIMS (+): 377 [M]⁺.

### <Example 116>

2-Cyclopropyl-7-methoxy-4-(2-(pyridin-4-yl )ethyl)pyrazolo[1,5 -a]pyridine

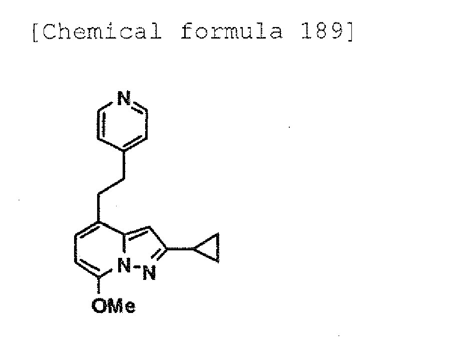

Methanesulfonic acid (5.15 mL) was added to a solution of the compound of Example 115 (2.00 g) in THF (75.0 mL) at 0°C, and the mixture was stirred at 60°C for 4.5 hours. An aqueous solution of sodium hydroxide was added to the reaction mixture, and the resultant mixture was extracted twice with ethyl acetate. The combined extract layer was washed with water and then saturated brine and dried over anhydrous sodium sulfate, and the solvent was evaporated under reduced pressure. The residue was purified by silica gel column chromatography (ethyl acetate : hexane = 2:1) to obtain crude 2-cyclopropyl-4-(2-(3,5-dichloropyridin-4-yl )vinyl)-7-methoxy pyrazolo[1,5-a]pyridine (1.62 g) as an orange solid.
10% Palladium-carbon (80.0 mg) was added to a solution of the crude 2-cyclopropyl-4-(2-(3,5-dichloropyridin-4-yl )vinyl)-7-methoxy pyrazolo[1,5-a]pyridine (800 mg) in acetic acid (45.0 mL) at room temperature, and the mixture was vigorously stirred at room temperature for 12.5 hours under hydrogen atmosphere. Insoluble material in the reaction mixture was removed by filtration, and the solvent of the filtrate was evaporated under reduced pressure. The residue was purified by silica gel column chromatography (ethyl acetate: methanol : triethylamine = 10:1:0.1), and the purified product was washed with diisopropyl ether to obtain the target product (353 mg) as a brown solid.
¹H NMR (CDCl₃, 400 MHz) d: 0.85-0.90 (2H, m), 1.02-1.07 (2H, m), 2.18-2.26 (1H, m), 2.97-3.01 (4H, m), 4.09 (3H, s), 5.87 (1H, d, J = 7.9 Hz), 6.09 (1H, s), 6.69 (1H, d, J = 7.9 Hz), 7.04-7.07 (2H, m), 8.46-8.49 (2H, m).
EIMS (+): 293 [M]⁺.
Elemental Analysis (%): Found C 73.52%, H 6.59%, N 14.22%, Calcd. for C₁₈H₁₉N₃O Calcd. for C 73.69%, H 6.53%, N 14.32%.

### <Example 117>

4-(2-(2-Cyclopropyl-7-methoxypyrazolo[1,5-a]pyridin-4-yl )ethyl)pyridine 1-oxide

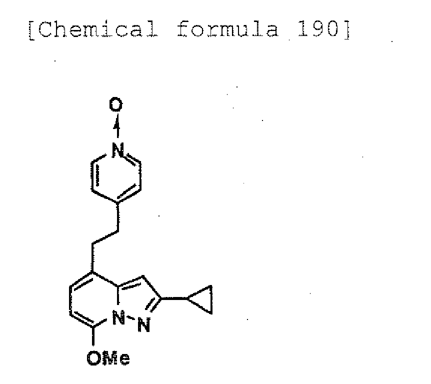

A 1.0 mol/L sodium bis(trimethylsilyl)amide-THF solution (1.27 mL) was added dropwise to a solution of the compound of Example 112 (150 mg) and 4-picoline-N-oxide (207 mg) in THF (6.50 mL) at 0°C, and the mixture was stirred at 0°C for 1 hour. A saturated aqueous ammonium chloride solution was added to the reaction mixture. Sodium chloride was added to the mixture to form a salt precipitate, and the product was extracted twice with ethyl acetate. The solvent of the combined extract layer was evaporated under reduced pressure, and the residue was purified on a silica gel plate (chloroform : methanol = 10:1) to obtain the target product (16.0 mg) as a brown amorphous.
¹H NMR (CDCl₃, 400 MHz) d: 0.86-0.90 (2H, m), 1.02-1.08 (2H, m), 2.18-2.26 (1H, m), 2.99 (4H, s), 4.09 (3H, s), 5.86 (1H, d, J = 7.9 Hz), 6.10 (1H, s), 6.61 (1H, d, J = 7.9 Hz), 6.95-6.98 (2H, m), 8.06-8.11 (2H, m).
HRESIMS (+): 310.1557 (Calcd. for C₁₈H₂₀N₃O₂ 310.1556).

### <Example 118>

1-(2-Cyclopropyl-7-methoxypyrazolo[1,5-a]pyridin-4-yl )-2-(3,5-dichlcropyridin-4-yl )ethanone

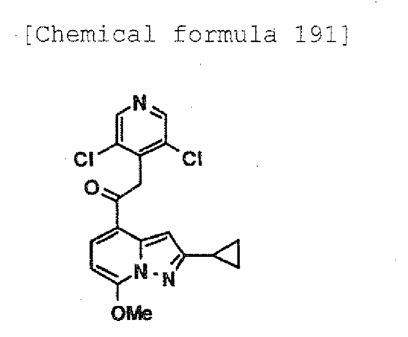

Triethylamine (1.11 mL) and a sulfur trioxide-pyridine complex (632 mg) were added to a solution of the compound of Example 115 (300 mg) in DMSO (8.00 mL) at room temperature, and the mixture was stirred at room temperature for 1.5 hours. Water was added to the reaction mixture, and the resultant mixture was extracted four times with ethyl acetate. The combined extract layer was washed with saturated brine and dried over anhydrous sodium sulfate. The solvent was evaporated under reduced pressure, and the residue was purified by silica gel column chromatography (ethyl acetate). The purified product was washed with diisopropyl ether to obtain the target product (99.0 mg) as a white solid.
Melting point: 220-224°C (d).
¹H NMR (CDCl₃, 400 MHz) d: 0.85-0.91 (2H, m), 1.02-1.08 (2H, m), 2.18-2.26 (1H, m), 4.27 (3H, s), 4.68 (2H, s), 6.15 (1H, d, J = 7.9 Hz), 6.90 (1H, s), 8.04 (1H, d, J = 7.9 Hz), 8.53 (2H, s). CIMS (+): 376 [M+H]⁺.
Elemental Analysis (%) : Found C 56.87%, H 3.88%, N 11.07%, Calcd. for C₁₈H₁₅Cl₂N₃O₂·0.2H₂O C 56.92%, H 4.09%, N 11.06%.

### <Example 119>

3,5-Dichloro-4-(2-(2-cyclopropyl-7-methoxypyrazolo[1,5-a]pyridin-4-yl )-2-oxoethyl)pyridine 1-oxide

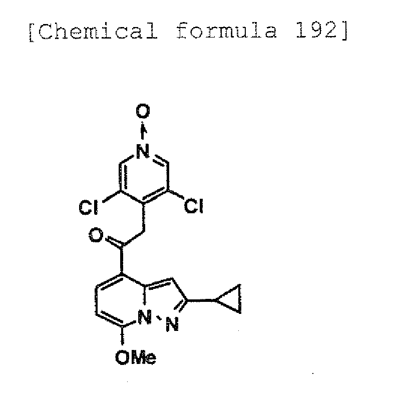

A 30% aqueous hydrogen peroxide solution (0.600 mL) was added to a solution of the compound of Example 118 (200 mg) and tungstic acid (26.5 mg) in N-methyl-2-pyrrolidinone (2.10 mL) at room temperature, and the mixture was stirred at 80°C for 9.5 hours. Subsequently, tungstic acid (26.5 mg) was added to the mixture at 80°C, and the resultant mixture was stirred for 14. 5 hours. Manganese dioxide (2 mg) was added to the reaction mixture at room temperature, and the mixture was stirred at room temperature for 1 hour. Subsequently, the reaction mixture was purified by silica gel column chromatography (ethyl acetate : methanol = 10:1) and further purified on a silica gel plate (chloroform : methanol = 10:1). The obtained compound was washed with diisopropyl ether to obtain the target product (19.0 mg) as a yellow solid.
¹H NMR (CDCl₃, 400 MHz) d: 0.87-0.92 (2H, m), 1.03-1.10 (2H, m), 2.18-2.27 (1H, m), 4.27 (3H, s), 4.61 (2H, s), 6.15 (1H, d, J = 7.9 Hz), 6.89 (1H, s), 8.02 (1H, d, J = 7.9 Hz), 8.23 (2H, s). CIMS (+): 392 [M+H]⁺.
Elemental Analysis (%): Found C 53.37%, H 3.94%, N 10.35%, Calcd. for C₁₈H₁₅Cl₂N₃O₃·0.7H₂O C 53.40%, H 4.08%, N 10.35%.

### <Example 120>

2-(3-Chloropyridin-4-yl )-1-(2-cyclopropyl-7-methoxypyrazolo[1,5-a]pyridin-4-yl )ethanone

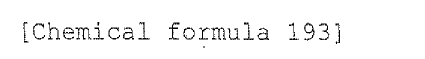

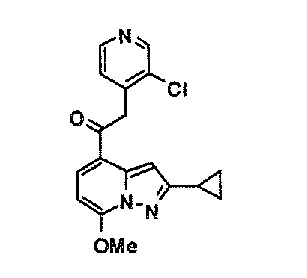

10% Palladium-carbon (34.0 mg) was added to a solution of the compound of Example 118 (170 mg) in acetic acid (9.00 mL) at room temperature, and the mixture was vigorously stirred at room temperature for 30 hours under hydrogen atmosphere. Insoluble material in the reaction mixture was removed by filtration, and the solvent of the filtrate was evaporated under reduced pressure. An aqueous potassium carbonate solution was added to the product, and the resultant mixture was extracted three times with ethyl acetate. The extract layer was dried over anhydrous sodium sulfate, and the solvent was evaporated under reduced pressure. The residue was separated and purified on a silica gel plate (chloroform : methanol = 10:1) to obtain the target product (21.7 mg) as a yellow solid.
¹H NMR (CDCl₃, 400 MHz) d: 0.87-0.92 (2H, m), 1.02-1.09 (2H, m), 2.18-2.27 (1H, m), 4.25 (3H, s), 4.43 (2H, s), 6.12 (1H, d, J = 7.9 Hz), 6.93 (1H, s), 7.24 (1H, d, J = 4.9 Hz), 7.97 (1H, d, J = 7.9 Hz), 8.46 (1H, d, J = 4.9 Hz), 8.62 (1H, s).
EIMS (+): 341 [M]⁺.
Elemental Analysis (%): Found C 62.22%, H 4.66%, N 11.94%, Calcd. for C₁₈H₁₆ClN₃O₂·0.3H₂O C 62.27%, H 4.82%, N 12.10%.

### <Example 121>

1-(2-Cyclopropyl-7-methoxypyrazolo[1,5-a]pyridin-4-yl )-2-(pyr idin-4-yl )ethanone

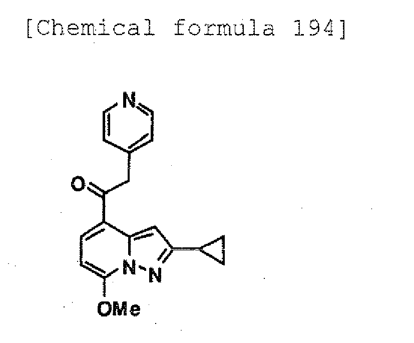

10% Palladium-carbon (34.0 mg) was added to a solution of the compound of Example 118 (170 mg) in acetic acid (9.00 mL) at room temperature, and the mixture was vigorously stirred at room temperature for 30 hours under hydrogen atmosphere. Insoluble material in the reaction mixture was removed by filtration, and the solvent of the filtrate was evaporated under reduced pressure. An aqueous potassium carbonate solution was added to the product, and the resultant mixture was extracted three times with ethyl acetate. The extract layer was dried over anhydrous sodium sulfate, and the solvent was evaporated under reduced pressure. The residue was separated and purified on a silica gel plate (chloroform : methanol = 10:1) to obtain the target product (61.6 mg) as a yellow solid.
¹H NMR (CDCl₃, 400 MHz) d: 0.87-0.92 (2H, m), 1.04-1.10 (2H, m), 2.18-2.27 (1H, m), 4.23 (3H, s), 4.27 (2H, s), 6.08 (1H, d, J = 7.9 Hz), 6.96 (1H, s), 7.21 (2H, d, J = 5.5 Hz), 7.90 (1H, d, J = 7.9 Hz), 8.57 (2H, d, J = 5.5 Hz).
EIMS (+) : 307 [M]⁺.
Elemental Analysis (%) ; Found C 70.18%, H 5.61%, N 13.54%, Calcd. for C₁₈H₁₇N₃O₂ C 70.34%, H 5.58%, N 13.67%.

### <Example 122>

1-(3-Chloro-2-cyclopropyl-7-methoxypyrazolo[1,5-a]pyridin-4-yl)-2-(3,5-dichloropyridin-4-yl )ethanone

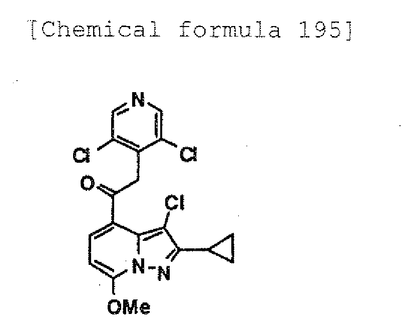

A solution of dimethyl sulfoxide (0.0563mL) in dichloromethane (0.15mL) was added dropwise to a solution of oxalyl chloride (0.0502 mL) in dichloromethane (3.00 mL) at -78°C, and the mixture was stirred at -78°C for 10 minutes. A solution of the compound of Example 115 (100 mg) in dichloromethane (2.50 mL) was added to the reaction mixture at -78°C, and the resultant mixture was stirred at -78°C for 1 hour. Triethylamine (0.269 mL) was added dropwise to the reaction mixture at -78°C, and the resultant mixture was stirred at 0°C for 1.5 hours. A saturated aqueous ammonium chloride solution was added to the reaction mixture, and the resultant mixture was extracted twice with ethyl acetate. The combined extract layer was washed with water and then saturated brine and dried over anhydrous sodium sulfate. The solvent was evaporated under reduced pressure, and the residue was purified on a silica gel plate (ethyl acetate). The purified product was washed with diisopropyl ether to obtain the target product (27.0 mg) as a bluish white solid.
¹H NMR (CDCl₃, 400 MHz) d: 1.04-1.12 (2H, m), 1.13-1.18 (2H, m), 2.16-2.24 (1H, m), 4.20 (3H, s), 4.64 (2H, s), 6.09 (1H, d, J = 7.9 Hz), 7.70 (1H, d, J = 7.9 Hz), 8.53 (2H, s).
EIMS (+): 409 [M]⁺.
Elemental Analysis (%) : Found C 50.91%, H 3.35%, N 9.85%, Calcd. for C₁₈H₁₄Cl₃N₃O₂·0.75H₂O C 50.97%, H 3.68%, N 9.91%.

### <Example 123>

1-(2-Cyclopropyl-7-(methylamino)pyrazolo[1,5-a]pyridin-4-yl )-2-(3,5-dichloropyridin-4-yl )ethanone

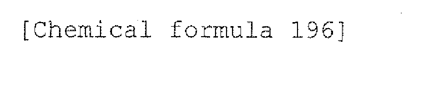

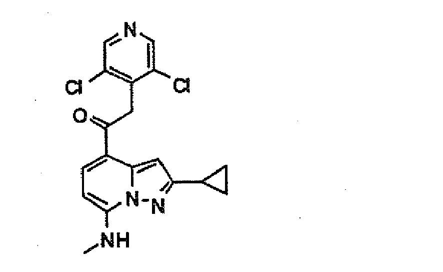

A 2.0 mol/L methylamine-THF solution (5.00 mL) was added to the compound of Example 118 (150 mg) at room temperature, and the mixture was stirred at room temperature for 3.5 hours. The solvent and the like were evaporated under reduced pressure, and the residue was washed with diisopropyl ether to obtain the target product (136 mg) as a pale yellow solid.
Melting point: 196-200°C.
IR (KBr): 3414, 1654, 1601, 1588, 1540, 1380, 1251, 1139, 1093, 791 cm⁻¹.
¹H NMR (CDCl₃, 400 MHz) d: 0.85-0.91 (2H, m), 1.00-1.05 (2H, m), 2.05-2.13 (1H, m), 3.19 (3H, d, J = 4.9 Hz), 4.65 (2H, s), 5.89 (1H, d, J = 8.6 Hz), 6.68 (1H, brd, J = 4.9 Hz), 6.89 (1H, s), 8.02 (1H, d, J = 8.6 Hz), 8.51 (2H, s).
EIMS (+): 374 [M]⁺.
Elemental Analysis (%): Found C 57.37%, H 4.41%, N 14.61%, Calcd. for C₁₈H₁₆Cl₂N₄O C 57.61%, H 4.30%, N 14.93%.

### <Example 124>

1-(2-Cyclopropyl-7-(dimethylamino)pyrazolo[1,5-a]pyridin-4-yl )-2-(3,5-dichloropyridin-4-yl )ethanone

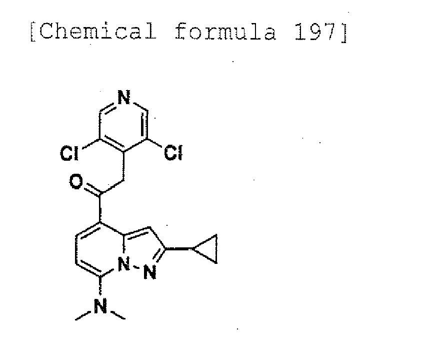

A 2.0 mol/L dimethylamine-THF solution (5.00 mL) was added to the compound of Example 118 (150 mg) at room temperature, and the mixture was stirred at room temperature for 9 hours and allowed to stand for 24 hours. The solvent and the like were evaporated under reduced pressure, and the residue was purified by silica gel column chromatography (ethyl acetate : hexane = 3 : 1). The purified product was washed with diisopropyl ether to obtain the target product (56.0 mg) as a yellow solid.
Melting point: 216-220°C.
IR (KBr) : 1654, 1649, 1595, 1555, 1437, 1294, 1203, 1104, 908 cm⁻¹. ¹H NMR (CDCl₃, 400 MHz) d: 0.86-0.92 (2H, m), 1.00-1.05 (2H, m), 2.11-2.19 (1H, m), 3.32 (6H, s), 4.66 (2H, s), 6.06 (1H, d, J = 7.9 Hz), 6.94 (1H, s), 7.95 (1H, d, J = 7.9 Hz), 8.52 (2H, s).
EIMS (+): 388 [M]⁺.
Elemental Analysis (%): Found C 58.43%, H 4.68%, N 14.41%, Calcd. for C₁₉H₁₈Cl₂N₄O C 58.62%, H 4.66%, N 14.39%.

### <Example 125>

4-(Acetoxymethyl)-2-(1,3-dioxolan-2-yl )-7-methoxypyrazolo[1,5-a]pyridine-3-carboxylic acid ethyl ester

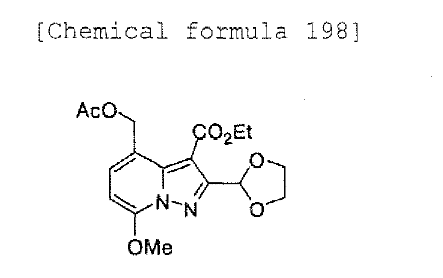

The compound of Example 42 (5.27 g) was dissolved in dichloromethane (150 mL), and 1,2-bis(trimethylsiloxy)ethane (8.04 mL) and trimethylsilyl trifluoromethanesulfonate (0.148 mL) were added to the prepared solution at -78°C. The mixture was stirred at -78°C for 30 minutes and at room temperature for 1.5 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (hexane : ethyl acetate = 1:3 -> ethyl acetate) to obtain the target product (5.46 g) as a colorless powder.
¹H NMR (CDCl₃, 400 MHz): δ 1.41 (3H, t, J = 7.0 Hz), 2.04 (3H, s), 4.07-4.10 (2H, m), 4.14 (3H, s), 4.23-4.26 (2H, m), 4.38 (2H, q, J = 7.1 Hz), 5.54 (2H, s), 6.24 (1H, d, J = 7.9 Hz), 6.60 (1H, s), 7.40 (1H, d, J = 7.9 Hz).
EIMS (+): 364 [M]⁺.

### <Example 126>

2-(1,2-Dioxolan-2-yl )-4-(hydroxymethyl)-7-methoxypyrazolo[1,5-a]pyridine-3-carboxylic acid

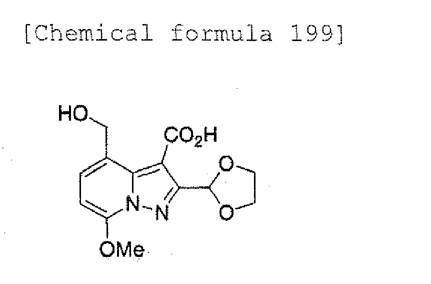

The compound of Example 125 (5.05 g) was dissolved in a mixed solvent of ethanol (70 mL) and water (70 mL), and potassium hydroxide (3.89 g) was added to the prepared solution. The mixture was stirred for 2 hours under heating and reflux. Ethanol was evaporated under reduced pressure, and 1.0 mol/L hydrochloric acid was added to the resultant mixture to adjust the pH of the mixture to 2. The resultant mixture was extracted three times with ethyl acetate. The combined extract layer was washed with saturated brine and dried over anhydrous sodium sulfate, and the solvent was evaporated under reduced pressure to obtain the target product (3.34 g) as a green powder.
¹H NMR (DMSO-d₆, 400 MHz) : δ 3.94-3.97 (2H, m), 4.06-4.07 (2H, m), 4.09 (3H, s), 4.79 (2H, s), 6.49 (1H, s), 6.59 (1H, d, J = 7.9 Hz), 7.51 (1H, d, J = 7.9 Hz).
ESIMS (+): 295 [M+H]⁺.

### <Example 127>

2-(1,3-Dioxolan-2-yl )-4-(hydroxymethyl)-7-methoxypyrazolo[1,5-a]pyridine

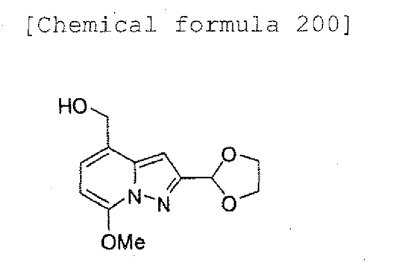

The compound of Example 126 (3.34 g) was dissolved in o-dichlorobenzene (100 mL), and the mixture was stirred at 150°C for 3 hours. o-Dichlorobenzene was evaporated under reduced pressure, and the residue was purified by silica gel column chromatography (ethyl acetate) to obtain the target product (1.83 g) as a colorless powder.
¹H NMR (CDCl₃, 400 MHz) : δ 4.08-4.10 (2H, m), 4.14 (3H, s), 4.18-4.22 (2H, m), 4.83 (2H, d, J = 4.3 Hz), 6.08 (1H, d, J = 7.9 Hz), 6.18 (1H, s), 6.72 (1H, s), 7.14 (1H, d, J = 7.9 Hz).
EIMS (+): 250 [M]⁺.

### <Example 128>

4-(2-(3,5-Dichloropyridin-4-yl ) ethyl)-2-(1,3-dioxolan-2-yl)-7-methoxypyrazolo[1,5-a]pyridine

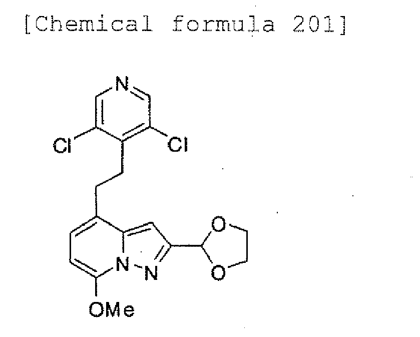

The compound of Example 127 (1.00 g) was dissolved in dichloromethane (40 mL) under argon atmosphere. Triethylamine (1.67 mL) was added to the prepared solution, and thionyl chloride (0.583 mL) was added to the mixture at 0°C. The resultant mixture was stirred at 0°C for 25 minutes. A saturated aqueous sodium hydrogen carbonate solution was added to the reaction mixture, and the resultant mixture was extracted three times with ethyl acetate. The combined extract layer was washed with saturated brine and dried over anhydrous sodium sulfate. The solvent was evaporated under reduced pressure to give a crude chloromethyl compound. 3,5-Dichloro-4-picoline (972 mg) was dissolved in THF (40 mL) under argon atmosphere. A solution of sodium hexamethyldisilazide in THF (1.0 mol/L, 6.60 mL) was added to the prepared solution at -78°C, and the resultant mixture was heated from -78°C to 0°C. Subsequently, a solution of the crude chloromethyl compound in THF (30 mL) was added to the mixture at -78°C, and the resultant mixture was stirred at -78°C for 1 hour and further stirred at room temperature for 1 hour. A saturated aqueous ammonium chloride solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (hexane : ethyl acetate = 1:3) to obtain the target product (361 mg) as a yellow oil
¹H NMR (CDCl₃, 400 MHz) : δ 3.00 (2H, dd, J = 9.2, 6.7 Hz), 3.28 (2H, dd, J = 9.2, 6.7 Hz), 4.08-4.10 (2H, m), 4.13 (3H, s), 4.20-4.22 (2H, m), 6.03 (1H, d, J = 7.3 Hz), 6.19 (1H, s), 6.73 (1H, s), 6.90 (1H, d, J = 7.3 Hz), 8.46 (2H, s).
EIMS (+): 393 [M]⁺.

### <Example 129>

4-(2-(3,5-Dichloropyridin-4-yl )ethyl)-7-methoxypyrazolo[1,5-a]pyridine-2-carbaldehyde

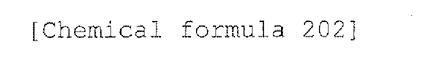

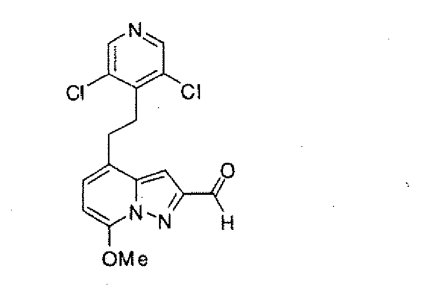

The compound of Example 128 (376 mg) was dissolved in acetone (5.0 mL) and water (5.0 mL). p-Toluenesulfonic acid monohydrate (18.1 mg) was added to the prepared solution, and the mixture was stirred at 60°C for 2.5 hours. A saturated aqueous sodium hydrogen carbonate solution was added to the reaction mixture. Subsequently, acetone was evaporated under reduced pressure, and insoluble material was removed by filtration to obtain the target product (322 mg) as a yellow powder.
¹H NMR (CDCl₃, 400 MHz): δ 3.06 (2H, dd, J = 8.6, 7.3 Hz), 3.29 (2H, dd, J = 10.4, 8.6 H), 4.20 (3H, s), 6.19 (1H, d, J = 7.9 Hz), 6.97 (1H, d, J = 7.9 Hz), 7.18 (1H, s), 8.47 (2H, s), 10.31 (1H, s). EIMS (+): 349 [M]⁺.

### <Example 130>

4-(2-(3,5-Dichloropyridin-4-yl )ethyl)-7-methoxypyrazolo[1,5-a]pyridine-2-carbonitrile

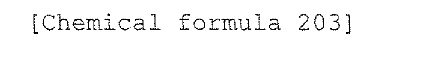

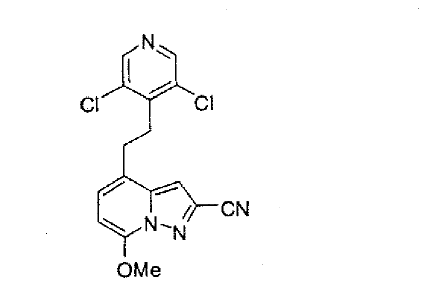

The compound of Example 129 (320 mg) was dissolved in methanol (10 mL). Sodium acetate (450 mg) and hydroxylamine hydrochloride (191 mg) were added to the prepared solution, and the mixture was stirred at room temperature for 2 hours. The solvent of the reaction mixture was evaporated under reduced pressure, and the residue was washed with water to give a yellow powder. The obtained powder was suspended in dichloromethane (10 mL), and triethylamine (0. 375 mL) and trifluoroacetic anhydride (0.249mL) were added to the suspension. The mixture was stirred at room temperature for 2.5 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was recrystallized (ethanol) to obtain the target product (225 mg) as a yellow powder.
Melting point: 208-209°C
¹H NMR (DMSO-d₆, 400 MHz): δ 3.05 (2H, t, J = 7.6 Hz), 3.21 (2H, t, J = 7.6 Hz), 4.09 (3H, s), 6.56 (1H d, J = 7.9 Hz), 7.11 (1H, d, J = 7.9 Hz), 7.42 (1H, s), 8.57 (2H, s).
HRESIMS (+): 347.04773 (Calcd. for C₁₆H₁₃Cl₂N₄O 347.04664). Elemental Analysis (%): Found C 55.15%, H 3.49%, N 15.93%, Calcd. for C₁₆H₁₂Cl₂N₄O C 55.35%, H 3.49%, N 15.93%.

### <Example 131>

3,5-Dichloro-4-(2-(2-cyano-7-methoxypyrazolo[1,5-a]pyridin-4-yl )ethyl)pyridine 1-oxide

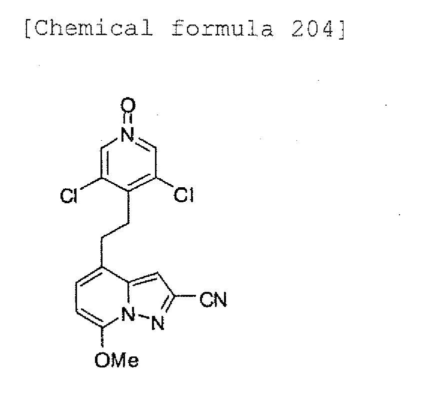

The compound of Example 130 (114 mg) was dissolved in chloroform (3.3 mL), and mCPBA (262 mg) was added to the prepared solution. The mixture was stirred at room temperature for 4.5 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by thin-layer silica gel chromatography (ethyl acetate : methanol = 10:1) to obtain the target product (21.9 mg) as a colorless powder.
Melting point: 212-213°C
¹H NMR (DMSO-d₆, 400 MHz): δ 3.02 (2H, t, J = 7.3 Hz), 3.12 (2H, t, J = 7.3 Hz), 4.09 (3H, s), 6.56 (1H, d, J = 7.9 Hz), 7.12 (1H, d, J = 7.9 Hz), 7.45 (1H, s), 8.53 (2H, s).
HRESIMS (+): 363.04173 (Calcd. for C₁₆H₁₃Cl₂N₄O₂ 363.04156).

### <Example 132>

2-(1,3-Dioxolan-2-yl )-7-methoxypyrazolo[1,5-a]pyridine-4-carbaldehyde

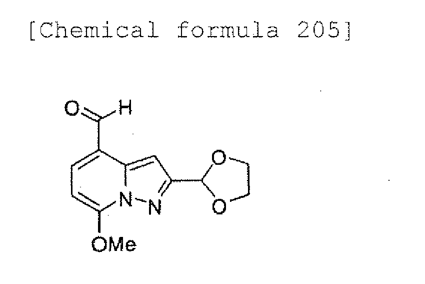

The compound of Example 127 (1.83g) was dissolved in chloroform (70 mL), and activated manganese dioxide (6.36 g) was added to the prepared solution. The mixture was stirred at 50°C for 1.5 hours. Insoluble material was removed by filtration through Celite, and the solvent of the filtrate was evaporated under reduced pressure to obtain the target product (1.75 g) as a yellow powder.
¹H NMR (CDCl₃, 400 MHz): δ 4.07-4.11 (2H, m), 4.20-4.22 (2H, m), 4.26 (3H, s), 6.23 (1H, s), 6.28 (1H, d, J = 7.9 Hz), 7.43 (1H, s), 7.76 (1H, d, J = 7.9 Hz), 9.95 (1H, s).
CIMS (+): 249 [M+H]⁺.

### <Example 133>

1-(2-(1,3-Dioxolan-2-yl )-7-methoxypyrazolo[1,5-a]pyridin-4-yl)-2-(3,5-dichloropyridin-4-yl )ethanol

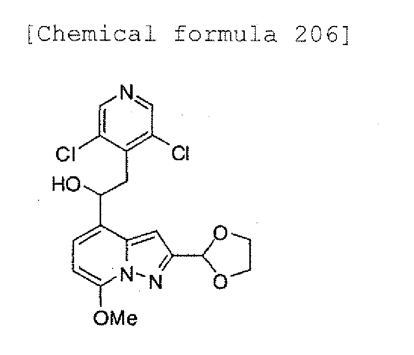

Diisopropylamine (0.724 mL) was dissolved in THF (30 mL) under argon atmosphere, and a solution of n-butyllithium in hexane (1.58 mol/L, 3.06 mL) was added to the prepared solution at 0°C. The mixture was stirred at 0°C for 30 minutes. A solution of 3,5-dichloro-4-picoline (773 mg) in THF (3.0 mL) was added to the resultant mixture at -78°C, and the mixture was heated from -78°C to 0°C. Subsequently, a solution of the compound of Example 132 (800 mg) in THF (5.0 mL) was added to the mixture at -73°C, and the resultant mixture was stirred at -78°C for 30 minutes. A saturated aqueous ammonium chloride solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was washed with diisopropyl ether to obtain the target product (1.27 g) as a colorless powder.
¹H NMR (CDCl₃, 400 MHz): δ 3.41 (1H, dd, J = 13.4, 4.9 Hz), 3.60 (1H, dd, J = 13.4, 8.6 Hz), 4.07-4.11 (2H, m), 4.15 (3H, s), 4.17-4.21 (2H, m), 5.32-5.36 (1H, m), 6.08 (1H, d, J = 7.9 Hz), 6.19 (1H, s), 6.85 (1H, s), 7.17 (1H, d, J = 7.9 Hz), 8.46 (2H, s).
ESIMS (+): 410 [M+H]⁺.

### <Example 134>

4-(2-(3,5-Dichloropyridin-4-yl )vinyl)-2-(1,3-dioxolan-2-yl)-7-methoxypyrazolo[1,5-a]pyridine

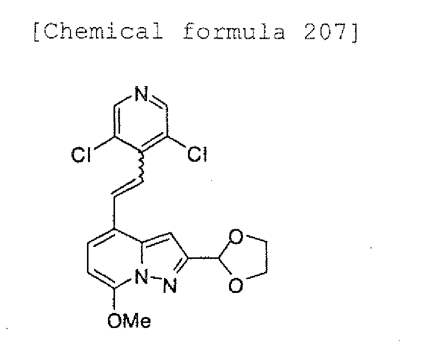

The compound of Example 133 (1.09 g) was dissolved in dichloromethane (25 mL) under argon atmosphere, and pyridine (2.16 mL) was added to the prepared solution. Then, trifluoromethanesulfonic anhydride (0.539 mL) was added to the mixture at 0°C, and the resultant mixture was stirred at room temperature for 1.5hours. A10% aqueous solution of sodium hydroxide (25 mL) was added to the reaction mixture, and the resultant mixture was stirred at 50°C for 2.5 hours. Water was added to the reaction mixture, and the resultant mixture was extracted three times with ethyl acetate. The combined extract layer was washed with saturated brine and dried over anhydrous sodium sulfate, and the solvent was evaporated under reduced pressure to obtain the target product (979 mg) as a yellow powder.
¹H NMR (CDCl₃, 400 MHz): δ 4.09-4.10 (2H, m), 4.20 (3H, s), 4.21-4.23 (2H, m), 6.20-6.21 (2H, m), 6.94 (1H, s), 7.19 (1H, d, J = 17.1 Hz), 7.40 (1H, d, J = 7.9 Hz), 7.63 (1H, d, J= 17.1 Hz), 8.52 (2H, s).
ESIMS (+): 392 [M+H]⁺.

### <Example 135>

2-(1,3-Dioxolan-2-yl )-7-methoxy-4-(2-(pyridin-4-yl )ethyl)pyrazolo[1,5-a]pyridine

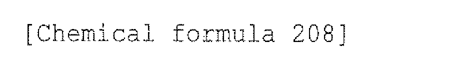

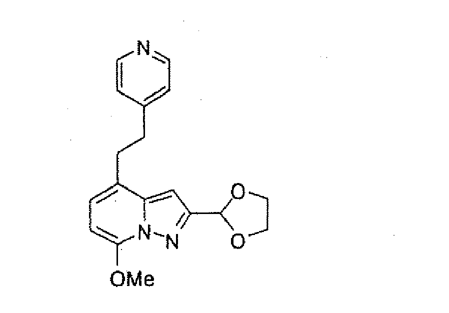

The compound of Example 134 (600 mg) was dissolved in DMF (30 mL). 10% Palladium-carbon (60.0 mg) and triethylamine (2.13 mL) were added to the prepared solution, and the mixture was stirred at room temperature for 2 hours under hydrogen atmosphere. Insoluble material was removed by filtration through Celite. Water was added to the filtrate, and the mixture was extracted three times with ethyl acetate. The combined extract layer was washed with saturated brine and dried over anhydrous sodium sulfate, and the solvent was evaporated under reduced pressure. The residue was dissolved in DMF (15 mL), and 10% palladium-carbon (60.0 mg) and triethylamine (2.13 mL) were added to the prepared solution. The mixture was stirred at room temperature for 3 hours under hydrogen atmosphere. Insoluble material was removed by filtration through Celite. Water was added to the filtrate, and the mixture was extracted three times with ethyl acetate. The combined extract layer was washed with saturated brine and dried over anhydrous sodium sulfate, and the solvent was evaporated under reduced pressure to obtain the target product (470 mg) as a yellow oil.
¹H NMR (CDCl₃, 400 MHz): δ 3.01-3.06 (4H, m), 4.07-4.13 (5H, m), 4.17-4.23 (2H, m), 5.98 (1H, d, J = 7.9 Hz), 6.18 (1H, s), 6.65 (1H, s), 6.75 (1H, d, J = 7.9 Hz), 7.07 (2H, d, J = 6.1 Hz), 8.49 (2H, d, J = 6.1 Hz).
EIMS (+): 325 [M]⁺.

### <Example 136>

7-Methoxy-4-(2-(pyridin-4-yl )ethyl)pyrazolo[1,5-a]pyridine-2-carbaldehyde

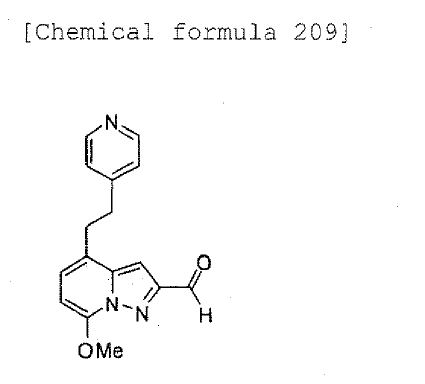

The compound of Example 135 (470 mg) was dissolved in acetone (7.0 mL) and water (7.0 mL). Then, p-toluenesulfonic acid monohydrate (299 mg) was added to the prepared solution, and the mixture was stirred at 70°C for 1 hour. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (ethyl acetate : methanol = 20:1) to obtain the target product (292 mg) as a colorless powder.
¹H NMR (CDCl₃, 400 MHz): δ 3.01-3.03 (2H, m), 3.10-3.11 (2H, m), 4.18 (3H, s), 6.14 (1H, d, J = 7.3 Hz), 6.84 (1H, d, J = 7.3 Hz), 7.06 (2H, d, J = 6.1 Hz), 7.10 (1H, s), 8.50 (2H, d, J = 6.1 Hz), 10.31 (1H, s).
EIMS (+): 281 [M]⁺.

### <Example 137>

7-Methoxy-4-(2-(pyridin-4-yl )ethyl)pyrazolo[1,5-a]pyridine-2-carbonitrile

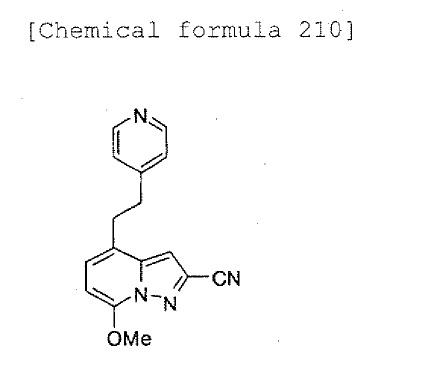

The compound of Example 136 (159 mg) was dissolved in methanol (6.0 mL). Then, sodium acetate (295 mg) and hydroxylamine hydrochloride (125 mg) were added to the prepared solution, and the mixture was stirred at room temperature for 2 hours. The solvent of the reaction mixture was evaporated under reduced pressure, and the residue was washed with water to give a yellow powder. The obtained yellow powder was suspended in dichloromethane (5.0 mL). Then, triethylamine (0.329 mL) and trifluoroacetic anhydride (0.131 mL) were added to the suspension, and the mixture was stirred at room temperature for 2 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (ethyl acetate : methanol = 20:1), and the purified product was washed with diisopropyl ether to obtain the target product (105 mg) as a yellow powder.
Melting point: 191-192°C
¹H NMR (DMSO-d₆, 400 MHz): δ 2.96 (2H, dd, J = 8.6, 5.5 Hz), 3.09 (2H, dd, J = 8.6, 5.5 Hz), 4.08 (3H, s), 6.57 (1H, d, J = 7. 3 Hz), 7.19 (1H, d, J = 7.3 Hz), 7.27 (2H, d, J = 6.1 Hz), 7.57 (1H, s), 8.43 (2H, d, J = 6.1 Hz).
HREIMS (+): 278.1135 (Calcd. for C₁₆H₁₄N₄O 278.1168).
Elemental Analysis (%): Found C 68.81%, H 5.14%, N 19.92%, Calcd. for C₁₆H₁₄N₄O C 69.05%, H 5.07%, N 20.13%.

### <Example 138>

4-(2-(2-Cyano-7-methoxypyrazolo[1,5-a]pyridin-4-yl )ethyl)pyridine 1-oxide

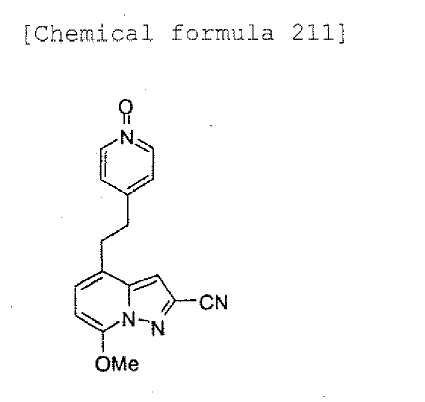

The compound of Example 137 (18.1 mg) was dissolved in chloroform (1.0 mL). Then, mCPBA (25.9mg) was added to the prepared solution at 0°C, and the mixture was stirred at room temperature for 45 minutes. The reaction mixture was purified by thin-layer chromatography (ethyl acetate: methanol = 4:1) to obtain the target product (13.7 mg) as a colorless powder.
Melting point: 206-208°C (decomp.)
¹H NMR (DMSO-d₆, 400 MHz): δ 3.02-3.10 (4H, m), 4.16 (3H, s), 6.18 (1H, d, J= 7.9 Hz), 6.85 (1H, d, J = 7.9 Hz), 6.98 (1H, s), 6.99 (2H, d, J = 6.7 Hz), 8.11 (2H, d, J = 6.7 Hz).

HRCIMS (+): 295.11756 (Calcd. for C₁₆H₁₅N₄O₂ 295.11950).

### <Example 139>

1-(2-(1,3-Dioxolan-2-yl )-7-methoxypyrazolo[1,5-a]pyridin-4-yl )-2-(3,5-dichloropyridin-4-yl )ethanone

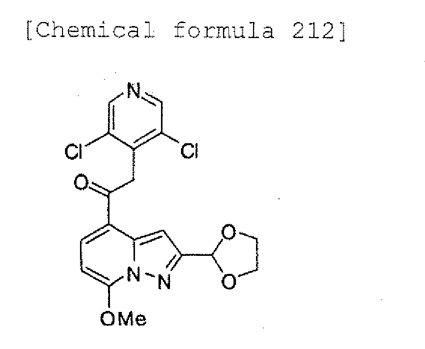

The compound of Example 133 (1.10 g) was dissolved in dichloromethane (30 mL) under argon atmosphere, and Dess-Martin periodinane (1.49 g) was added to the prepared solution at 0°C. The mixture was stirred at 0°C for 1 hour. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (ethyl acetate) to obtain the target product (973 mg) as a yellow powder.
¹H NMR (CDCl₃, 400 MHz): δ 4.06-4.08 (2H, m), 4.14-4.17 (2H, m), 4.28 (3H, s), 4.70 (2H, s), 6.22 (1H, s), 6.25 (1H, d, J = 7.9 Hz), 7.40 (1H, s), 8.10 (1H, d, J = 7.9 Hz), 8.54 (2H, s).
EIMS (+): 407 [M]⁺.

### <Example 140>

4-(2-(3,5-Dichloropyridin-4-yl )acetyl)-7-methoxypyrazolo[1,5-a]pyridine-2-carbaldehyde

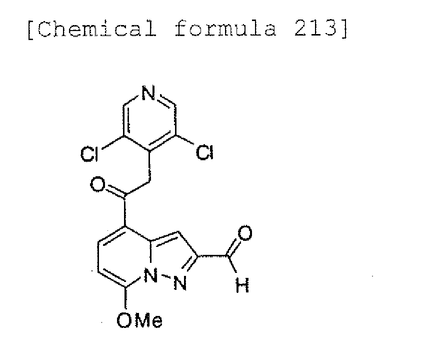

The compound of Example 139 (973 mg) was dissolved in acetone (20 mL) and water (10 mL). Then, p-toluenesulfonic acid monohydrate (97.0 mg) was added to the prepared solution, and the mixture was stirred at 60°C for 8 hours. A saturated aqueous sodium hydrogen carbonate solution was added to the reaction mixture, and acetone was evaporated under reduced pressure. The precipitate was collected by filtration to obtain the target product (801 mg) as a yellow powder.
¹H NMR (CDCl₃, 400 MHz): δ 4.35 (3H, s), 4.71 (2H, s), 6.41 (1H, d, J = 7.9 Hz), 7.81 (1H, s), 8.16 (1H, d, J = 7.9 Hz), 8.55 (2H, s), 10.31 (1H, s).
EIMS (+): 203 [M]⁺.

### <Example 141>

4-(2-(3,5-Dichloropyridin-4-yl )acetyl)-7-methoxypyrazolo[1,5-a]pyridine-2-carbonitrile

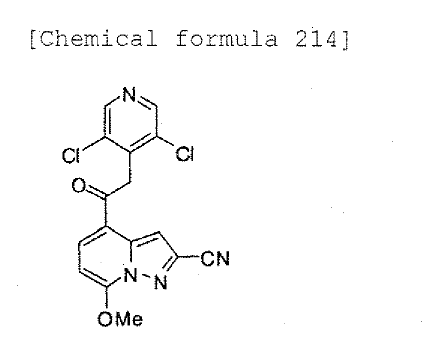

The compound of Example 140 (801 mg) was dissolved in methanol (25 mL). Then, sodium acetate (1.08 g) and hydroxylamine hydrochloride (459 mg) were added to the prepared solution, and the mixture was stirred at room temperature for 4 hours. The solvent of the reaction mixture was evaporated under reduced pressure, and the residue was washed with water to obtain a yellow powder. The obtained powder was suspended in dichloromethane (20 mL). Then, triethylamine (0.882 mL) and trifluoroacetic anhydride (0.586 mL) were added to the suspension, and the mixture was stirred at room temperature for 2 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was recrystallized (methanol) to obtain the target product (489 mg) as a green powder.
Melting point: 194-196°C
¹H NMR (DMSO-d₆, 400 MHz): δ 4.30 (3H, s), 4.88 (2H, s), 6.91 (1H, d, J = 7.9 Hz), 7.73 (1H, s), 8.68 (2H, s), 8.72 (1H, d, J = 7.9 Hz).
HREIMS (+): 360.0154 (Calcd. for C₁₆H₁₀Cl₂N₄O₂ 360.0181).
Elemental Analysis (%): Found C 52.99%, H 2.89%, N 15.31%, Calcd. for C₁₆H₁₀Cl₂N₄O₂ C 53.21%, H 2.79%, N 15.51%.

### <Example 142>

3,5-Dichloro-4-(2-(2-cyano-7-methoxypyrazolo[1,5-a]pyridin-4-yl )-2-oxoethyl)pyridine 1-oxide

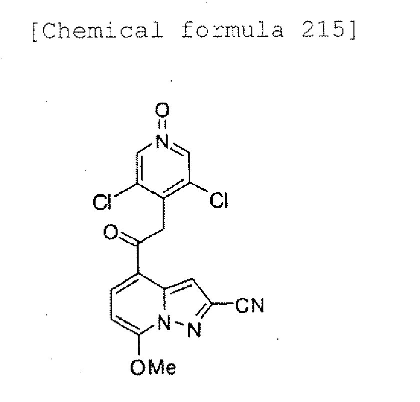

The compound of Example 141 (100 mg) was dissolved in chloroform (5.0 mL) . Then, mCPBA (220 mg) was added to the prepared solution, and the mixture was stirred at room temperature for 25 hours. A saturated aqueous sodium hydrogen carbonate solution was added to the reaction mixture, and the resultant mixture was extracted three times with ethyl acetate. The combined extract layer was washed with saturated brine and dried over anhydrous sodium sulfate, and the solvent was evaporated under reduced pressure. The residue was purified by silica gel column chromatography (ethyl acetate : methanol = 4:1), and the purified product was washed with diisopropyl ether to obtain the target product (57.8 mg) as a yellow powder. Melting point: 186-189°C
¹H NMR (DMSO-d₆, 400 MHz): δ 4.29 (3H, s), 4.78 (2H, s), 6.90 (1H, d, J = 7.9 Hz), 7.74 (1H, s), 8.65 (2H, s), 8.70 (1H, d, J = 7.9 Hz).
HRESIMS (+): 377.02108 (Calcd. for C₁₆H₁₁Cl₂N₄O₃ 377.02082).

### <Example 143>

7-Methoxy-4-(2-(pyridin-4-yl )acetyl)pyrazolo[1,5-a]pyridine-2-carbonitrile

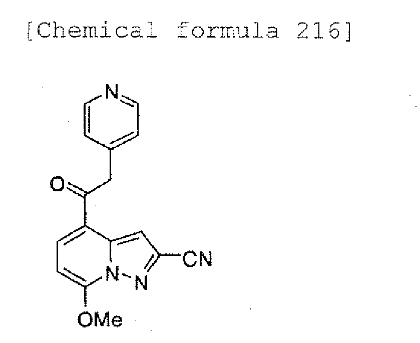

The compound of Example 141 (361 mg) was dissolved in DMF (10 mL). Then, triethylamine (1.39 mL) and 10% palladium-carbon (35 mg) were added to the prepared solution, and the mixture was stirred at room temperature for 15.5 hours under hydrogen atmosphere. A saturated aqueous sodium hydrogen carbonate solution and ethyl acetate were added to the reaction mixture, and insoluble material was removed by filtration through Celite. The filtrate was extracted three times with ethyl acetate. The combined extract layer was washed with saturated brine and dried over anhydrous sodium sulfate, and the solvent was evaporated under reduced pressure. The residue was purified by silica gel column chromatography (ethyl acetate : methanol = 10:1), and the purified product was recrystallized (ethanol) to obtain the target product (60.0 mg) as a yellow powder.
¹H NMR (DMSO-d₆, 400 MHz): δ 4.27 (3H, s), 4.52 (2H, s), 6.86 (1H, d, J = 7.9 Hz), 7.31 (2H, d, J = 6.1 Hz), 7.76 (1H, s), 8.50 (2H, d, J = 6.1 Hz), 8.56 (1H, d, J = 7.9 Hz).

HREIMS (+): 292.0983 (Calcd. for C₁₆H₁₂N₄O₂ 292.0960).

### <Example 144>

4-(2-(2-Cyano-7-methoxypyrazolo[1,5-a]pyridin-4-yl )-2-oxoethyl)pyridine 1-oxide

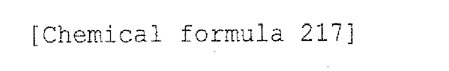

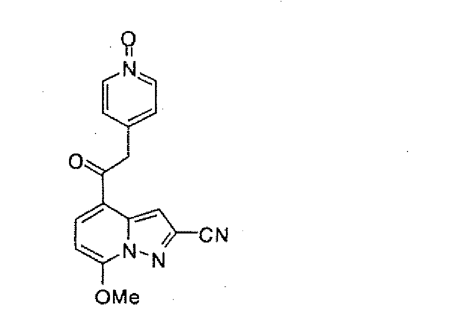

The compound of Example 143 (19.0 mg) was dissolved in chloroform (1.0 mL). Then, mCPBA (28.0 mg) was added to the prepared solution at 0°C, and the mixture was stirred at room temperature for 1 hour. The reaction mixture was purified by thin-layer chromatography to obtain the target product (9.9 mg) as a yellow powder.
Melting point: 170-172°C
¹H NMR (DMSO-d₆, 400 MHz): δ 4.27 (3H, s), 4.52 (2H, s), 6.87 (1H, d, J = 7.9 Hz), 7.31 (2H, d, J = 6.8 Hz), 7.77 (1H, s), 8.17 (2H, d, J = 6.8 Hz), 8.53 (1H, d, J = 7.9 Hz).
HRESIMS (+): 309.09877 (Calcd. for C₁₆H₁₃N₄O₃ 309.09876).

### <Example 145>

4-(2-(7-Methoxy-2-(trifluoromethyl)pyrazolo[1,5-a]pyridin-4-yl)-2-phenylethyl)pyridine 1-oxide

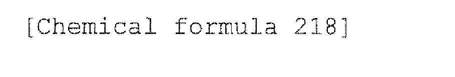

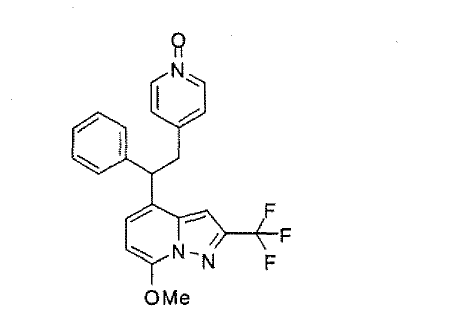

The compound of Example 86 (189 mg) was dissolved in chloroform (5.0 mL). Then, mCPBA (160 mg) was added to the prepared solution, and the mixture was stirred at room temperature for 1 hour. A saturated aqueous sodium hydrogen carbonate solution was added to the reaction mixture, and the resultant mixture was extracted three times with ethyl acetate. The obtained organic layer was washed with saturated brine and dried over anhydrous sodium sulfate, and the solvent was evaporated under reduced pressure. The residue was purified by silica gel column chromatography (ethyl acetate : methanol=4:1) to obtain the target product (190 mg) as a yellow amorphous.
¹H NMR (CDCl₃, 400 MHz): δ 3.35 (1H, dd, J = 13.8, 7.9 Hz), 3.47 (1H, dd, J = 13.8, 7.9 Hz), 4.17 (3H, s), 4.34 (1H, t, J = 7.9 Hz), 6.23 (1H, d, J = 7. 9 Hz), 6.60 (1H, s), 6.86 (2H, d, J = 7.3 Hz), 7.13-7.17 (3H, m), 7.23-7.29 (3H, m), 8.05 (2H, d, J = 7.3 Hz).
HREIMS (+): 414.1388 (Calcd. for C₂₂H₁₉F₃N₃O₂ 414.1429).

### <Experimental Example 1> Phosphodiesterase inhibitory activity

The cDNA of PDE4B catalytic domain (hereinafter abbreviated as Cat) was isolated from human RNA by RT-PCR. The isolated cDNA fragments were inserted into Sf9 insect cells using Gateway system (product of Invitrogen Corporation) and Bac-to-Bac (registered trademark) Baculovirus Expression system (product of Invitrogen Corporation) to express a target PDE protein. The recombinant PDE4Bcat was purified from the culture supernatants or cell extracts of Sf9 cells expressing high levels of the PDE protein by ion exchange chromatography and was used for the following experiments.

A 4 mmol/L solution of each test compound was serially diluted four-fold with a 15% DMSO solution to prepare solutions with concentrations of 15 nmol/L to 4 mmol/L (the final concentrations used in the experiments were 1.5 nmol/L to 400 µmol/L). 10 µL of the prepared test compound solutions, [³H] cAMP diluted with a buffer solution (40 mmol/L Tris-HCl (pH: 7.4), 10 mmol/L MgCl₂), and 40 µL of the recombinant human PDE protein (2 x 10⁻⁶ units, 1 unit is defined as the amount of PDE that breaks down 1 µmol/L of cAMP in one minute under the conditions of a pH of 7.5 and 30°C) were added to a 96-well plate. The mixtures were reacted at 30°C for 20 minutes. Then, the resultant mixtures were reacted at 65°C for 2 minutes. Subsequently, 25 µL of 1 mg/mL 5' -nucleotidase (Crotalus atrox venom, product of Sigma) was added to the mixtures, and the mixtures were reacted at 30°C for 10 minutes. After completion of the reaction, 200 µL of a solution of Dowex (300 mg/mL Dowex 1x8-400 (product of Sigma Aldrich), 33% ethanol) was added to the mixtures, and the mixtures were shaken at 4°C for 20 minutes. Subsequently, 200 µL of MicroScint 20 (product of Packard) was added to the mixtures, and measurement was performed using a scintillation counter (Topcount, product of Packard). IC₅₀ values were calculated using Graph Pad Prism v3.03 (product of GraphPad Software). When 1 µmol/L > the IC₅₀ value ≥ 0.1 µmol/L, the results were represented by "+." When 0.1 µmol/L > the IC₅₀ value, the results were represented by "++."

The results are shown in Table 1.

**Table 1**

| Example No. | IC₅₀(µmol/L) PDE4 | Example No. | IC₅₀(µmol/L) PDE4 |
|---|---|---|---|
| 77 | + | 91 | ++ |
| 78 | ++ | 92 | ++ |
| 79 | ++ | 93 | ++ |
| 80(90) | ++ | 99 | ++ |
| 84 | ++ | 108 | ++ |
| 86 | ++ | 121 | ++ |
| 87 | ++ | 122 | ++ |
| 88 | ++ | 142 | ++ |
| 89 | ++ | | |

### <Experimental Example 2> Histamine-induced airway contraction reaction in guinea pigs

Guinea pigs were anesthetized with pentobarbital (30 mg/kg, i.p.). A cannula for intravenous administration, a cannula for collecting blood and measuring blood pressure, and a tracheal cannula were inserted into the left external jugular vein, right internal carotid artery, and trachea, respectively. The guinea pigs were maintained on artificial respiration under the conditions of 60 times/min and 10 mL/kg/stroke. The airflow from the side branch of the tracheal cannula was measured by a bronchospasm transducer (Ugo-Basile) and recorded on a computer via Power Lab (ADInstruments Japan). The guinea pigs were immobilized with gallamine (10 mg/kg, i.v.), and histamine was administered at 10-minutes intervals (12.5 µg/kg, i.v.). After the histamine-induced bronchoconstriction was constant, one of the test compounds (1 mg/kg, i.v.) dissolved in DMSO was administered. We examined the inhibitory action of test compounds on histamine-induced bronchoconstriction 30 seconds after compounds administration. Bronchoconstriction was recorded as the value of airflow, and the results were represented by the ratio of the maximum value of the histamine-induced airflow 30 seconds after the administration to the maximum value of the airflow before the administration. When the inhibition ratio ≥ 90%, the results were represented by "+++." When 90% > the inhibition ratio ≥ 70%, the results were represented by "++."' When 70% > the inhibition ratio ≥ 30%, the results were represented by "+."

The results are shown in Table 2.

Table 2

**Table 2**

| Example No. | Inhibition ratio |
|---|---|
| 77 | + |
| 78 | + |
| 79 | + |
| 80(90) | +++ |
| 88 | ++ |
| 89 | + |

### <Experimental Example 3> LPS acute inflammation model in rats

3 mg/kg of the compounds were orally administered to rats one hour before inhalation of lipopolysaccharide (LPS) from E.coli serotype 055:B5, rats were inhalted nebulizing LPS solution (50 mL) by nebulizer for 30 minutes. Then, 3 hours after the LPS inhalation, rats were euthanized with 20% urethane (5 ml/rat, i.p.). 5 ml of physiological saline for bronchoalveolar lavage was injected into the bronchoalveolar space through the airway, and the bronchoalveolar space was washed 3 times using a 5 mL syringe. This procedure was repeated twice, and the solution was collected as bronchoalveolar lavage fluid (BALF). The collected BALF was centrifuged at 1,200 rpm and 4°C for 10 minutes (Hirtachi; himac CR 5 DL). The sediment was re-suspended in 10 mL of a 0.1% bovine serum albumin-physiological saline, and an equivalent amount of Turk's solution was added to the suspension to stain leukocytes. The total number of leukocytes was counted under a microscope to determine the inhibition ratio. When the inhibition ratio ≥ 60%, the results were represented by "++." When 60% > the inhibition ratio ≥ 30%, the results were represented by "+."

The results are shown in Table 3.

**Table 3**

| Example No. | Inhibition ratio | Example No. | Inhibition ratio |
|---|---|---|---|
| 77 | NT | 92 | ++ |
| 78 | + | 93 | ++ |
| 79 | ++ | 108 | ++ |
| 80(90) | NT | 121 | ++ |
| 88 | ++ | 122 | ++ |
| 89 | + | 142 | ++ |
| 91 | ++ | | |

As described above, the compounds represented by the general formula (1) of the present invention have PDE inhibitory activity, and the effects of the compounds have been confirmed the experimental models in animals.

### INDUSTRIAL APPLICABILITY

As has been described, the present invention is based on the findings that the novel pyrazolopyridine derivatives and addition salts thereof have excellent PDE inhibitory activity. Such compounds having PDE inhibitory activity are useful as preventive and therapeutic drugs for bronchial asthma, chronic obstructive pulmonary disease (COPD), interstitial pneumonia, allergic rhinitis, atopic dermatitis, rheumatic arthritis, multiple sclerosis, Huntington's disease, Alzheimer's disease, dementia, Parkinson's disease, schizophrenia, and the like.

## Claims

1. A pyrazolopyridine derivative represented by the general formula (1): 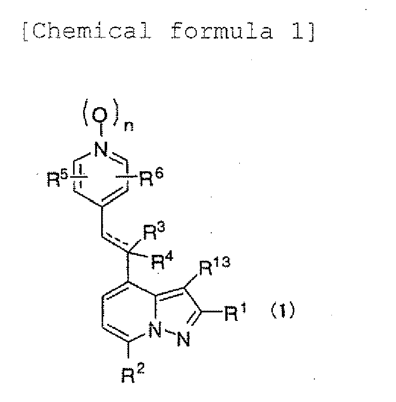 [wherein R¹ is a hydrogen atom, an optionally substituted alkyl group having 1 to 6 carbon atoms (optionally substituted with a halogen atom, an alkoxy group having 1 to 6 carbon atoms, or a hydroxyl group), a cycloalkyl group having 3 to 8 carbon atoms, an alkanoyl group having 1 to 6 carbon atoms, an oxime group, or a cyano group, R² is a hydrogen atom, an optionally substituted alkyl group having 1 to 6 carbon atoms (optionally substituted with an alkoxy group having 1 to 6 carbon atoms or a hydroxyl group), an alkoxy group having 1 to 6 carbon atoms, an alkylsulfinyl group having 1 to 6 carbon atoms, an alkylsulfinyl group having 1 to 6 carbon atoms, an alkylsulfonyl group having 1 to 6 carbon atoms, an optionally substituted amino group (optionally substituted with an alkyl group having 1 to 6 carbon atoms), or an alkanoyl group having 1 to 6 carbon atoms, R⁵ and R⁶ are independently a hydrogen atom or a halogen atom, R¹³ is a hydrogen atom or a halogen atom, n is 0 or 1, and 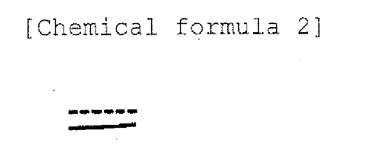 represents a single or double bond, wherein, when 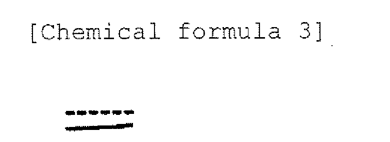 is a double bond, R³ is involved in formation of the double bond, and R⁴ is a hydrogen atom or a phenyl group, and wherein, when 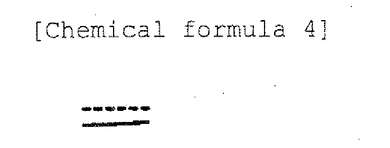 is a single bond, R³ is a hydrogen atom or a hydroxyl group and R⁴ is a hydrogen atom or a phenyl group, or R³ and R⁴ together form oxo] or a pharmaceutically acceptable salt or hydrate thereof.

2. The pyrazolopyridine derivative according to claim 1, or a pharmaceutically acceptable salt or hydrate thereof, wherein the compound represented by the general formula (1) is represented by the general formula (1a): 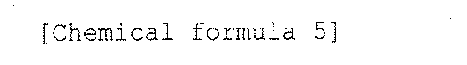 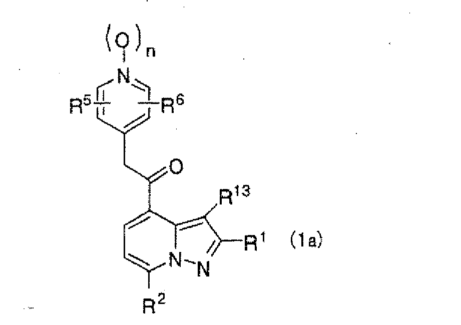 [wherein R¹, R², R⁵, R⁶, R¹³, and n are as described above].

3. The pyrazolopyridine derivative according to claim 1, or a pharmaceutically acceptable salt or hydrate thereof, wherein the compound represented by the general formula (1) is represented by the general formula (1b): 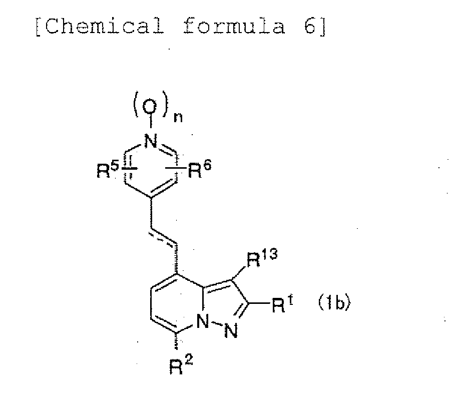 [wherein R¹, R², R⁵, R⁶, R¹³, n, and 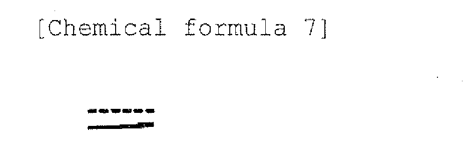 are as described above].

4. The pyrazolopyridine derivative according to claim 1, or a pharmaceutically acceptable salt or hydrate thereof, wherein, in the compound represented by the general formula (1), R¹³ is a hydrogen atom.

5. The pyrazolopyridine derivative according to claim 1, or a pharmaceutically acceptable salt or hydrate thereof, wherein the compound represented by the general formula (1) is
7-methoxy-4-[(2-pyridin-4-yl )vinyl]-2-trifluoromethylpyrazolo [1,5-a]pyridine,
7-methoxy-4-[(2-pyridi-n-4-yl )ethyl]-2-trifluoromethylpyrazolo [1,5-a]pyridine,
1-(7-methoxy-2-trifluoromethylpyrazolo[1,5-a]pyridin-4-yl )-2-(pyridin-4-yl )ethanone,
2-(3,5-dichloropyridin-4-yl )-1-(7-methoxy-2-trifluoromethylpy razolo[1,5-a]pyridin-4-yl )ethanone,
(E)-4-[2-(3,5-dichloropyridin-4-yl )vinyl]-7-methoxy-2-trifluo romethylpyrazolo[1,5-a]pyridine,
2-(pyridin-4-yl )-1-(7-methoxy-2-trifluoromethylpyrazolo[1,5-a ]pyridin-4-yl )ethanone,
4-(2-(7-methoxy-2-(trifluoromethyl)pyrazolo[1, 5-a]pyridin-4-yl )ethyl)pyridine 1-oxide,
3,5-dichloro-4-(2-(7-methoxy-2-(trifluoromethyl)pyrazolo[1, 5-a]pyridin-4-yl )-2-oxoethyl)pyridine 1-oxide,
4-(2-(7-methoxy-2-(trifluoromethyl)pyrazolo[1,5-a]pyridin-4-yl )-2-oxoethyl)pyridine 1-oxide,
2-(3,5-dichloropyridin-4-yl )-1-(7-(methylamino)-2-(trifluorom ethyl)pyrazolo[1,5-a]pyridin-4-yl )ethanone,
3,5-dichloro-4-(2-(2-(difluoromethyl)-7-methoxypyrazolo[1,5-a] pyridin-4-yl )-2-oxoethyl)pyridine 1-oxide,
1-(2-cyclopropyl-7-methoxypyrazolo[1,5-a]pyridin-4-yl )-2-(pyridin-4-yl )ethanone,
1-(3-chloro-2-cyclopropyl-7-methoxypyrazolo[1,5-a]pyridin-4-yl )-2-(3,5-dichloropyridin-4-yl )ethanone, or
3,5-dichloro-4-(2-(2-cyano-7-methoxypyrazolo[1,5-a]pyridin-4-y 1 )-2-oxoethyl)pyridine 1-oxide.

6. A phosphodiesterase (PDE) inhibitor comprising the pyrazolopyridine derivative according to any of claims 1 to 5, or a pharmaceutically acceptable salt or hydrate thereof.

7. A pharmaceutical comprising the pyrazolopyridine derivative according to any of claims 1 to 5, or a pharmaceutically acceptable salt or hydrate thereof.
